# EUROPEAN PATENT SPECIFICATION

(11) **EP 3 141 550 B1**
(45) Date of publication and mention of the grant of the patent: **18.03.2020**
(21) Application number: 16186849.2
(22) Date of filing: 01.09.2016
(51) Int. Cl.: C07D 487/04, C07D 491/04, C07D 495/04, C07D 209/94, C09K 11/06, H01L 51/50

(54) **CONDENSED CYCLIC COMPOUND AND ORGANIC LIGHT-EMITTING DEVICE INCLUDING THE SAME**
KONDENSIERTE CYCLISCHE VERBINDUNG UND ORGANISCHE LICHTEMITTIERENDE VORRICHTUNG DAMIT
COMPOSÉ CYCLIQUE CONDENSÉ ET DISPOSITIF ÉLECTROLUMINESCENT ORGANIQUE LE COMPRENANT

(30) Priority: 09.09.2015 KR 20150127715; 29.08.2016 KR 20160110092
(43) Date of publication of application: 15.03.2017
(73) Proprietor: Samsung Electronics Co., Ltd., Suwon-si, Gyeonggi-do 16677 (KR); Samsung SDI Co., Ltd., Giheung-gu Yongin-si, Gyeonggi-do (KR)
(72) Inventor: KIM, Sangmo, 16678 Gyeonggi-do (KR); KIM, Hyunjung, 44714 Ulsan (KR); JEON, Soonok, 16678 Gyeonggi-do (KR); CHUNG, Yeonsook, 16678 Gyeonggi-do (KR); CHAE, Miyoung, 16678 Gyeonggi-do (KR); HUH, Dalho, 16678 Gyeonggi-do (KR); KWON, Eunsuk, 16678 Gyeonggi-do (KR); KIM, Jongsoo, 16678 Gyeonggi-do (KR); SIM, Myungsun, 16678 Gyeonggi-do (KR)
(74) Representative: Elkington and Fife LLP

(56) References cited:
- EP-A1- 2 298 774
- KR-A- 20150 077 219
- US-A1- 2015 171 340
- US-A1- 2015 236 262

## Description

### FIELD OF THE INVENTION

The present disclosure relates to a condensed cyclic compound and an organic light-emitting device including the condensed cyclic compound.

### BACKGROUND OF THE INVENTION

Organic light-emitting devices (OLEDs) are self-emission devices that have wide viewing angles, high contrast ratios, and short response times. In addition, the OLEDs exhibit excellent luminance, driving voltage, and response speed characteristics, and produce full-color images.

A typical organic light-emitting device includes an anode, a cathode, and an organic layer that is disposed between the anode and the cathode, wherein the organic layer may include an emission layer. A hole transport region may be disposed between the anode and the emission layer, and an electron transport region may be disposed between the emission layer and the cathode. Holes provided from the anode may move toward the emission layer through the hole transport region, and electrons provided from the cathode may move toward the emission layer through the electron transport region. The holes and the electrons recombine in the emission layer to produce excitons. These excitons change from an excited state to a ground state to thereby generate light.

Different types of organic light emitting devices are known. However, there still remains a need in OLEDs having low driving voltage, high efficiency, high brightness, and long lifespan.

US 2015/236262 discloses an organic light-emitting device including: a first electrode; a second electrode facing the first electrode; and an organic layer including an emission layer between the first electrode and the second electrode. The emission layer includes at least one compound selected from carbazole-based compounds, and at least one compound selected from certain heterocyclic compounds.

EP 2 298 774 discloses polycyclic compound of a specific structure having a π-conjugated heteroacene skeleton crosslinked with a carbon atoms, a nitrogen atom, or an oxygen atom, and a halogen compound which can be used for the synthesis of the polycyclic compound.

US 2015/171340 discloses condensed-cyclic compounds and organic light-emitting devices including the condensed-cyclic compounds.

KR 2015 0077219 discloses a benzocarbazole compound and an organic electroluminescent device comprising the compound in one or more organic layers.

### SUMMARY OF THE INVENTION

Provided are a condensed cyclic compound and an organic light-emitting device including the condensed cyclic compound.

Additional aspects will be set forth in part in the description which follows and, in part, will be apparent from the description, or may be learned by practice of the presented exemplary embodiments.

According to the invention, provided is a condensed cyclic compound represented by one of Formulae 1-1 to 1-3: 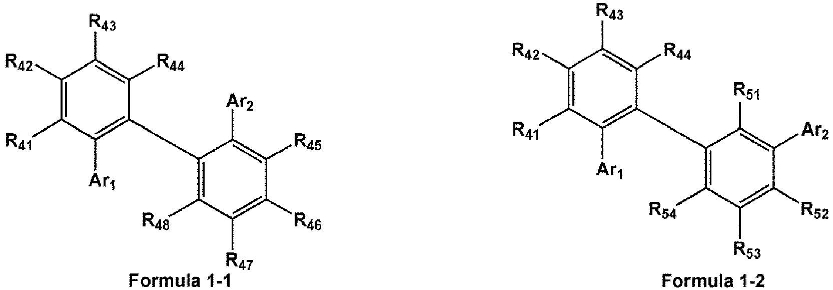 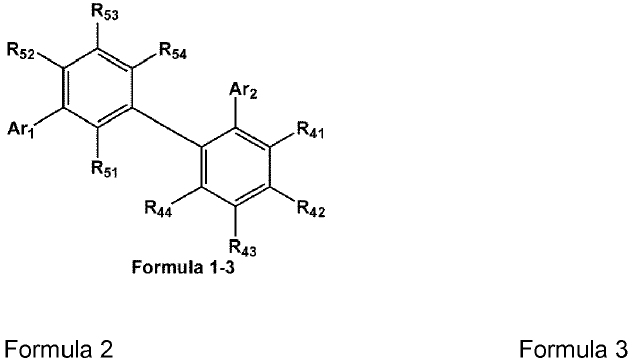 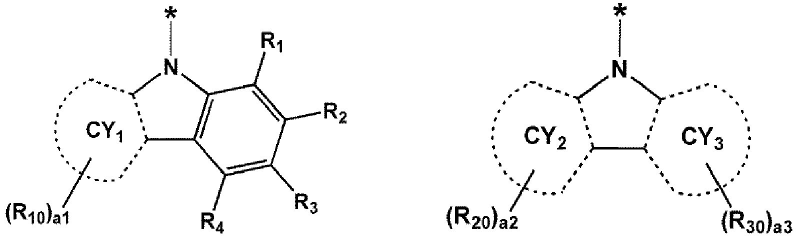 wherein
Ar₁ is a group represented by Formula 2,
Ar₂ is a group represented by Formula 3,
CY₁ is selected from a fluorene group, a carbazole group, a dibenzofuran group, and a dibenzothiophene group,
CY₂ and CY₃ may be each independently selected from a benzene group, a fluorene group, a carbazole group, a dibenzofuran group, and a dibenzothiophene group,
R₁ to R₄, R₁₀, R₂₀ and R₃₀ are each independently selected from a hydrogen, a deuterium, a hydroxyl group, a cyano group (CN), a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₂-C₆₀ heteroaryloxy group, a substituted or unsubstituted C₂-C₆₀ heteroarylthio group, a substituted or unsubstituted C₃-C₆₀ heteroarylalkyl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -N(Q₄)(Q₅), and -B(Q₆)(Q₇),
R₄₁₋₄₈ and R₅₁₋₅₄ are each independently selected from a hydrogen, a deuterium, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₂-C₆₀ heteroaryloxy group, a substituted or unsubstituted C₂-C₆₀ heteroarylthio group, a substituted or unsubstituted C₃-C₆₀ heteroarylalkyl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -N(Q₄)(Q₅), and -B(Q₆)(Q₇),
a1 to a3 are each independently an integer of 0 to 10,
* is a binding site to a neighboring atom, and
at least one substituent of the substituted C₁-C₆₀ alkyl group, substituted C₂-C₆₀ alkenyl group, substituted C₂-C₆₀ alkynyl group, substituted C₃-C₁₀ cycloalkyl group, substituted C₁-C₁₀ heterocycloalkyl group, substituted C₃-C₁₀ cycloalkenyl group, substituted C₁-C₁₀ heterocycloalkenyl group, substituted C₆-C₆₀ aryl group, substituted C₆-C₆₀ aryloxy group, substituted C₆-C₆₀ arylthio group, substituted C₇-C₆₀ arylalkyl group, substituted C₁-C₆₀ heteroaryl group, substituted C₂-C₆₀ heteroaryloxy group, substituted C₂-C₆₀ heteroarylthio group, substituted C₃-C₆₀ heteroarylalkyl group, substituted monovalent non-aromatic condensed polycyclic group and substituted monovalent non-aromatic condensed heteropolycyclic group is selected from :
   a deuterium, -CD₃, -CD₂H, -CDH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group and a C₁-C₆₀ alkoxy group;
   a C₁-C₆₀ alkyl group, a C₂-C₆₀ alkenyl group, a C₂-C₆₀ alkynyl group and a C₁-C₆₀ alkoxy group, each substituted with at least one selected from a deuterium, -CD₃, -CD₂H, -CDH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₂-C₆₀ heteroaryloxy group, a C₂-C₆₀ heteroarylthio group, a C₃-C₆₀ heteroarylalkyl group, a monovalent non-aromatic condensed polycyclic group, a monovalent non-aromatic condensed heteropolycyclic group, -Si(Q₁₁)(Q₁₂)(Q₁₃), -N(Q₁₄)(Q₁₅) and -B(Q₁₆)(Q₁₇);
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₂-C₆₀ heteroaryloxy group, a C₂-C₆₀ heteroarylthio group, a C₃-C₆₀ heteroarylalkyl group, a monovalent non-aromatic condensed polycyclic group and a monovalent non-aromatic condensed heteropolycyclic group;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₂-C₆₀ heteroaryloxy group, a C₂-C₆₀ heteroarylthio group, a C₃-C₆₀ heteroarylalkyl group, a monovalent non-aromatic condensed polycyclic group and a monovalent non-aromatic condensed heteropolycyclic group, each substituted with at least one selected from a deuterium, -CD₃, -CD₂H, -CDH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₂-C₆₀ heteroaryloxy group, a C₂-C₆₀ heteroarylthio group, a C₃-C₆₀ heteroarylalkyl group, a monovalent non-aromatic condensed polycyclic group, a monovalent non-aromatic condensed heteropolycyclic group, -Si(Q₂₁)(Q₂₂)(Q₂₃), -N(Q₂₄)(Q₂₅) and-B(Q₂₆)(Q₂₇); and
   -Si(Q₃₁)(Q₃₂)(Q₃₃), -N(Q₃₄)(Q₃₅) and -B(Q₃₆)(Q₃₇),
   wherein Q₁ to Q₇, Q₁₁ to Q₁₇, Q₂₁ to Q₂₇ and Q₃₁ to Q₃₇ are each independently selected from a hydrogen, a deuterium, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₂-C₆₀ heteroaryloxy group, a substituted or unsubstituted C₂-C₆₀ heteroarylthio group, a substituted or unsubstituted C₃-C₆₀ heteroarylalkyl group, a substituted or unsubstituted monovalent non-aromatic condensed polycyclic group and a substituted or unsubstituted monovalent non-aromatic condensed heteropolycyclic group.

According to another aspect of an exemplary embodiment, provided is an organic light-emitting device including:
a first electrode;
a second electrode; and
an organic layer disposed between the first electrode and the second electrode, wherein the organic layer includes an emission layer, and
wherein the organic layer includes one or more of the condensed cyclic compounds represented by one of Formulae 1-1 to 1-3.

### BRIEF DESCRIPTION OF THE DRAWINGS

These and/or other aspects will become apparent and more readily appreciated from the following description of the exemplary embodiments, taken in conjunction with the accompanying drawings in which:

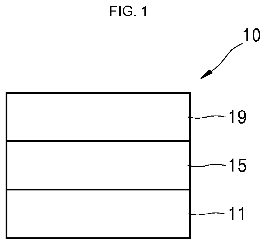

FIG. 1 illustrates a schematic view of an organic light-emitting device according to an embodiment.

### DETAILED DESCRIPTION OF THE EMBODIMENTS

Reference will now be made in detail to exemplary embodiments, examples of which are illustrated in the accompanying drawings, wherein like reference numerals refer to like elements throughout. In this regard, the present exemplary embodiments may have different forms and should not be construed as being limited to the descriptions set forth herein. Accordingly, the exemplary embodiments are merely described below, by referring to the figures, to explain aspects. Expressions such as "at least one of," when preceding a list of elements, modify the entire list of elements and do not modify the individual elements of the list.

It will be understood that when an element is referred to as being "on" another element, it can be directly in contact with the other element or intervening elements may be present therebetween. In contrast, when an element is referred to as being "directly on" another element, there are no intervening elements present.

It will be understood that, although the terms first, second, third etc. may be used herein to describe various elements, components, regions, layers, and/or sections, these elements, components, regions, layers, and/or sections should not be limited by these terms. These terms are only used to distinguish one element, component, region, layer, or section from another element, component, region, layer, or section. Thus, a first element, component, region, layer, or section discussed below could be termed a second element, component, region, layer, or section without departing from the teachings of the present embodiments.

The terminology used herein is for the purpose of describing particular embodiments only and is not intended to be limiting. As used herein, the singular forms "a," "an," and "the" are intended to include the plural forms as well, unless the context clearly indicates otherwise.

The term "or" means "and/or." It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art to which this general inventive concept belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ± 30%, 20%, 10%, 5% of the stated value.

A condensed cyclic compound is represented by Formulae 1-1 to 1-3 as described above.

According to an embodiment, in Formulae 1-1 to 1-3, Ar₁ may be selected from groups represented by Formulae 2-1 to 2-6 and Ar₂ may be selected from groups represented by Formulae 3-1 to 3-7, but embodiments are not limited thereto: 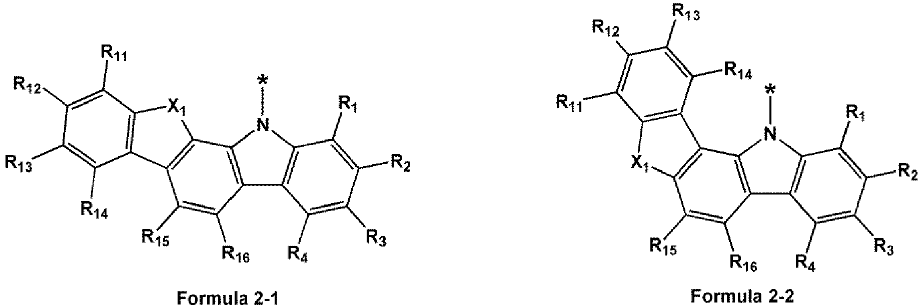 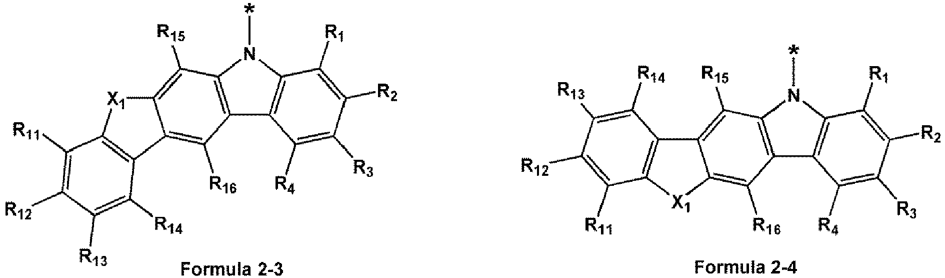 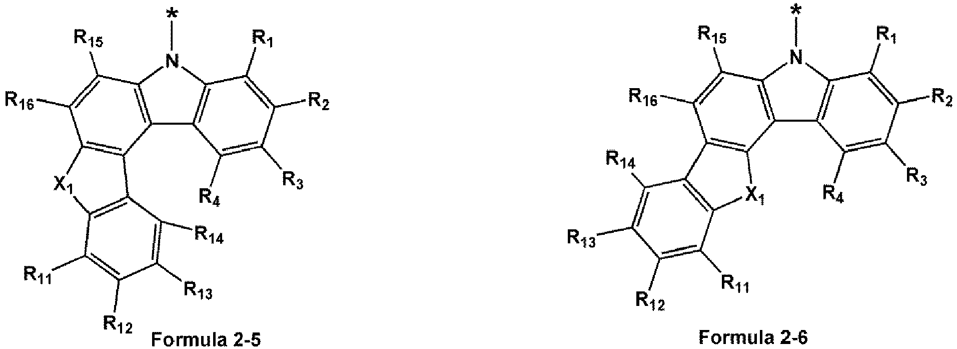 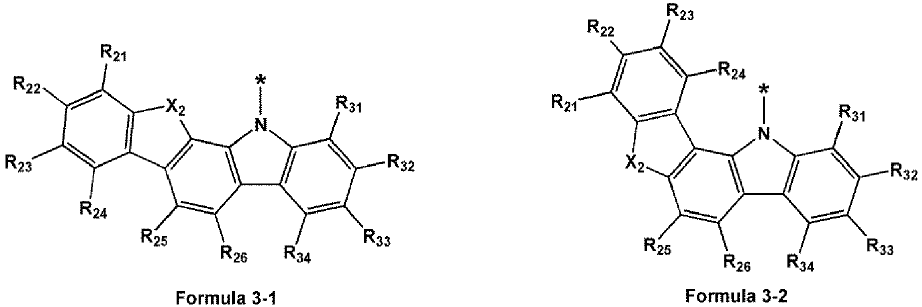 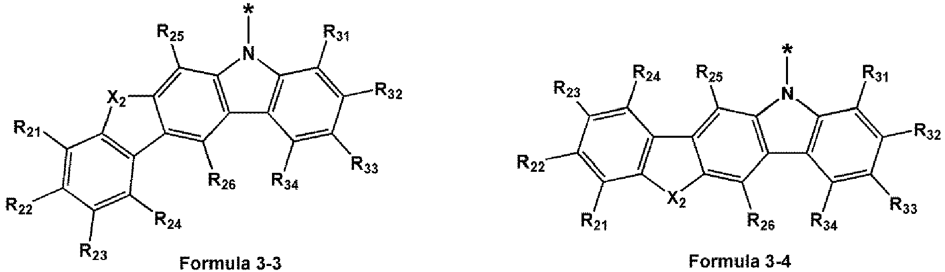 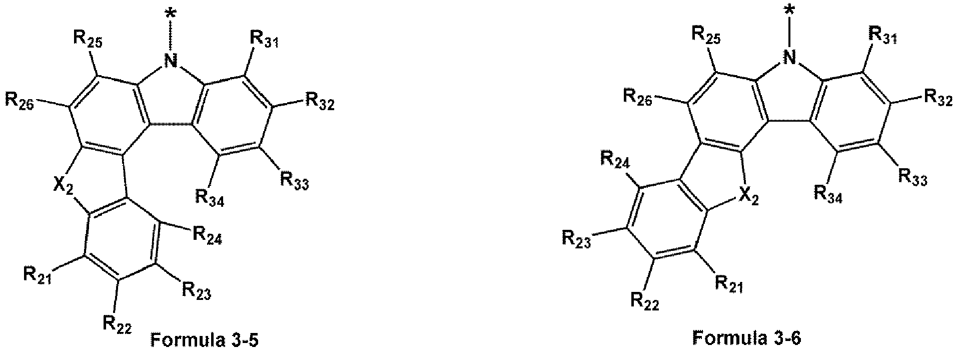 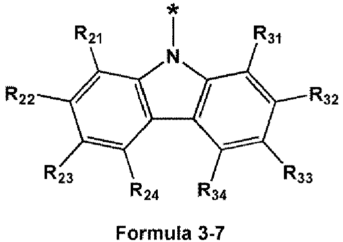 In Formulae 2-1 to 2-6 and 3-1 to 3-7,
X₁ is C(R₁₇)(R₁₈), N(R₁₉), O or S,
X₂ is C(R₂₇)(R₂₈), N(R₂₉), O or S,
descriptions of R₁ to R₄ are the same as provided herein,
descriptions of R₁₁ to R₁₉ are the same as the description of R₁₀,
descriptions of R₂₁ to R₂₉ are the same as the description of R₂₀,
descriptions of R₃₁ to R₃₄ are the same as the description of R₃₀, and
* is a binding site to a neighboring atom.

For example, R₁ to R₄, R₁₁ to R₁₉, R₂₁ to R₂₉ and R₃₁ to R₃₄ in Formulae 2-1 to 2-6 and 3-1 to 3-7 may be each independently selected from
a hydrogen, a deuterium, a hydroxyl group, a cyano group (CN), a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group and a C₁-C₂₀ alkoxy group;
a C₁-C₂₀ alkyl group and a C₁-C₂₀ alkoxy group, each substituted with at least one selected from a deuterium, a hydroxyl group, a cyano group (CN), a nitro group, an amino group, an amidino group, a hydrazine group, a hydrazone group, a carboxylic acid group or a salt thereof, a sulfonic acid group or a salt thereof, a phosphoric acid group or a salt thereof, a phenyl group, a biphenyl group, a terphenyl group and a naphthyl group;
a cyclopentyl group, a cyclohexyl group, a cyclopentenyl group, a cyclohexenyl group, a cycloheptenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spirobifluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyrimidinyl group, an imidazopyridinyl group, a pyridoindolyl group, a benzofuropyridinyl group, a benzothienopyridinyl group, a pyrimidoindolyl group, a benzofuropyrimidinyl group, a benzothienopyrimidinyl group, a phenoxazinyl group, a pyridobenzooxazinyl group and a pyridobenzothiazinyl group;
a cyclopentyl group, a cyclohexyl group, a cyclopentenyl group, a cyclohexenyl group, a cycloheptenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzoxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyrimidinyl group, an imidazopyridinyl group, a pyridoindolyl group, a benzofuropyridinyl group, a benzothienopyridinyl group, a pyrimidoindolyl group, a benzofuropyrimidinyl group, a benzothienopyrimidinyl group, a phenoxazinyl group, a pyridobenzooxazinyl group and a pyridobenzothiazinyl group, each substituted with at least one selected from a deuterium, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a biphenyl group, a terphenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a pyrazinyl group, a pyridazinyl group, a triazinyl group, a quinolinyl group, an isoquinolinyl group, a phthalazinyl group, a quinoxalinyl group, a cinnolinyl group, a quinazolinyl group, -Si(Q₃₁)(Q₃₂)(Q₃₃),-N(Q₃₄)(Q₃₅) and -B(Q₃₆)(Q₃₇); and
-Si(Q₁)(Q₂)(Q₃), -N(Q₄)(Q₅), and -B(Q₆)(Q₇),
wherein Q₁ to Q₇ and Q₃₁ to Q₃₇ are each independently selected from a hydrogen, a C₁-C₂₀ alkyl group, a C₁-C₂₀ alkoxy group, a phenyl group, a biphenyl group, a terphenyl group, a naphthyl group, a fluorenyl group, a carbazolyl group, a dibenzofuranyl group and a dibenzothiophenyl group.

In some embodiments, R₁ to R₄, R₁₁ to R₁₉, R₂₁ to R₂₉ and R₃₁ to R₃₄ in Formulae 2-1 to 2-6 and 3-1 to 3-7 may be each independently selected from
a hydrogen, a deuterium, a hydroxyl group, a cyano group (CN), a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group and a C₁-C₂₀ alkoxy group;
a C₁-C₂₀ alkyl group and a C₁-C₂₀ alkoxy group, each substituted with at least one selected from a deuterium, a hydroxyl group, a cyano group (CN), a nitro group, an amino group, an amidino group, a hydrazine group, a hydrazone group, a carboxylic acid group or a salt thereof, a sulfonic acid group or a salt thereof, a phosphoric acid group or a salt thereof, a phenyl group, a biphenyl group, a terphenyl group and a naphthyl group;
a cyclopentyl group, a cyclohexyl group, a cyclopentenyl group, a cyclohexenyl group, a cycloheptenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spirobifluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a carbazolyl group, a furanyl group, a benzofuranyl group, a thiophenyl group, a benzothiophenyl group, a dibenzofuranyl group, a dibenzothiophenyl group, a benzocarbazolyl group and a dibenzocarbazolyl group;
a cyclopentyl group, a cyclohexyl group, a cyclopentenyl group, a cyclohexenyl group, a cycloheptenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spirobifluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a carbazolyl group, a furanyl group, a benzofuranyl group, a thiophenyl group, a benzothiophenyl group, a dibenzofuranyl group, a dibenzothiophenyl group, a benzocarbazolyl group and a dibenzocarbazolyl group, each substituted with at least one selected from a deuterium, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a biphenyl group, a terphenyl group, a naphthyl group and -Si(Q₃₁)(Q₃₂)(Q₃₃); and
-Si(Q₁)(Q₂)(Q₃),
wherein Q₁ to Q₃ and Q₃₁ to Q₃₃ are each independently selected from a hydrogen, a C₁-C₂₀ alkyl group, a C₁-C₂₀ alkoxy group, a phenyl group, a biphenyl group, a terphenyl group and a naphthyl group.

In some embodiments, R₁ to R₄, R₁₁ to R₁₉, R₂₁ to R₂₉ and R₃₁ to R₃₄ in Formulae 2-1 to 2-6 and 3-1 to 3-7 may be each independently selected from
a hydrogen, a deuterium, a cyano group (CN), a C₁-C₂₀ alkyl group and a C₁-C₂₀ alkoxy group;
a C₁-C₂₀ alkyl group and a C₁-C₂₀ alkoxy group, each substituted with at least one selected from a deuterium, a cyano group (CN), a phenyl group, a biphenyl group, a terphenyl group and a naphthyl group;
a cyclopentyl group, a cyclohexyl group, a cyclopentenyl group, a cyclohexenyl group, a cycloheptenyl group, a phenyl group, a biphenyl group, a terphenyl group, a naphthyl group, a fluorenyl group, a carbazolyl group, a dibenzofuranyl group and a dibenzothiophenyl group;
a cyclopentyl group, a cyclohexyl group, a cyclopentenyl group, a cyclohexenyl group, a cycloheptenyl group, a phenyl group, a biphenyl group, a terphenyl group, a naphthyl group, a fluorenyl group, a carbazolyl group, a dibenzofuranyl group and a dibenzothiophenyl group, each substituted with at least one selected from a deuterium, a cyano group, a C₁-C₂₀ alkyl group, a C₁-C₂₀ alkoxy group, a phenyl group, a biphenyl group, a terphenyl group, a naphthyl group and -Si(Q₃₁)(Q₃₂)(Q₃₃); and
-Si(Q₁)(Q₂)(Q₃),
wherein Q₁ to Q₃ and Q₃₁ to Q₃₃ are each independently selected from a hydrogen, a C₁-C₂₀ alkyl group, a C₁-C₂₀ alkoxy group, a phenyl group, a biphenyl group, a terphenyl group and a naphthyl group, but embodiments are not limited thereto.

In some embodiments, X₁ in Formula 2-1 to 2-6 may be O or S, but embodiments are not limited thereto.

In some embodiments, X₁ and X₂ in Formulae 2-1 to 2-6 and Formulae 3-1 to 3-6 may each independently be O or S.

In some embodiments, each of CY₂ and CY₃ in Formula 3 may be a benzene group, but embodiments are not limited thereto.

In some embodiments,
Ar₁ may be represented by Formula 2-1, Ar₂ may be represented by Formula 3-1;
Ar₁ may be represented by Formula 2-2, Ar₂ may be represented by Formula 3-2; or
Ar₁ may be represented by Formula 2-6, and Ar₂ may be represented by Formula 3-6, but embodiments are not limited thereto.

According to an embodiment, Ar₁ in Formulae 1-1 to 1-3 is selected from groups represented by Formulae 2-1 to 2-6, and Ar₂ is selected from groups represented by Formulae 3-1 to 3-7.

In some embodiments, Ar₁ in Formulae 1-1 to 1-3 is selected from groups represented by Formulae 2-1 to 2-6, and Ar₂ is selected from groups represented by Formulae 3-1 to 3-7,
R₁ to R₄, R₁₁ to R₁₉, R₂₁ to R₂₉ and R₃₁ to R₃₄ are each independently selected from
a hydrogen, a deuterium, a hydroxyl group, a cyano group (CN), a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group and a C₁-C₂₀ alkoxy group;
C₁-C₂₀ alkyl group and C₁-C₂₀ alkoxy group, each substituted with at least one selected from a deuterium, a hydroxyl group, a cyano group (CN), a nitro group, an amino group, an amidino group, a hydrazine group, a hydrazone group, a carboxylic acid group or a salt thereof, a sulfonic acid group or a salt thereof, a phosphoric acid group or a salt thereof, a phenyl group, a biphenyl group, a terphenyl group and a naphthyl group;
a cyclopentyl group, a cyclohexyl group, a cyclopentenyl group, a cyclohexenyl group, a cycloheptenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spirobifluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a carbazolyl group, a furanyl group, a benzofuranyl group, a thiophenyl group, a benzothiophenyl group, a dibenzofuranyl group, a dibenzothiophenyl group, a benzocarbazolyl group and a dibenzocarbazolyl group;
a cyclopentyl group, a cyclohexyl group, a cyclopentenyl group, a cyclohexenyl group, a cycloheptenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spirobifluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a carbazolyl group, a furanyl group, a benzofuranyl group, a thiophenyl group, a benzothiophenyl group, a dibenzofuranyl group, a dibenzothiophenyl group, a benzocarbazolyl group and a dibenzocarbazolyl group, each substituted with at least one selected from a deuterium, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a biphenyl group, a terphenyl group, a naphthyl group and -Si(Q₃₁)(Q₃₂)(Q₃₃); and
-Si(Q₁)(Q₂)(Q₃),
R₄₁ to R₄₈ and R₅₁ to R₅₄ are each independently selected from
a hydrogen, a deuterium, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group and a C₁-C₂₀ alkoxy group;
a C₁-C₂₀ alkyl group and a C₁-C₂₀ alkoxy group, each substituted with at least one selected from a deuterium, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a phenyl group, a biphenyl group, a terphenyl group and a naphthyl group;
a cyclopentyl group, a cyclohexyl group, a cyclopentenyl group, a cyclohexenyl group, a cycloheptenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spirobifluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a carbazolyl group, a furanyl group, a benzofuranyl group, a thiophenyl group, a benzothiophenyl group, a dibenzofuranyl group, a dibenzothiophenyl group, a benzocarbazolyl group and a dibenzocarbazolyl group;
a cyclopentyl group, a cyclohexyl group, a cyclopentenyl group, a cyclohexenyl group, a cycloheptenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spirobifluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a carbazolyl group, a furanyl group, a benzofuranyl group, a thiophenyl group, a benzothiophenyl group, a dibenzofuranyl group, a dibenzothiophenyl group, a benzocarbazolyl group and a dibenzocarbazolyl group, each substituted with at least one selected from a deuterium, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a biphenyl group, a terphenyl group, a naphthyl group and -Si(Q₃₁)(Q₃₂)(Q₃₃); and
-Si(Q₁)(Q₂)(Q₃),
wherein Q₁ to Q₃ and Q₃₁ to Q₃₃ are each independently selected from a hydrogen, a C₁-C₂₀ alkyl group, a C₁-C₂₀ alkoxy group, a phenyl group, a biphenyl group, a terphenyl group and a naphthyl group.

In some embodiments, in Formulae 1-1 to 1-3, Ar₁ is selected from groups represented by Formulae 2-1 to 2-6, and Ar₂ is selected from groups represented by Formulae 3-1 to 3-7,
R₁ to R₄, R₁₁ to R₁₉, R₂₁ to R₂₉ and R₃₁ to R₃₄ are each independently selected from
a hydrogen, a deuterium, a cyano group (CN), a C₁-C₂₀ alkyl group and a C₁-C₂₀ alkoxy group;
a C₁-C₂₀ alkyl group and a C₁-C₂₀ alkoxy group, each substituted with at least one selected from a deuterium, a cyano group (CN), a phenyl group, a biphenyl group, a terphenyl group and a naphthyl group;
a cyclopentyl group, a cyclohexyl group, a cyclopentenyl group, a cyclohexenyl group, a cycloheptenyl group, a phenyl group, a biphenyl group, a terphenyl group, a naphthyl group, a fluorenyl group, a carbazolyl group, a dibenzofuranyl group and a dibenzothiophenyl group;
a cyclopentyl group, a cyclohexyl group, a cyclopentenyl group, a cyclohexenyl group, a cycloheptenyl group, a phenyl group, a biphenyl group, a terphenyl group, a naphthyl group, a fluorenyl group, a carbazolyl group, a dibenzofuranyl group and a dibenzothiophenyl group, each substituted with at least one selected from a deuterium, a cyano group, a C₁-C₂₀ alkyl group, a C₁-C₂₀ alkoxy group, a phenyl group, a biphenyl group, a terphenyl group, a naphthyl group and -Si(Q₃₁)(Q₃₂)(Q₃₃); and
-Si(Q₁)(Q₂)(Q₃),
R₄₁ to R₄₈ and R₅₁ to R₅₄ are each independently selected from
a hydrogen, a deuterium, a C₁-C₂₀ alkyl group and a C₁-C₂₀ alkoxy group;
a C₁-C₂₀ alkyl group and a C₁-C₂₀ alkoxy group, each substituted with at least one selected from a deuterium, a phenyl group, a biphenyl group, a terphenyl group and a naphthyl group;
a cyclopentyl group, a cyclohexyl group, a cyclopentenyl group, a cyclohexenyl group, a cycloheptenyl group, a phenyl group, a biphenyl group, a terphenyl group, a naphthyl group, a fluorenyl group, a carbazolyl group, a dibenzofuranyl group and a dibenzothiophenyl group;
a cyclopentyl group, a cyclohexyl group, a cyclopentenyl group, a cyclohexenyl group, a cycloheptenyl group, a phenyl group, a biphenyl group, a terphenyl group, a naphthyl group, a fluorenyl group, a carbazolyl group, a dibenzofuranyl group and a dibenzothiophenyl group, each substituted with at least one selected from a deuterium, a C₁-C₂₀ alkyl group, a C₁-C₂₀ alkoxy group, a phenyl group, a biphenyl group, a terphenyl group, a naphthyl group and -Si(Q₃₁)(Q₃₂)(Q₃₃); and
-Si(Q₁)(Q₂)(Q₃),
wherein Q₁ to Q₃ and Q₃₁ to Q₃₃ are each independently selected from a hydrogen, a C₁-C₂₀ alkyl group, a C₁-C₂₀ alkoxy group, a phenyl group, a biphenyl group, a terphenyl group and a naphthyl group, but embodiments are not limited thereto.

In some embodiments, in Formula 1-1 to 1-3, Ar₁ is selected from groups represented by Formulae 2-1 to 2-6 and Ar₂ is selected from groups represented by Formulae 3-1 to 3-7; and X₁ in Formulae 2-1 to 2-6 is O or S (for example, X₁ and X₂ in Formulae 2-1 to 2-6 and 3-1 to 3-6 may each independently be O or S).

In some embodiments, the condensed cyclic compound is represented by Formula 1-1, and in Formula 1-1, Ar₁ is selected from groups represented by Formulae 2-1 to 2-6 and Ar₂ is selected from groups represented by Formulae 3-1 to 3-7.

In some embodiments, the condensed cyclic compound is represented by Formula 1-1; in Formula 1-1, Ar₁ is selected from groups represented by Formulae 2-1 to 2-6 and Ar₂ is selected from groups represented by Formulae 3-1 to 3-7; and X₁ in Formulae 2-1 to 2-6 is O or S (for example, X₁ and X₂ in Formulae 2-1 to 2-6 and 3-1 to 3-6 may each independently be O or S).

The condensed cyclic compound may be selected from Compounds 1 to 108, but embodiments are not limited thereto:

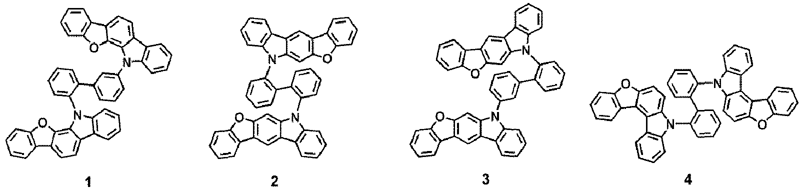

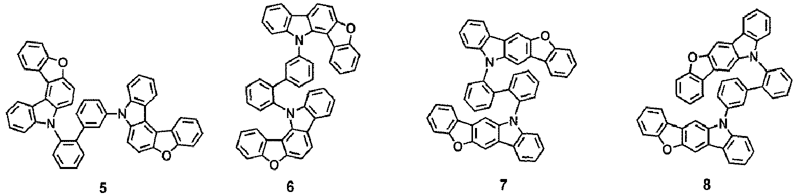

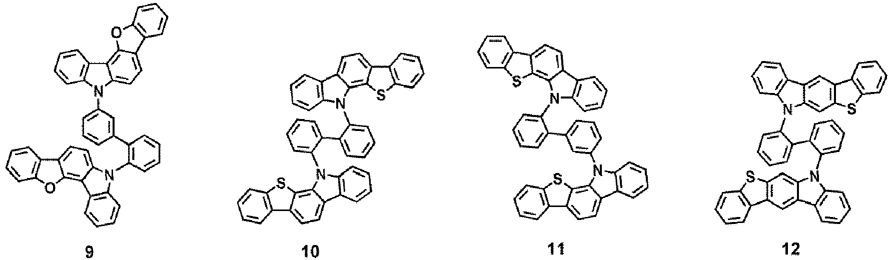

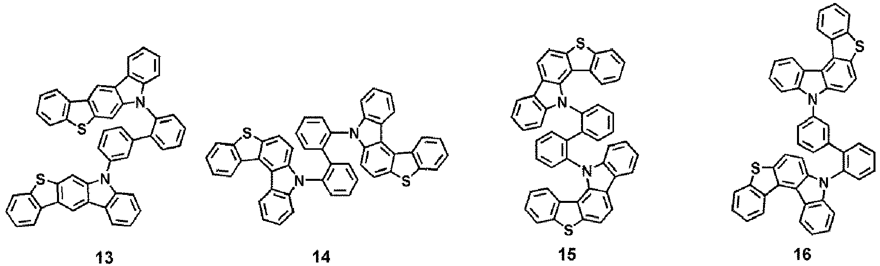

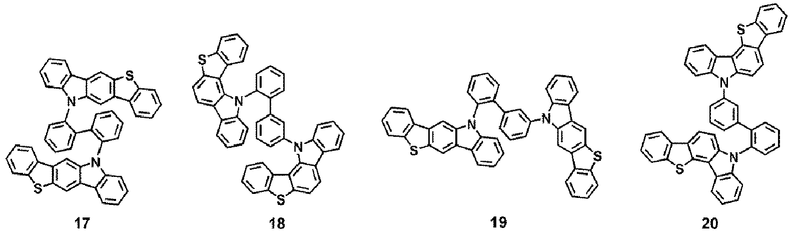

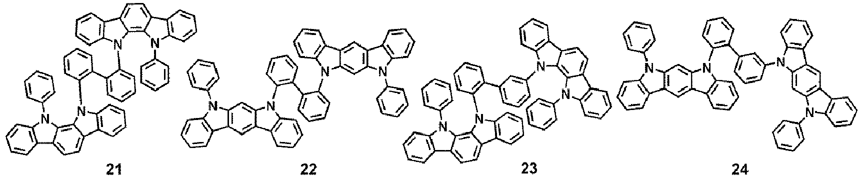

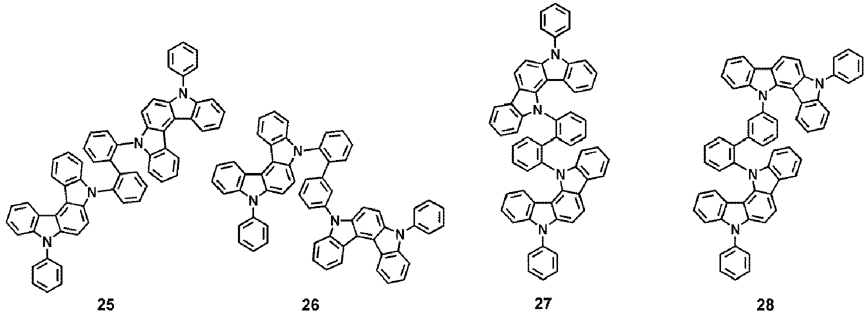

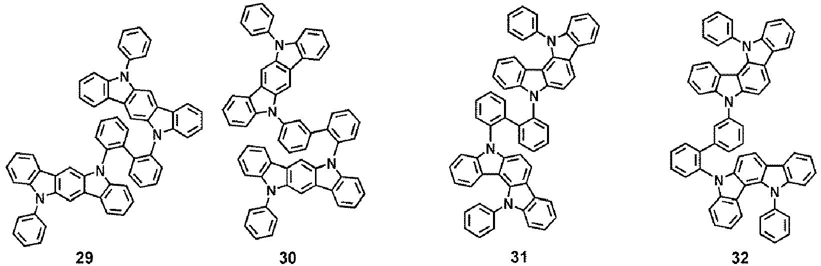

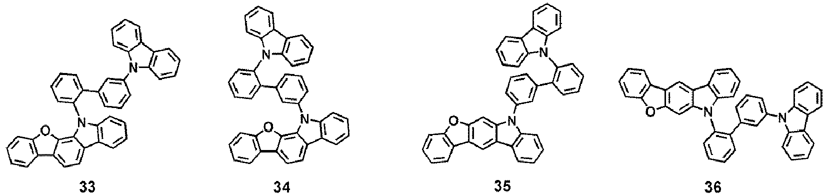

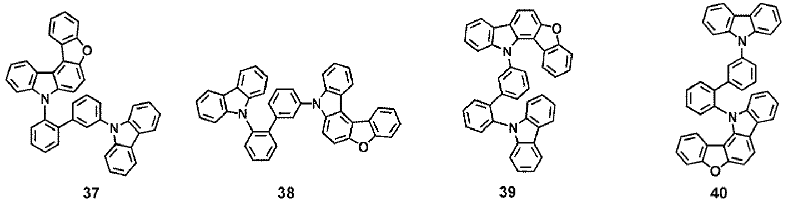

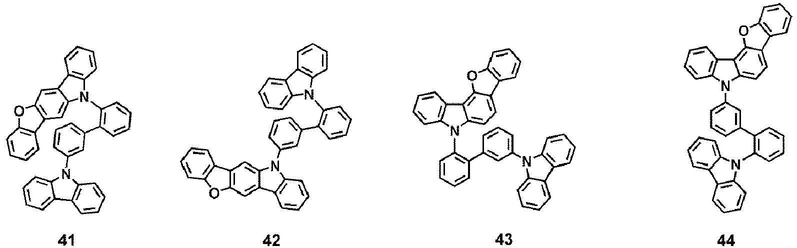

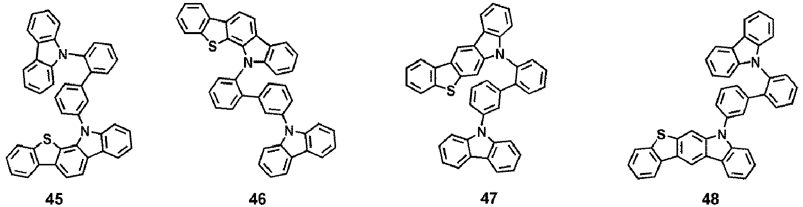

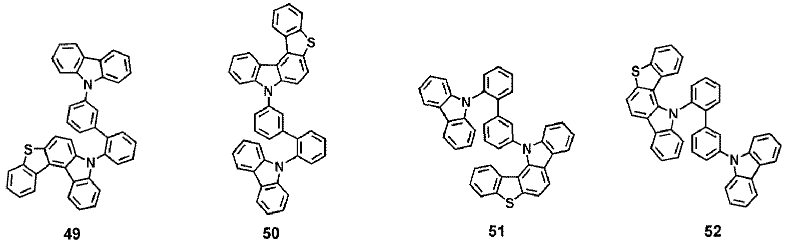

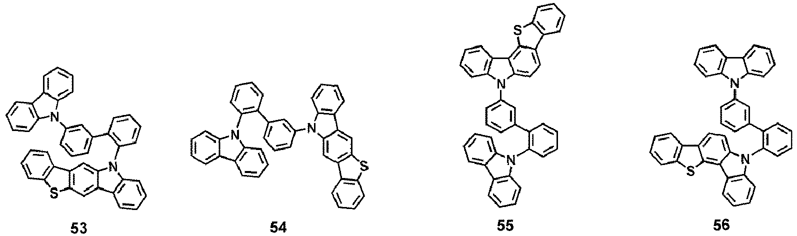

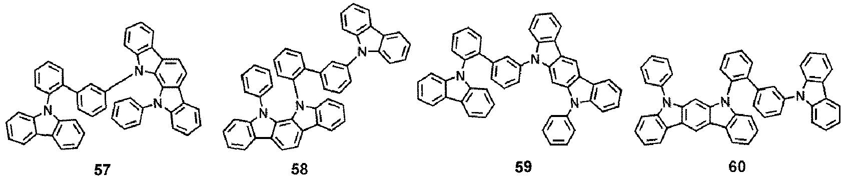

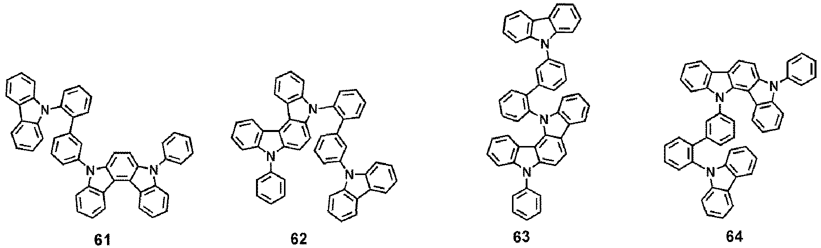

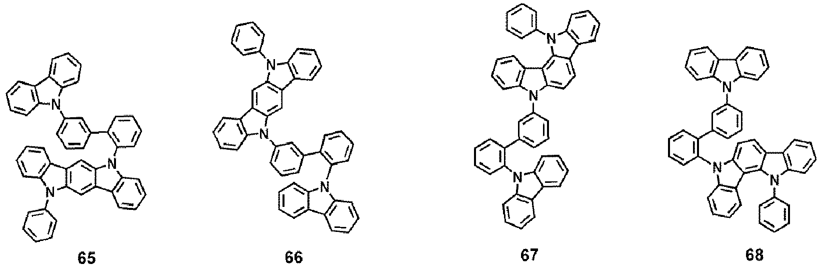

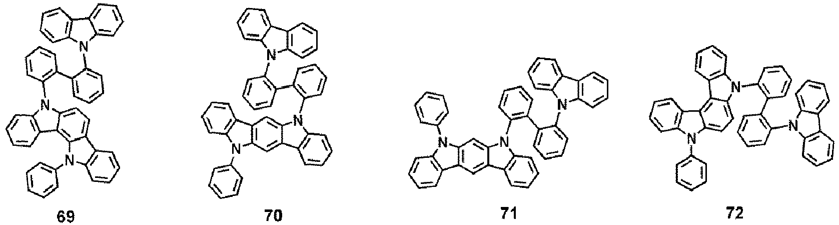

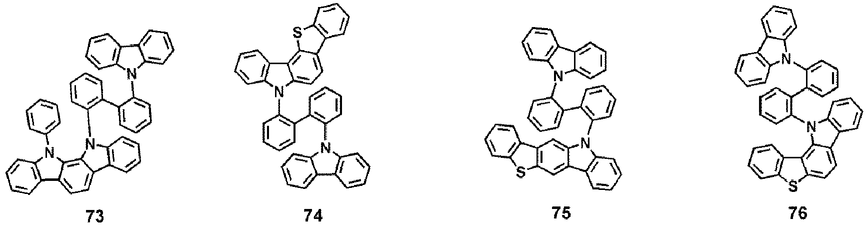

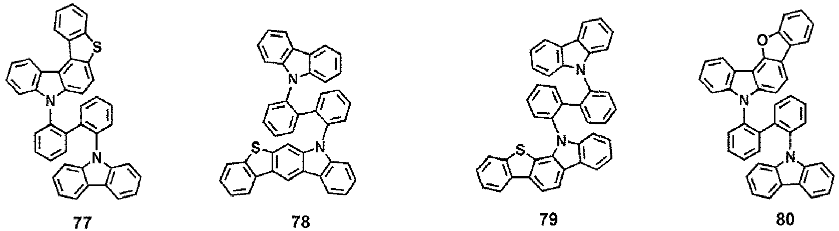

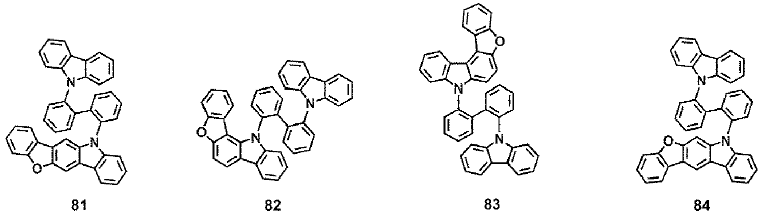

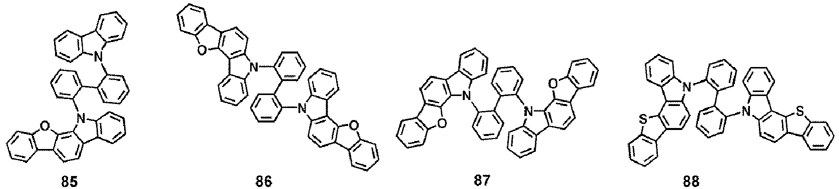

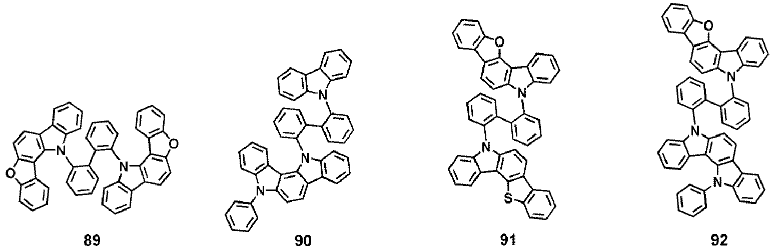

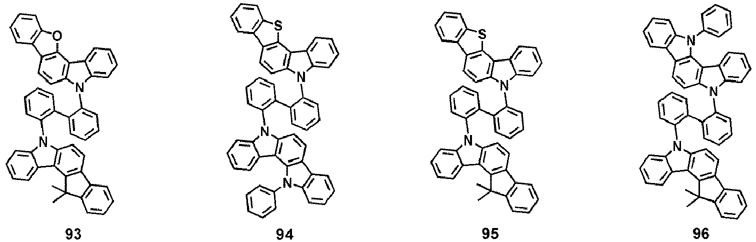

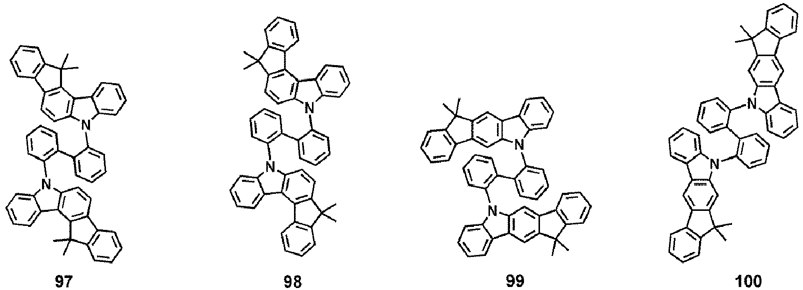

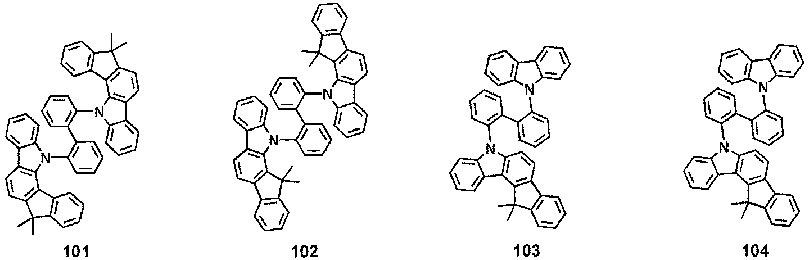

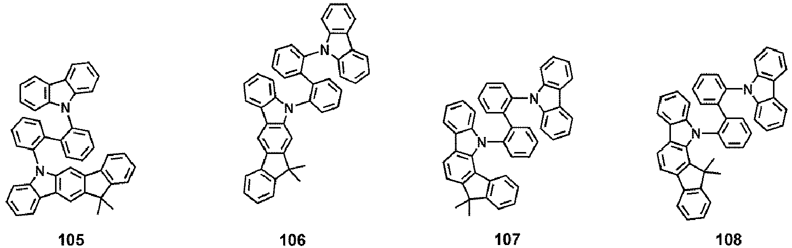

In Formulae 1-1 to 1-3, Ar₁ is a group represented by Formula 2 and Ar₂ is a group represented by Formula 3, and thus the condensed cyclic compound may have excellent thermal stability. As a result, an electronic device including the condensed cyclic compound, for example, an organic light-emitting device, may have long lifespan.

R₁ to R₄, R₁₀, R₂₀ and R₃₀ may be selected from a hydrogen, a deuterium, a hydroxyl group, a cyano group (CN), a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₂-C₆₀ heteroaryloxy group, a substituted or unsubstituted C₂-C₆₀ heteroarylthio group, a substituted or unsubstituted C₃-C₆₀ heteroarylalkyl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -N(Q₄)(Q₅), and -B(Q₆)(Q₇), and R₄₁₋₄₈ and R₅₁₋₅₄ may be selected from a hydrogen, a deuterium, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₂-C₆₀ heteroaryloxy group, a substituted or unsubstituted C₂-C₆₀ heteroarylthio group, a substituted or unsubstituted C₃-C₆₀ heteroarylalkyl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -N(Q₄)(Q₅), and -B(Q₆)(Q₇).

R₁ to R₄, R₁₀, R₂₀, R₃₀, R₄₁₋₄₈ and R₅₁₋₅₄ do not include "a halogen group including a fluoro group (-F)", and thus the condensed cyclic compound may have excellent heat resistance and thermal stability. Thus, when a thin film including the condensed cyclic compound is formed through a deposition process, the condensed cyclic compound may not decompose at a deposition temperature of a deposition process. As a result, an electronic device including the condensed cyclic compound, for example, an organic light-emitting device, may have long lifespan. In some embodiments, since R₁ to R₄, R₁₀, R₂₀, R₃₀, R₄₁₋₄₈ and R₅₁₋₅₄ may not include "a fluoro group (-F)", "Ar₁ and Ar₂" may have an orbital contributing HOMO energy level, and "L₁ and L₂" may have an orbital contributing to LUMO energy level, and thus the orbital contributing to HOMO energy level and the orbital contributing to LUMO energy level may be separated and respectively distributed in moieties having different structures. As a result, the condensed cyclic compound may have excellent charge transfer characteristics, and an electronic device including the condensed cyclic compound, for example, an organic light-emitting device, may present high efficiency.

For example, from the molecular orbital distribution of Compound 23 evaluated by using a Density Functional Theory (DFT) method including a Gaussian program structurally optimized at B3LYP/6-31G(d,p), it was found that B₁ and B₃ (an indolocarbazole ring) have an orbital contributing to HOMO energy level, and B₂ (a biphenylene group) has an orbital contributing to LUMO energy level. Thus the orbital contributing to HOMO energy level and the orbital contributing to LUMO energy level in a molecule of Compound 23 may be separated and respectively distributed in moieties having different structures.

### Compound 23

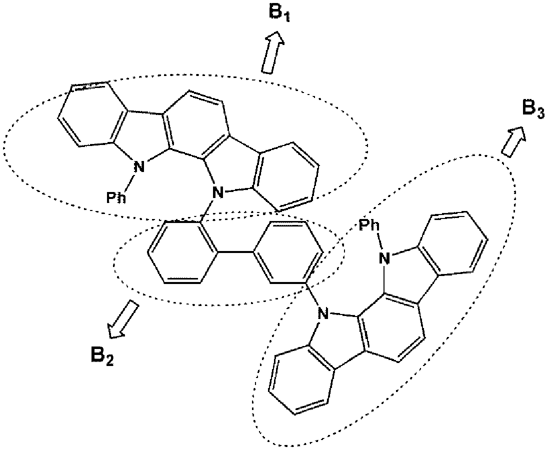

For comparison, from the molecular orbital distribution of Compound A evaluated by using a DFT method including a Gaussian program structurally optimized at B3LYP/6-31G(d,p), it was found that A₁ (an indolocarbazole ring substituted with -F) may have an orbital contributing to HOMO energy level and A₂ (an indolocarbazole ring substituted with -F) may have an orbital contributing to LUMO energy level. Thus, the orbital contributing to HOMO energy level and the orbital contributing to LUMO energy level may be separated and respectively distributed in moieties having the same structures. Compound A having the aforementioned orbital distribution may have poor charge transfer characteristics, and thus an electronic device, including Compound A, for example, an organic light-emitting device, may have low luminescent efficiency.

### Compound A

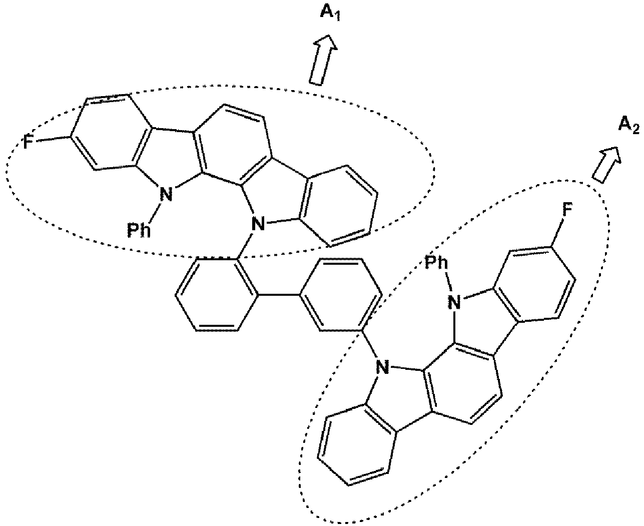

Ph in Compound A denotes a phenyl group.

Each of R₁ to R₄, R₁₀, R₂₀, R₃₀, R₄₀ and R₅₀ may not have "a halogen atom" having high electron affinity, and thus the condensed cyclic compound may have excellent hole transportation capability. As a result, an electronic device including the condensed cyclic compound, for example, an organic light-emitting device, may present high efficiency.

For example, HOMO, LUMO, and T₁ energy levels of Compounds 1 to 90 were evaluated through a simulation using a DFT method including a Gaussian program structurally optimized at B3LYP/6-31G(d,p). The results thereof are shown in Table 1 below:

**Table 1**

| Compound No. | T₁ (eV) | HOMO (eV) | LUMO (eV) |
|---|---|---|---|
| 1 | 2.957 | -5.27 | -0.972 |
| 2 | 2.924 | -5.285 | -1.146 |
| 3 | 2.907 | -5.188 | -1.12 |
| 4 | 2.835 | -5.328 | -1.254 |
| 5 | 2.832 | -5.183 | -1.157 |
| 6 | 2.984 | -5.255 | -1.191 |
| 7 | 2.771 | -5.348 | -1.281 |
| 8 | 2.78 | -5.204 | -1.211 |
| 9 | 3.005 | -5.162 | -1.142 |
| 10 | 2.892 | -5.249 | -1.137 |
| 11 | 2.9 | -5.175 | -1.126 |
| 12 | 2.87 | -5.292 | -1.196 |
| 13 | 2.862 | -5.196 | -1.148 |
| 14 | 2.802 | -5.286 | -1.299 |
| 15 | 2.913 | -5.434 | -1.324 |
| 16 | 2.801 | -5.133 | -1.199 |
| 17 | 2.757 | -5.276 | -1.298 |
| 18 | 2.919 | -5.26 | -1.154 |
| 19 | 2.762 | -5.093 | -1.272 |
| 20 | 2.97 | -5.214 | -1.16 |
| 21 | 2.908 | -5.099 | -1.043 |
| 22 | 2.824 | -5.012 | -1.199 |
| 23 | 2.906 | -5.047 | -0.972 |
| 24 | 2.824 | -4.894 | -1.016 |
| 25 | 2.734 | -4.904 | -1.019 |
| 27 | 2.967 | -4.975 | -1.038 |
| 28 | 2.957 | -4.885 | -1.019 |
| 29 | 2.679 | -4.86 | -1.057 |
| 30 | 2.668 | -4.802 | -1.032 |
| 31 | 2.966 | -5.035 | -1.024 |
| 32 | 2.958 | -4.952 | -1.06 |
| 33 | 2.96 | -5.249 | -1.015 |
| 34 | 2.961 | -5.278 | -0.996 |
| 35 | 2.906 | -5.168 | -1.083 |
| 36 | 2.923 | -5.25 | -1.093 |
| 37 | 2.84 | -5.251 | -1.135 |
| 38 | 2.83 | -5.181 | -1.107 |
| 39 | 2.982 | -5.238 | -1.202 |
| 40 | 2.984 | -5.255 | -1.143 |
| 41 | 2.78 | -5.266 | -1.189 |
| 42 | 2.781 | -5.175 | -1.112 |
| 43 | 3.013 | -5.236 | -1.084 |
| 44 | 3.006 | -5.161 | -1.084 |
| 45 | 2.908 | -5.174 | -1.08 |
| 46 | 2.899 | -5.236 | -1.067 |
| 47 | 2.873 | -5.252 | -1.107 |
| 48 | 2.86 | -5.17 | -1.094 |
| 49 | 2.808 | -5.207 | -1.176 |
| 50 | 2.799 | -5.141 | -1.126 |
| 51 | 2.919 | -5.257 | -1.101 |
| 52 | 2.925 | -5.268 | -1.176 |
| 53 | 2.762 | -5.2 | -1.206 |
| 54 | 2.761 | -5.094 | -1.125 |
| 55 | 2.972 | -5.207 | -1.107 |
| 56 | 2.975 | -5.268 | -1.092 |
| 57 | 2.923 | -5.006 | -0.955 |
| 58 | 2.909 | -5.164 | -1.108 |
| 59 | 2.824 | -4.919 | -1.042 |
| 60 | 2.833 | -4.977 | -4.015 |
| 61 | 2.744 | -4.821 | -1.03 |
| 62 | 2.735 | -4.848 | -1.03 |
| 63 | 2.974 | -4.965 | -1.042 |
| 64 | 2.962 | -4.92 | -1.017 |
| 65 | 2.683 | -4.846 | -1.07 |
| 66 | 2.678 | -4.789 | -1.001 |
| 67 | 2.966 | -4.926 | -1.028 |
| 68 | 2.961 | -4.976 | -1.069 |
| 69 | 2.967 | -5.07 | -1.041 |
| 70 | 2.688 | -4.904 | -1.06 |
| 71 | 2.835 | -5.059 | -1.06 |
| 72 | 2.732 | -4.944 | -1.035 |
| 73 | 2.913 | -5.13 | -1.155 |
| 74 | 2.97 | -5.354 | -1.127 |
| 75 | 2.757 | -5.267 | -1.275 |
| 76 | 2.93 | -5.357 | -1.195 |
| 77 | 2.806 | -5.286 | -1.264 |
| 78 | 2.875 | -5.312 | -1.106 |
| 80 | 3.008 | -5.307 | -1.112 |
| 81 | 2.772 | -5.343 | -1.261 |
| 82 | 2.984 | -5.383 | -1.151 |
| 83 | 2.838 | -5.319 | -1.194 |
| 84 | 2.925 | -5.32 | -1.114 |
| 85 | 5.953 | -5.355 | -1.11 |
| 86 | 3.006 | -5.308 | -1.153 |
| 87 | 2.952 | -5.351 | -1.091 |
| 88 | 2.97 | -5.363 | -1.18 |
| 89 | 2.984 | -5.393 | -1.244 |
| 90 | 2.969 | -5.029 | -1.04 |

Table 1 shows that Compounds 1 to 90 have excellent electric characteristics, for example, high T₁ energy level.

A method of synthesizing the condensed cyclic compound may be understood by ordinary skill in the art by referring to Synthesis Examples.

In this regard, the condensed cyclic compound may be suitable as a material of an organic layer of an organic light-emitting device, for example, a material for a hole transport layer, a material for an electron blocking layer and/or a host of an emission layer. According to another aspect, provided is an organic light-emitting device that includes:
a first electrode;
a second electrode; and
an organic layer disposed between the first electrode and the second electrode,
wherein the organic layer includes an emission layer, and
wherein the organic layer includes at least one of the condensed cyclic compounds.

While not wishing to be bound by theory, it is understood that when the organic light-emitting device includes the organic layer including the condensed cyclic compoundas described above, the organic light-emitting device has low driving voltage, high power efficiency, high luminous efficiency, high quantum luminance efficiency, and long lifespan.

For example, the condensed cyclic compound may be included in the emission layer.

In some embodiments, the condensed cyclic compound may be included in the emission layer, and the condensed cyclic compound may be a material for delayed fluorescence.

According to an embodiment, the emission layer may include a host and a dopant (wherein an amount of the host is greater than an amount of the dopant), and the host may include the condensed cyclic compound. The condensed cyclic compound serving as a host may transfer energy to a dopant according to a delayed fluorescence emission mechanism. The dopant may include at least one of a fluorescent dopant and a phosphorescent dopant. The dopant may be selected from known dopants. The host may further include any suitable host selected from known hosts.

In some embodiments, the emission layer may include a host and a dopant (an amount of the host is greater than an amount of the dopant), and the dopant may include the condensed cyclic compound. The condensed cyclic compound as a dopant may emit delayed fluorescence according to a delayed fluorescence emission mechanism. The host may be selected from known dopants.

The emission layer may emit red, green or blue light.

According to an embodiment, the emission layer may be a blue emission layer including a phosphorescent dopant, but embodiments are not limited thereto.

In some embodiments, the condensed cyclic compound may be included in a hole transport region.

For example, the hole transport region of the organic light-emitting device may include at least one of a hole transport layer and an electron blocking layer, and at least one of the hole transport layer and the electron blocking layer may include the condensed cyclic compound.

According to an embodiment, a hole transport region of the organic light-emitting device may include a hole transport layer, and the hole transport layer may include the condensed cyclic compound.

In some embodiments, a hole transport region of the organic light-emitting device may include an electron blocking layer, and the electron blocking layer may include the condensed cyclic compound. The electron blocking layer may directly contact the emission layer.

The expression as used herein "(an organic layer) includes at least one condensed cyclic compound" may be understood as "(organic layer) may include one condensed cyclic compound or two or more different condensed cyclic compounds".

For example, the organic layer may include only Compound 1 as the condensed cyclic compound. In this regard, Compound 1 may be included in the emission layer of the organic light-emitting device. Alternatively, the organic layer may include Compound 1 and Compound 2 as the condensed cyclic compounds. In this regard, Compound 1 and Compound 2 may be included in the same layer (for example, both Compound 1 and Compound 2 may be included in the emission layer), or in different layers (for example, Compound 1 may be included in the emission layer and Compound 2 may be included in the electron blocking layer).

The first electrode may be an anode, which is a hole injection electrode, and the second electrode may be a cathode, which is an electron injection electrode. Alternatively, the first electrode may be a cathode, which is an electron injection electrode, and the second electrode may be an anode, which is a hole injection electrode.

For example, in the organic light-emitting device,
the first electrode may be an anode,
the second electrode may be a cathode, and
the organic layer may include a hole transport region disposed between the first electrode and the emission layer,
wherein an electron transport region may be disposed between the emission layer and the second electrode,
the hole transport region may include at least one selected from a hole injection layer, a hole transport layer and an electron blocking layer, and
the electron transport region may include at least one selected from a hole blocking layer, an electron transport layer and an electron injection layer.

As used herein, the term the "organic layer" refers to a single and/or a plurality of layers disposed between the first electrode and the second electrode in an organic light-emitting device. The "organic layer" may include not only organic compounds but also organometallic complexes including metals.

FIG. 1 is a schematic view of an organic light-emitting device 10 according to an embodiment of the present disclosure. Hereinafter, a structure and a method of manufacturing an organic light-emitting device according to an embodiment of the present disclosure will be described with reference to FIG. 1. The organic light-emitting device 10 includes a first electrode 11, an organic layer 15, and a second electrode 19, which are sequentially layered in the stated order.

A substrate may be additionally disposed under the first electrode 11 or on the second electrode 19. The substrate may be a conventional substrate that is used in an organic light-emitting device, such as glass substrate or a transparent plastic substrate, each having excellent mechanical strength, thermal stability, transparency, surface smoothness, ease of handling, and water repellency.

The first electrode 11 may be formed by vacuum-depositing or sputtering a material for forming the first electrode 11 on the substrate. The first electrode 11 may be an anode. The material for the first electrode 11 may be selected from materials with a high work function for easy hole injection. The first electrode 11 may be a reflective electrode, a semi-transmissive electrode, or a transmissive electrode. The material for the first electrode 11 may be selected from indium tin oxide (ITO), indium zinc oxide (IZO), tin oxide (SnO₂), and zinc oxide (ZnO). Alternatively, a metal such as magnesium (Mg), aluminum (Al), aluminum-lithium (Al-Li), calcium (Ca), magnesium-indium (Mg-In), and magnesium-silver (Mg-Ag) may be used herein.

The first electrode 11 may have a single layer structure or a multi-layer structure including a plurality of layers. For example, the first electrode 11 may have a triple-layer structure of ITO/Ag/ITO, but embodiments are not limited thereto.

The organic layer 15 is disposed on the first electrode 11.

The organic layer 15 may include a hole transport region, an emission layer, and an electron transport region.

The hole transport region may be disposed between the first electrode 11 and the emission layer.

The hole transport region may include at least one selected from a hole injection layer, a hole transport layer, an electron blocking layer, and a buffer layer.

The hole transport region may only include a hole injection layer or a hole transport layer. Alternatively, the hole transport region may include a structure in which a hole injection layer/a hole transport layer or a hole injection layer/a hole transport layer/an electron blocking layer are sequentially layered on the first electrode 11.

When the hole transport region includes a hole injection layer, the hole injection layer may be formed on the first electrode 11 by using various methods such as vacuum-deposition, spin coating, casting, and a Langmuir-Blodgett (LB) method.

When a hole injection layer is formed by vacuum-deposition, though the conditions may vary depending on a compound that is used as a hole injection material and a structure and thermal properties of a desired hole injection layer, for example, the vacuum-deposition may be performed at a deposition temperature in a range of about 100°C to about 500°C, at a vacuum degree in a range of about 10⁻⁸ to about 10⁻³ torr, and at a deposition rate in a range of about 0.001 nm/sec to about 10 nm/sec (about 0.01 Angstroms per second (Å/sec) to about 100 Å/sec), but embodiments are not limited thereto.

When a hole injection layer is formed by spin coating, though the conditions may vary depending on a compound that is used as a hole injection material and a structure and thermal properties of a desired hole injection layer, the spin coating may be performed at a coating rate in a range of about 2,000 revolutions per minute (rpm) to about 5,000 rpm, and at a temperature in a range of about 80°C to 200°C for removing a solvent after the spin coating, but embodiments are not limited thereto.

The conditions for forming a hole transport layer and an electron blocking layer may be inferred based on the conditions for forming the hole injection layer.

The hole transport region may include at least one selected from m-MTDATA, TDATA, 2-TNATA, NPB, β-NPB, TPD, spiro-TPD, spiro-NPB, methylated-NPB, TAPC, HMTPD, 4,4',4"-tris(N-carbazolyl)triphenylamine (TCTA), polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), (polyaniline)/poly(4-styrenesulfonate) (PANI/PSS), a compound represented by Formula 201 below, and a compound represented by Formula 202 below: 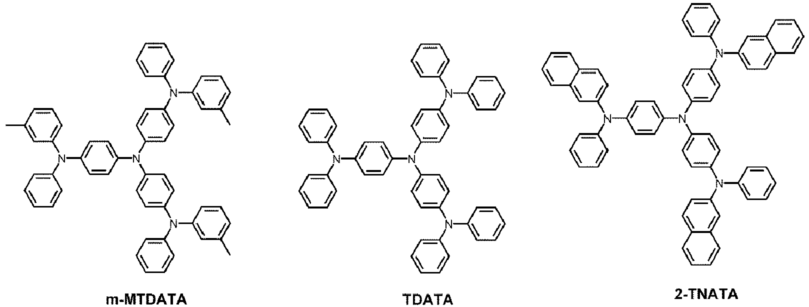 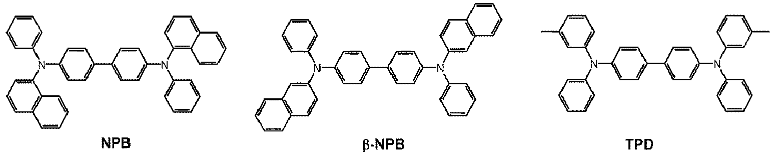 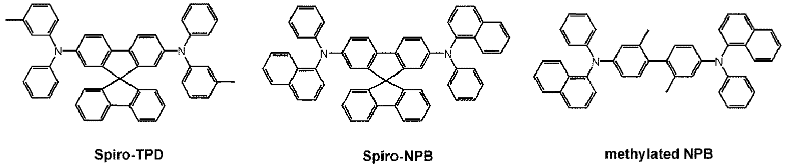 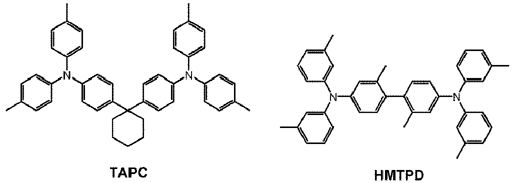 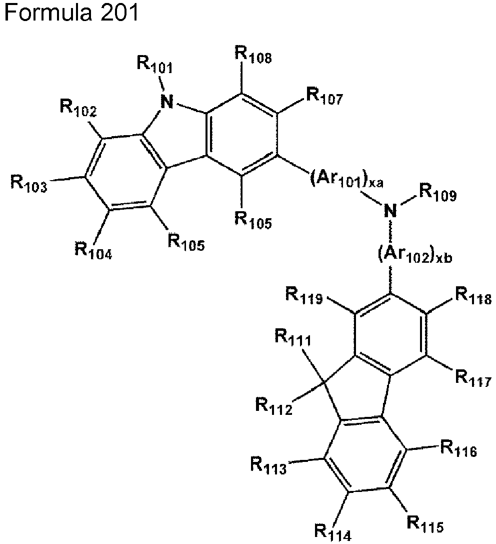 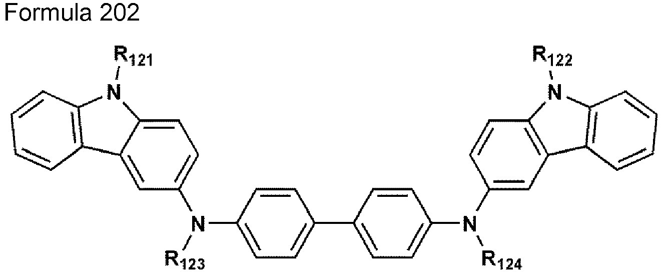
Ar₁₀₁ and Ar₁₀₂ in Formula 201 may be each independently selected from
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group and a pentacenylene group; and
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group and a pentacenylene group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ cycloalkenyl group, a C₁-C₁₀ heterocycloalkyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₂-C₆₀ heteroaryloxy group, a C₂-C₆₀ heteroarylthio group, a C₃-C₆₀ heteroarylalkyl group, a monovalent non-aromatic condensed polycyclic group and a monovalent non-aromatic condensed heteropolycyclic group.
xa and xb in Formula 201 may each independently be an integer of 0 to 5, or may be 0, 1 or 2. For example, xa may be 1 and xb may be 0, but embodiments are not limited thereto.
R₁₀₁ to R₁₀₈, R₁₁₁ to R₁₁₉ and R₁₂₁ to R₁₂₄ in Formulae 201 and 202 may be each independently selected from
a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group (for example, a methyl group, an ethyl group, a propyl group, a butyl group, pentyl group, a hexyl group, etc.) and a C₁-C₆₀ alkoxy group (for example, a methoxy group, an ethoxy group, a propoxy group, butoxy group, a pentoxy group, etc.);
a C₁-C₆₀ alkyl group and a C₁-C₆₀ alkoxy group, each substituted with at least one selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof and a phosphoric acid group or a salt thereof;
a phenyl group, a naphthyl group, an anthracenyl group, a fluorenyl group and a pyrenyl group; and
a phenyl group, a naphthyl group, an anthracenyl group, a fluorenyl group and a pyrenyl group, each substituted with at least one selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group and a C₁-C₆₀ alkoxy group,
but embodiments are not limited thereto.
R₁₀₉ in Formula 201 may be selected from
a phenyl group, a naphthyl group, an anthracenyl group and a pyridinyl group; and
a phenyl group, a naphthyl group, an anthracenyl group and a pyridinyl group, each substituted with at least one selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, an anthracenyl group and a pyridinyl group.

According to an embodiment, the compound represented by Formula 201 may be represented by Formula 201A, but embodiments are not limited thereto:

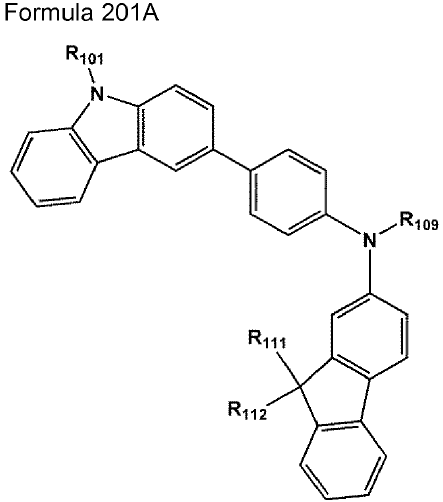

Descriptions of R₁₀₁, R₁₁₁, R₁₁₂ and R₁₀₉ in Formula 201A are the same as provided herein.

For example, the compound represented by Formula 201 and the compound represented by Formula 202 may include Compounds HT1 to HT20, but embodiments are not limited thereto:

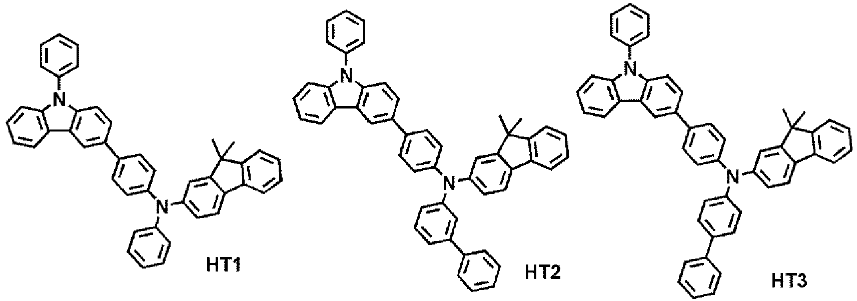

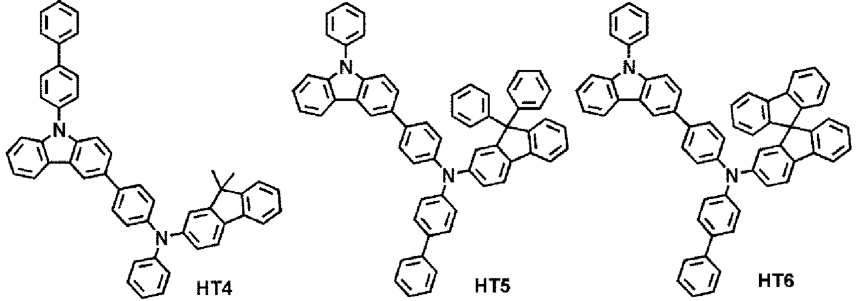

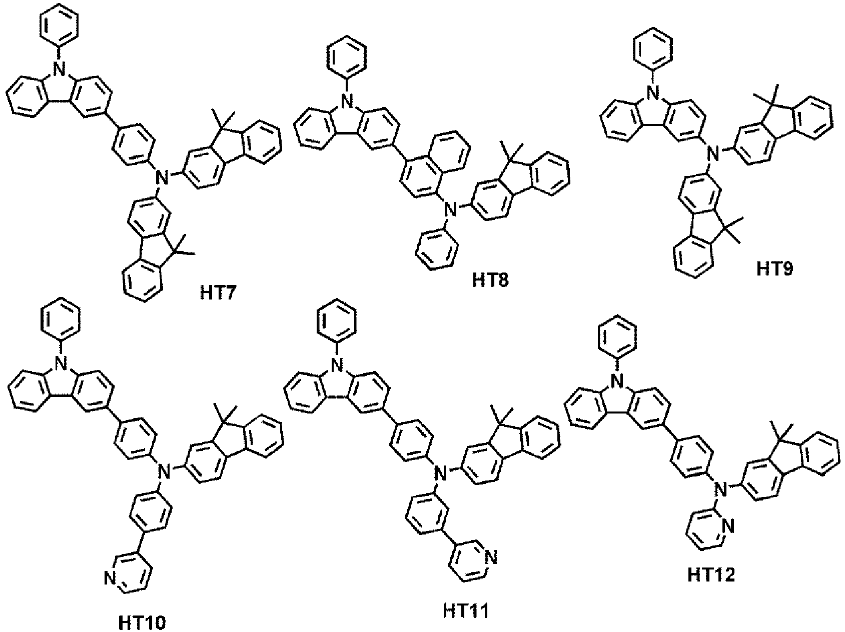

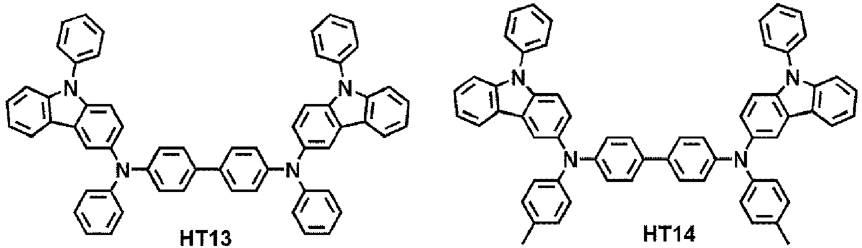

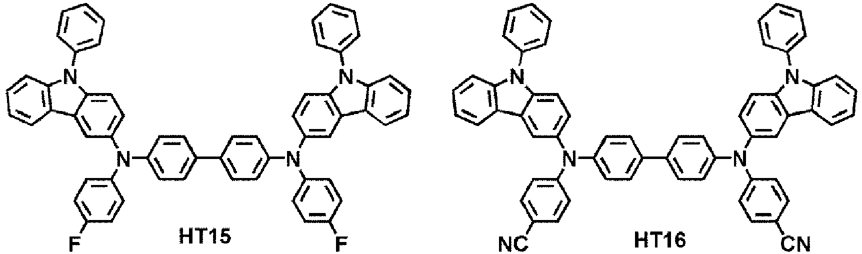

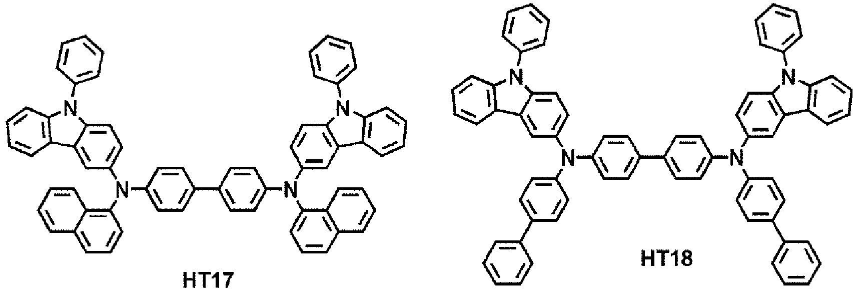

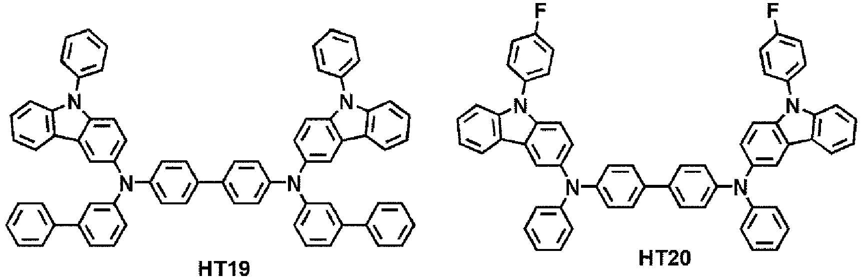

In some embodiments, the hole transport layer may include the condensed cyclic compound.

A thickness of the hole transport region may be in a range of about 10 nm to about 1,000 nm (about 100 Angstroms (Å) to about 10,000 Å), for example, about 10 nm (about 100 Å) to about 100 nm (about 1,000 Å). When the hole transport region includes at least one of a hole injection layer and a hole transport layer, the thickness of the hole injection layer may be in a range of about 10 nm (about 100 Å) to about 1,000 nm (about 10,000 Å), for example, about 10 nm (about 100 Å) to about 100 nm (about 1,000 Å), and the thickness of the hole transport layer may be in a range of about 5nm (about 50 Å) to about 200 nm (about 2,000 Å), for example, about 10 nm (about 100 Å) to about 150 nm (about 1,500 Å). While not wishing to be bound by theory, it is understood that when the thicknesses of the hole transport region, the hole injection layer, and the hole transport layer are within these ranges, excellent hole transport characteristics may be obtained without a substantial increase in driving voltage.

The hole transport region may further include, in addition to the mentioned materials above, a charge-generating material to improve conductive properties. The charge-generating material may be homogeneously or non-homogeneously dispersed throughout the hole transport region.

The charge-generating material may be, for example, a p-dopant. The p-dopant may be one selected from a quinone derivative, a metal oxide, and a cyano group-containing compound, but embodiments are not limited thereto. For example, non-limiting examples of the p-dopant are a quinone derivative, such as tetracyanoquinonedimethane (TCNQ) or 2,3,5,6-tetrafluoro-tetracyano-1,4-benzoquinonedimethane (F4-TCNQ); a metal oxide, such as a tungsten oxide or a molybdenum oxide; and a compound containing a cyano group, such as Compound HT-D1 and HP-1, but embodiments are not limited thereto.

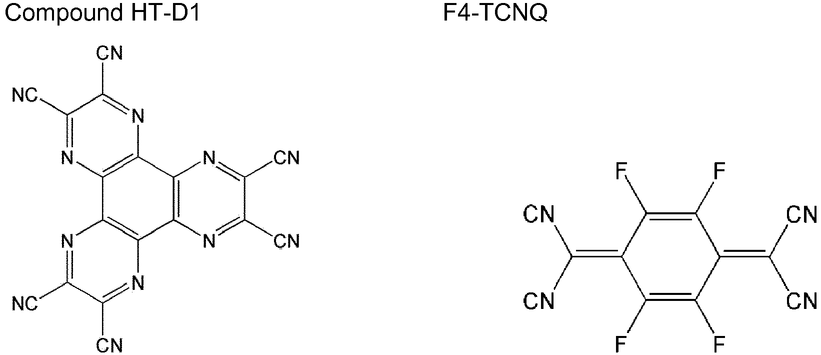

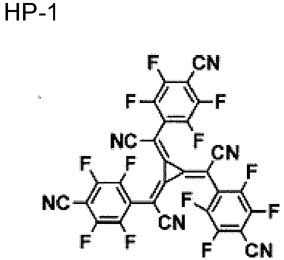

The hole transport region may further include a buffer layer.

The buffer layer may compensate for an optical resonance distance according to a wavelength of light emitted from the emission layer to improve the efficiency of an organic light-emitting device.

An emission layer may be formed on the hole transport region by using various methods, such as vacuum-deposition, spin coating, casting, or an LB method. When the emission layer is formed by vacuum-deposition or spin coating, vacuum-deposition and coating conditions for the emission layer may be generally similar to the conditions for forming a hole injection layer, though the conditions may vary depending on the compound used.

The hole transport region may further include an electron blocking layer. The electron blocking layer may include a known material, for example, mCP, but embodiments are not limited thereto.

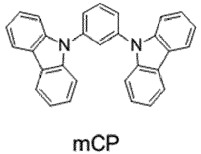

In some embodiments, the hole transport region may include an electron blocking layer, and the electron blocking layer may include the condensed cyclic compound.

A thickness of the electron blocking layer may be in a range of about 5 nm (about 50 Å) to about 100 nm (about 1,000 Å), for example, about 7 nm (about 70 Å) to about 50 nm (about 500 Å). While not wishing to be bound by theory, it is understood that when the thickness of the electron blocking layer is within this range, excellent electron blocking characteristics may be obtained without a substantial increase in driving voltage.

When the organic light-emitting device is a full color organic light-emitting device, the emission layer may be patterned into a red emission layer, a green emission layer, and a blue emission layer. Alternatively, the emission layer may have a structure in which the red emission layer, the green emission layer, and/or the blue emission layer are layered to emit white light or other various embodiments are possible.

The emission layer may include the condensed cyclic compound. For example, the emission layer may only include the condensed cyclic compound. In some embodiments, the emission layer may include a host and a dopant, and the host may include the condensed cyclic compound. In some embodiments, the emission layer may include a host and a dopant, and the dopant may include the condensed cyclic compound.

According to an embodiment, a dopant in the emission layer may be a phosphorescent dopant, and the phosphorescent dopant may include an organometallic compound represented by Formula 81:

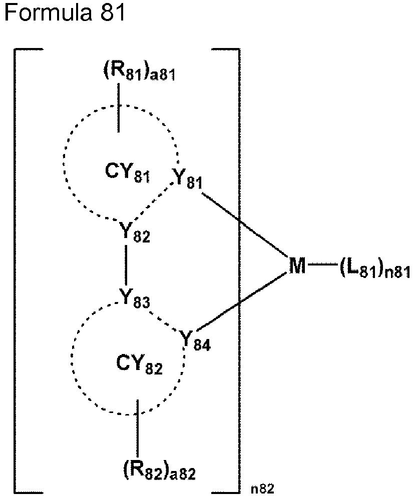

In Formula 81,
M may be selected from iridium (Ir), platinum (Pt), osmium (Os), titanium (Ti), zirconium (Zr), hafnium (Hf), europium (Eu), terbium (Tb), palladium (Pd) and thulium (Tm),
Y₈₁ to Y₈₄ may each independently be carbon (C) or nitrogen (N),
Y₈₁ and Y₈₂ may be linked to each other by a single bond or a double bond, and Y₈₃ and Y₈₄ may be linked to each other by a single bond or a double bond,
CY₈₁ and CY₈₂ may be each independently selected from a benzene group, a naphthalene group, a fluorene group, a spiro-bifluorene group, an indene group, a pyrrole group, a thiophene group, a furan group, an imidazole group, a pyrazole group, a thiazole group, an isothiazole group, an oxazole group, an isooxazole group, a pyridine group, a pyrazine group, a pyrimidine group, a pyridazine group, a quinoline group, an isoquinoline group, a benzoquinoline group, a quinoxaline group, a quinazoline group, a carbazole group, a benzoimidazole group, a benzofuran group, a benzothiophene group, an isobenzothiophene group, a benzoxazole group, an isobenzoxazole group, a triazole group, a tetrazole group, an oxadiazole group, a triazine group, a dibenzofuran group or a dibenzothiophene group, and CY₈₁ and CY₈₂ may be optionally further linked to each other by an organic linking group,
R₈₁ and R₈₂ may be each independently selected from a hydrogen, a deuterium,-F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₂-C₆₀ heteroaryloxy group, a substituted or unsubstituted C₂-C₆₀ heteroarylthio group, a substituted or unsubstituted C₃-C₆₀ heteroarylalkyl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), and -B(Q₆)(Q₇),
a81 and a82 may each independently be an integer of 1 to 5,
n81 may be an integer of 0 to 4,
n82 may be 1, 2 or 3,
L₈₁ may be a monovalent organic ligand, a divalent organic ligand or a trivalent organic ligand, and
descriptions for Q₁ to Q₇ are same as descriptions for Q₁ to Q₃ in -Si(Q₁)(Q₂)(Q₃)
Descriptions of R₈₁ and R₈₂ are the same as the description of R₁₁.

The phosphorescent dopant may include at least one selected from Compounds PD1 to PD78 or FIr₆, but embodiments are not limited thereto:

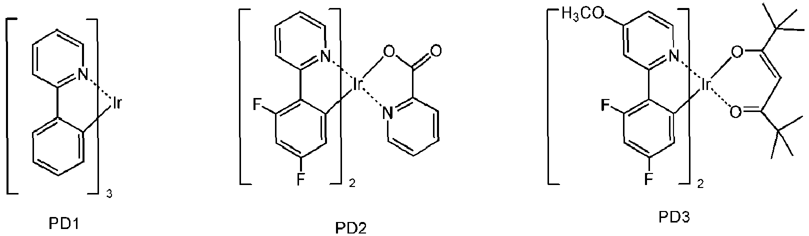

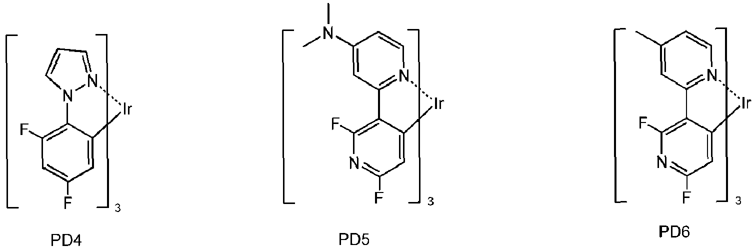

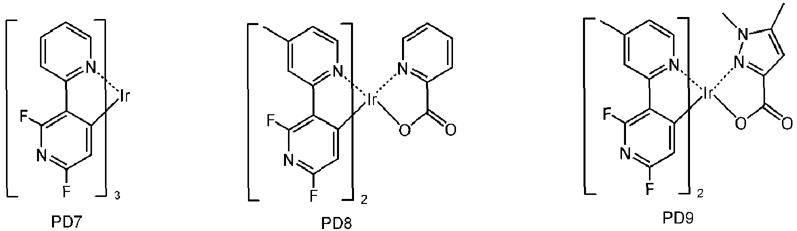

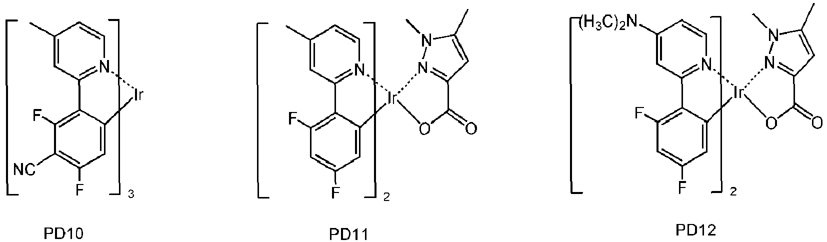

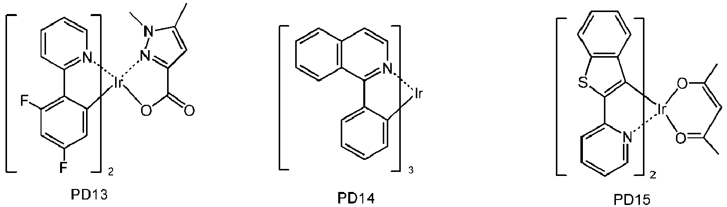

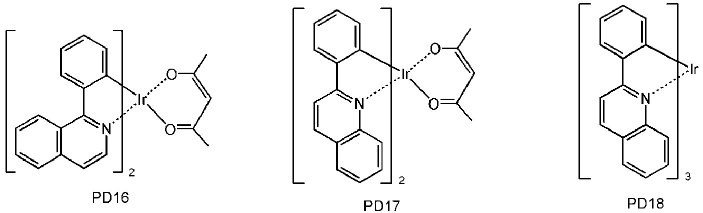

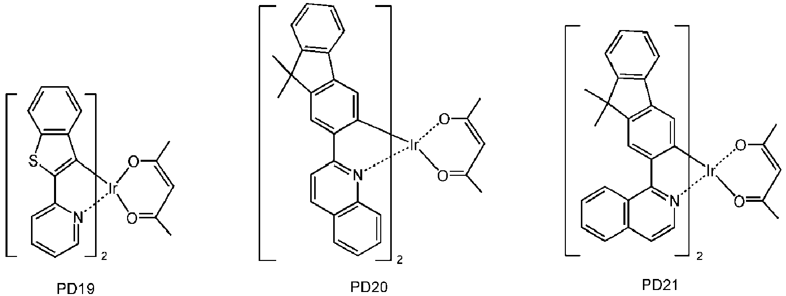

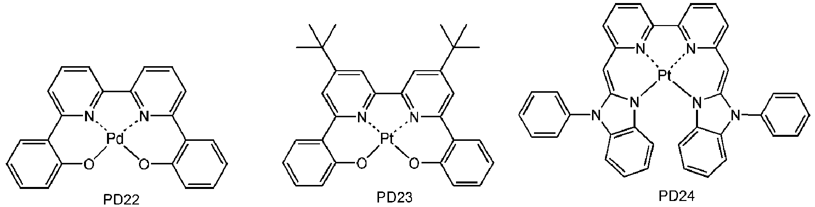

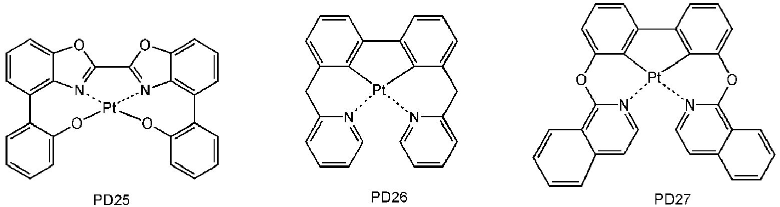

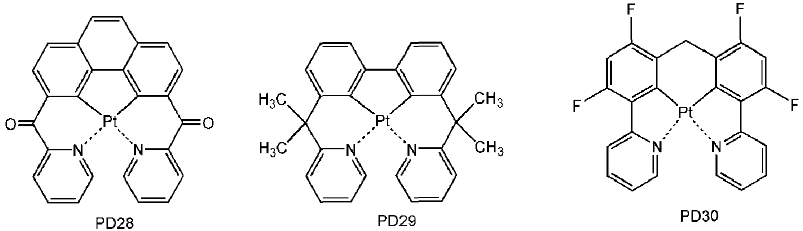

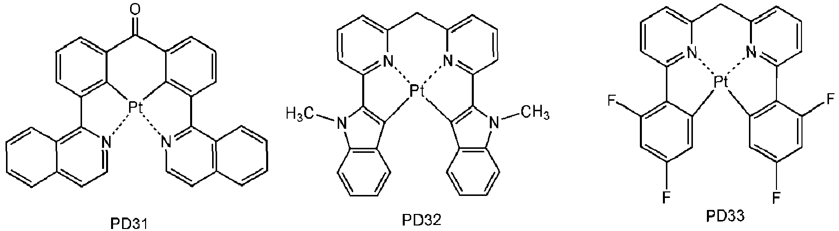

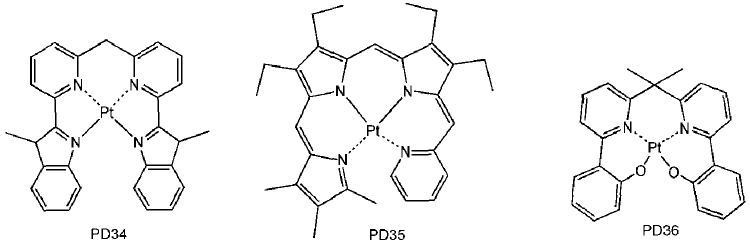

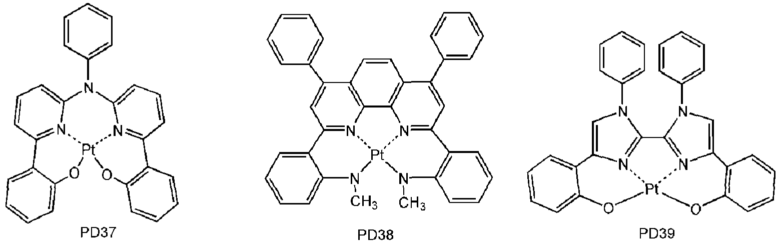

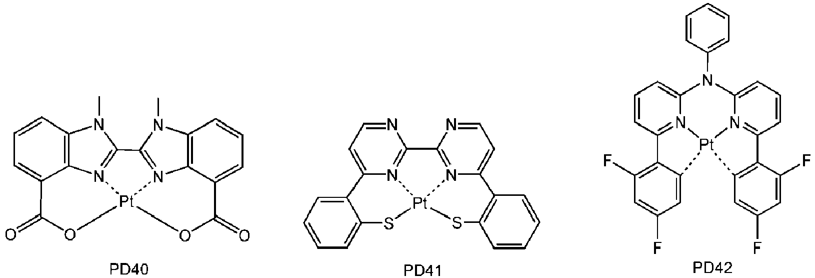

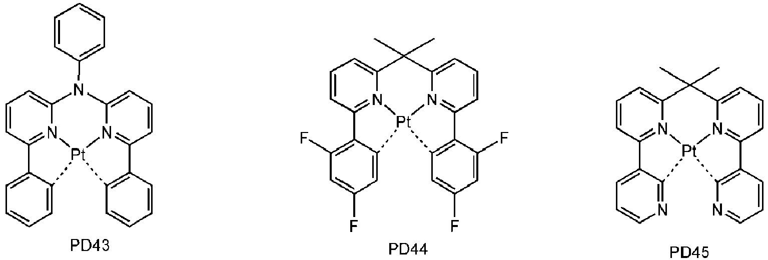

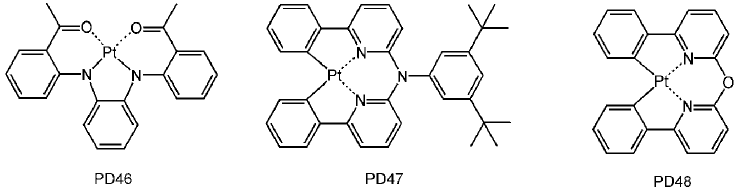

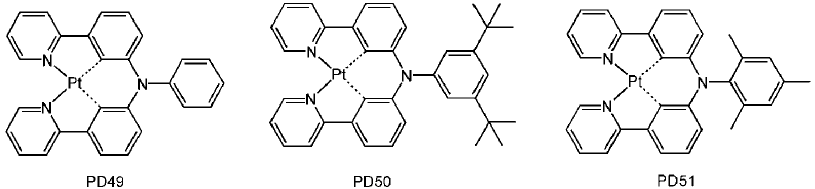

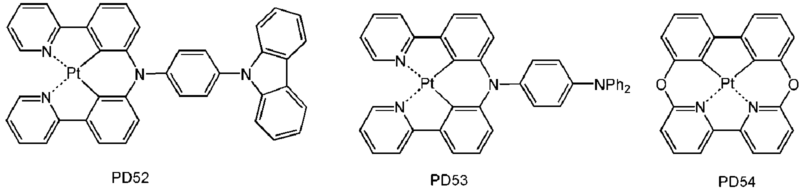

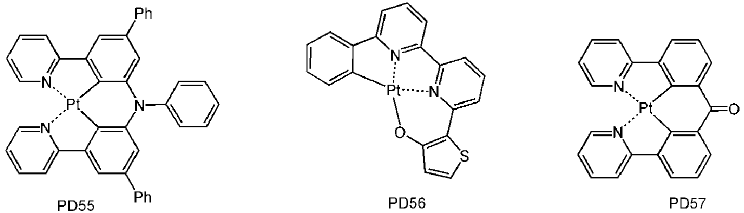

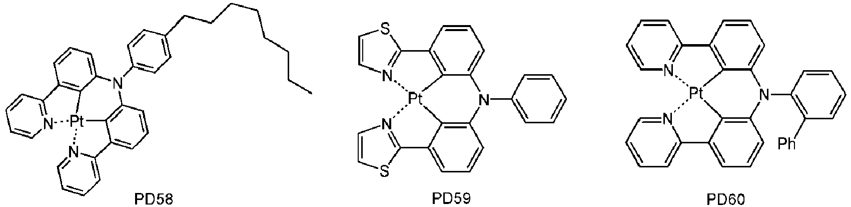

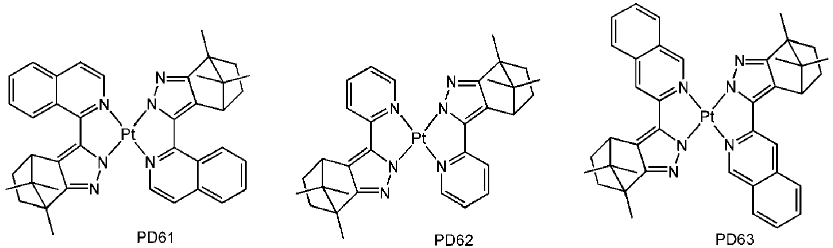

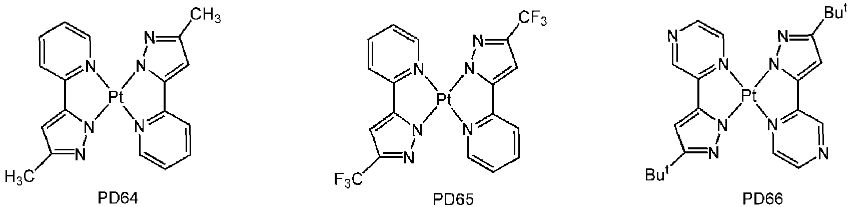

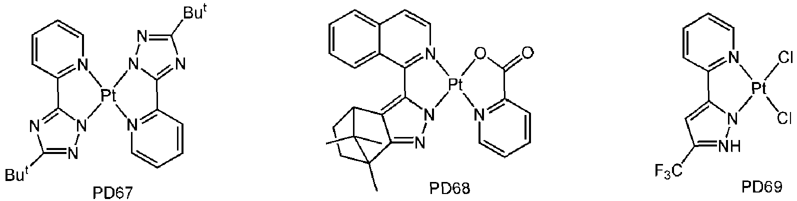

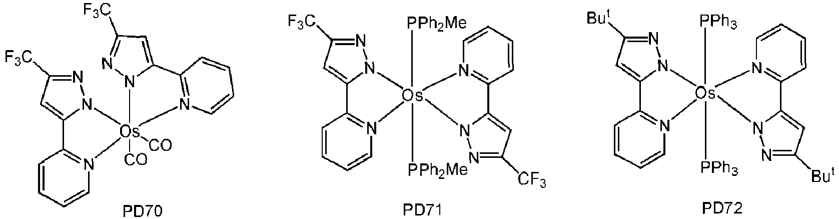

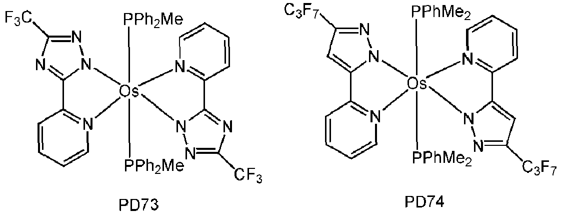

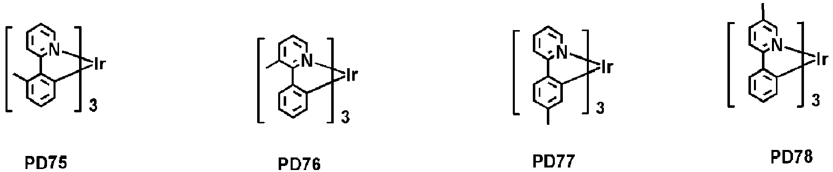

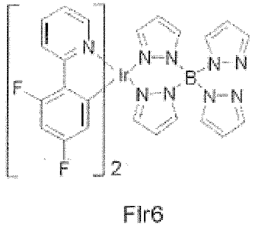

In some embodiments, the phosphorescent dopant may include PtOEP:

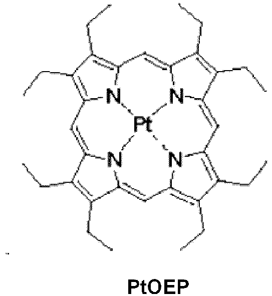

When the emission layer includes a host and a dopant, an amount of the dopant may be selected from a range of about 0.01 part by weight to about 20 parts by weight based on about 100 parts by weight of the host, but embodiments are not limited thereto.

A thickness of the emission layer may be in a range of about 10 nm (about 100 A) to about 100 nm (about 1,000 A), for example, about 20 nm (about 200 A) to about 60 nm (about 600 A). While not wishing to be bound by theory, it is understood that when the thickness of the emission layer is within this range, excellent light-emission characteristics may be obtained without a substantial increase in driving voltage.

Then, an electron transport region may be disposed on the emission layer.

The electron transport region may include at least one selected from a hole blocking layer, an electron transport layer, and an electron injection layer, but is not limited thereto.

For example, the electron transport region may have a structure of a hole blocking layer/ an electron transport layer/an electron injection layer or an electron transport layer/an electron injection layer, but it is not limited thereto. The electron transport layer may have a single layer structure or a multi-layer structure including two or more different materials.

The conditions for forming a hole blocking layer, an electron transport layer, and an electron injection layer, of the electron transport region, may be inferred based on the conditions for forming the hole injection layer.

When the electron transport region includes a hole blocking layer, the hole blocking layer may, for example, include at least one of BCP and Bphen, but is not limited thereto.

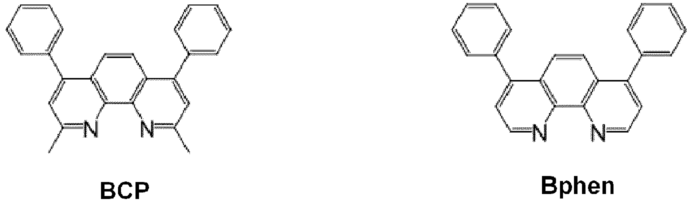

A thickness of the hole blocking layer may be in a range of about 2 nm (about 20 A) to about 100 nm (about 1,000 A), for example, about 3 nm (about 30 A) to about 30 nm (about 300 A). While not wishing to be bound by theory, it is understood that when the thickness of the hole blocking layer is within this range, excellent hole blocking characteristics may be obtained without a substantial increase in driving voltage.

The electron transport layer may further include at least one selected from BCP, BPhen, Alq3, BAIq, TAZ, and NTAZ.

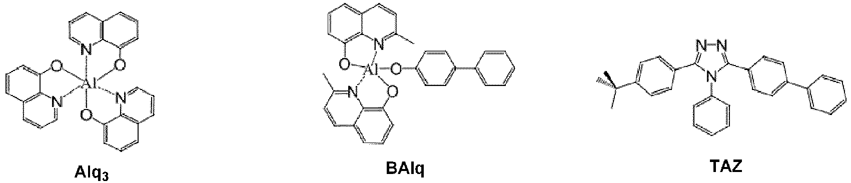

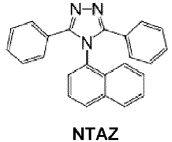

In some embodiments, the electron transport layer may include at least one selected from Compounds ET1, ET2 and ET3, but it is not limited thereto.

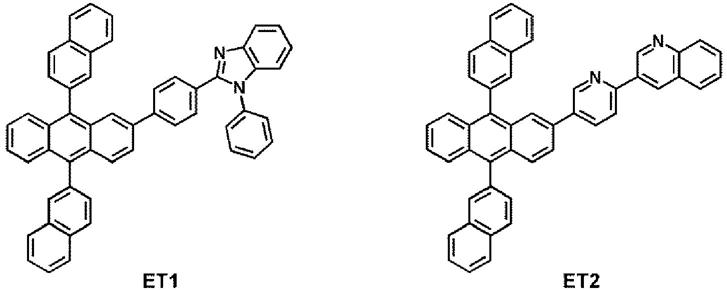

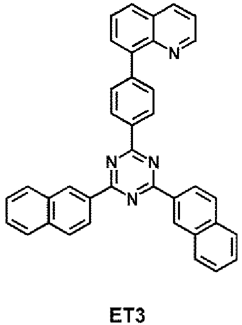

A thickness of the electron transport layer may be in a range of about 10 nm (about 100 Å) to about 100 nm (about 1, 000 Å), for example, about 15 nm (about150 Å) to about 50 nm (about 500 Å). While not wishing to be bound by theory, it is understood that when the thickness of the electron transport layer is within this range, excellent electron transport characteristics may be obtained without a substantial increase in driving voltage.

The electron transport layer may further include a metal-containing material in addition to the materials described above.

The metal-containing material may include a Li complex. The Li complex may include, for example, Compound ET-D1 (lithium quinolate, LiQ) or ET-D2.

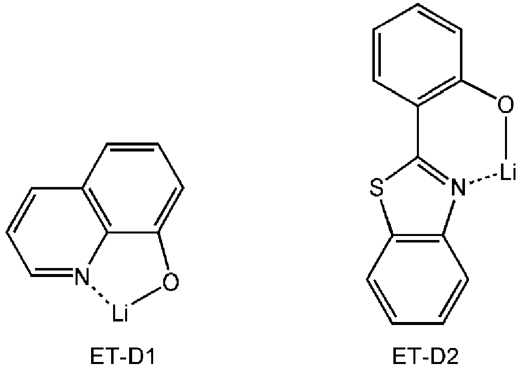

The electron transport region may include an electron injection layer (EIL) that facilitates electron injection from the second electrode 19.

The electron injection layer may include at least one selected from LiQ, LiF, NaCl, CsF, Li₂O, and BaO.

A thickness of the electron injection layer may be in a range of about 0.1 nm (about 1 Å) to about 10 nm (about 100 Å), for example, about 0.3 nm (about 3 Å) to about 9 nm (about 90 Å). While not wishing to be bound by theory, it is understood that when the thickness of the electron injection layer is within this range, excellent electron injection characteristics may be obtained without a substantial increase in driving voltage.

The second electrode 19 is disposed on the organic layer 15. The second electrode 19 may be a cathode. A material for the second electrode 19 may be a material having a relatively low work function, such as a metal, an alloy, an electrically conductive compound, and a mixture thereof. Detailed examples of the material for forming the second electrode 19 are lithium (Li), magnesium (Mg), aluminum (Al), aluminum-lithium (Al-Li), calcium (Ca), magnesium-indium (Mg-In), and magnesium-silver (Mg-Ag). Alternatively, ITO or IZO may be used to form a transmissive second electrode 19 to manufacture a top emission light-emitting device, and such a variation may be possible.

According to an embodiment, the organic layer 15 of the organic light-emitting device 10 may include a hole transport region and an emission layer, and the hole transport region and the emission layer may include the condensed cyclic compound, wherein the condensed cyclic compound included in the hole transport region may be the same as the condensed cyclic compound included in the emission layer.

In some embodiments, the organic layer 15 of the organic light-emitting device 10 may include a hole transport region and an emission layer, and the hole transport region and the emission layer may include the condensed cyclic compound, wherein the condensed cyclic compound included in the hole transport region may be different than the condensed cyclic compound included in the emission layer.

Here, the hole transport region may include at least one of a hole transport layer and an electron blocking layer, and the condensed cyclic compound may be included in:
i) the hole transport layer,
ii) the electron blocking layer or
iii) the hole transport layer and the electron blocking layer. The electron blocking layer may directly contact the emission layer.

Hereinbefore, an organic light-emitting device has been described with reference to FIG. 1, but embodiments are not limited thereto.

A C₁-C₆₀ alkyl group as used herein refers to a linear or branched aliphatic saturated hydrocarbon monovalent group having 1 to 60 carbon atoms. Detailed examples thereof are a methyl group, an ethyl group, a propyl group, an iso-butyl group, a sec-butyl group, a tert-butyl group, a pentyl group, an iso-amyl group, and a hexyl group. A C₁-C₆₀ alkylene group as used herein refers to a divalent group having the same structure as a C₁-C₆₀ alkyl group.

A C₁-C₆₀ alkoxy group as used herein refers to a monovalent group represented by -OA₁₀₁ (wherein A₁₀₁ is the C₁-C₆₀ alkyl group). Detailed examples thereof are a methoxy group, an ethoxy group, and an isopropyloxy group.

A C₂-C₆₀ alkenyl group as used herein refers to a group formed by substituting at least one carbon-carbon double bond in the middle or at the terminal of the C₂-C₆₀ alkyl group. Detailed examples thereof are an ethenyl group, a propenyl group, and a butenyl group. A C₂-C₆₀ alkenylene group as used herein refers to a divalent group having the same structure as a C₂-C₆₀ alkenyl group.

A C₂-C₆₀ alkynyl group as used herein refers to a group formed by substituting at least one carbon-carbon triple bond in the middle or at the terminal of the C₂-C₆₀ alkyl group. Detailed examples thereof are an ethynyl group and a propynyl group. A C₂-C₆₀ alkynylene group as used herein refers to a divalent group having the same structure as a C₂-C₆₀ alkynyl group.

A C₃-C₁₀ cycloalkyl group as used herein refers to a monovalent monocyclic saturated hydrocarbon group including 3 to 10 carbon atoms. Detailed examples thereof are a cyclopropyl group, a cyclobutyl group, a cyclopentyl group, a cyclohexyl group, and a cycloheptyl group. A C₃-C₁₀ cycloalkylene group as used herein refers to a divalent group having the same structure as a C₃-C₁₀ cycloalkyl group.

A C₁-C₁₀ heterocycloalkyl group as used herein refers to a monovalent monocyclic group including at least one heteroatom selected from N, O, P, Si, and S as a ring-forming atom and 1 to 10 carbon atoms. Detailed examples thereof are a tetrahydrofuranyl group and a tetrahydrothiophenyl group. A C₁-C₁₀ heterocycloalkylene group as used herein refers to a divalent group having the same structure as a C₁-C₁₀ heterocycloalkyl group.

A C₃-C₁₀ cycloalkenyl group as used herein refers to a monovalent monocyclic group that has 3 to 10 carbon atoms and at least one double bond in its ring, and which is not aromatic. Detailed examples thereof are a cyclopentenyl group, a cyclohexenyl group, and a cycloheptenyl group. A C₃-C₁₀ cycloalkenylene group as used herein refers to a divalent group having the same structure as a C₃-C₁₀ cycloalkenyl group.

A C₁-C₁₀ heterocycloalkenyl group as used herein refers to a monovalent monocyclic group including at least one heteroatom selected from N, O, P, Si and S as a ring-forming atom, 1 to 10 carbon atoms, and at least one double bond in its ring. Detailed examples of the C₁-C₁₀ heterocycloalkenyl group are a 2,3-dihydrofuranyl group and a 2,3-dihydrothiophenyl group. A C₂-C₁₀ heterocycloalkenylene group as used herein refers to a divalent group having the same structure as a C₁-C₁₀ heterocycloalkenyl group.

A C₆-C₆₀ aryl group as used herein refers to a monovalent group having a carbocyclic aromatic system having 6 to 60 carbon atoms, and a C₆-C₆₀ arylene group as used herein refers to a divalent group having a carbocyclic aromatic system having 6 to 60 carbon atoms. Detailed examples of the C₆-C₆₀ aryl group are a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other.

A C₁-C₆₀ heteroaryl group as used herein refers to a monovalent group having a cyclic aromatic system including at least one heteroatom selected from N, O, P, Si and S as a ring-forming atom and 1 to 60 carbon atoms. A C₁-C₆₀ heteroarylene group as used herein refers to a divalent group having a cyclic aromatic system including at least one heteroatom selected from N, O, P, Si and S as a ring-forming atom and 1 to 60 carbon atoms. Detailed examples of the C₁-C₆₀ heteroaryl group include a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include a plurality of rings, the rings may be fused to each other.

A C₆-C₆₀ aryloxy group as used herein indicates -OA₁₀₂ (wherein A₁₀₂ is the C₆-C₆₀ aryl group), a C₆-C₆₀ arylthio group as used herein indicates -SA₁₀₃ (wherein A₁₀₃ is the C₆-C₆₀ aryl group), and a C₇-C₆₀ arylalkyl group as used herein indicates -A₁₀₄A₁₀₅ (wherein A₁₀₅ is the C₆-C₅₉ aryl group and A₁₀₄ is the C₁-C₅₃ alkyl group).

A C₂-C₆₀ heteroaryloxy group as used herein indicates -OA₁₀₆ (wherein A₁₀₆ is the C₂-C₆₀ heteroaryl group), a C₂-C₆₀ heteroarylthio group as used herein indicates -SA₁₀₇ (wherein A₁₀₇ is the C₂-C₆₀ heteroaryl group), and a C₃-C₆₀ heteroarylalkyl group as used herein indicates -A₁₀₈A₁₀₉ (wherein A₁₀₉ is the C₂-C₅₉ heteroaryl group and A₁₀₉ is the C₁-C₅₈ alkyl group).

A monovalent non-aromatic condensed polycyclic group as used herein refers to a monovalent group that has two or more rings condensed to each other, and only carbon atoms (for example, the number of carbon atoms may be in a range of 8 to 60) as ring-forming atoms, wherein the molecular structure as a whole is non-aromatic in the entire molecular structure. Detailed examples of the non-aromatic condensed polycyclic group include a fluorenyl group. A divalent non-aromatic condensed polycyclic group as used herein refers to a divalent group having the same structure as the monovalent non-aromatic condensed polycyclic group.

A monovalent non-aromatic condensed hetero-polycyclic group as used herein refers to a monovalent group that has a plurality of rings condensed with each other, has a heteroatom selected from N, O P, Si and S, other than carbon atoms (for example, the number of carbon atoms may be in a range of 1 to 60), as ring-forming atoms, wherein the molecular structure as a whole is non-aromatic in the entire molecular structure. The monovalent non-aromatic condensed heteropolycyclic group includes a carbazolyl group. A divalent non-aromatic condensed hetero-polycyclic group as used herein refers to a divalent group having the same structure as the monovalent non-aromatic condensed hetero-polycyclic group.

At least one substituent of the substituted C₁-C₆₀ alkyl group, substituted C₂-C₆₀ alkenyl group, substituted C₂-C₆₀ alkynyl group, substituted C₃-C₁₀ cycloalkyl group, substituted C₁-C₁₀ heterocycloalkyl group, substituted C₃-C₁₀ cycloalkenyl group, substituted C₁-C₁₀ heterocycloalkenyl group, substituted C₆-C₆₀ aryl group, substituted C₆-C₆₀ aryloxy group, substituted C₆-C₆₀ arylthio group, substituted C₇-C₆₀ arylalkyl group, substituted C₁-C₆₀ heteroaryl group, substituted C₂-C₆₀ heteroaryloxy group, substituted C₂-C₆₀ heteroarylthio group, substituted C₃-C₆₀ heteroarylalkyl group, substituted monovalent non-aromatic condensed polycyclic group and substituted monovalent non-aromatic condensed heteropolycyclic group may be selected from
a deuterium, -CD₃, -CD₂H, -CDH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group and a C₁-C₆₀ alkoxy group;
a C₁-C₆₀ alkyl group, a C₂-C₆₀ alkenyl group, a C₂-C₆₀ alkynyl group and a C₁-C₆₀ alkoxy group, each substituted with at least one selected from a deuterium, -CD₃, -CD₂H, -CDH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₂-C₆₀ heteroaryloxy group, a C₂-C₆₀ heteroarylthio group, a C₃-C₆₀ heteroarylalkyl group, a monovalent non-aromatic condensed polycyclic group, a monovalent non-aromatic condensed heteropolycyclic group, -Si(Q₁₁)(Q₁₂)(Q₁₃), -N(Q₁₄)(Q₁₅) and -B(Q₁₆)(Q₁₇);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₂-C₆₀ heteroaryloxy group, a C₂-C₆₀ heteroarylthio group, a C₃-C₆₀ heteroarylalkyl group, a monovalent non-aromatic condensed polycyclic group and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₂-C₆₀ heteroaryloxy group, a C₂-C₆₀ heteroarylthio group, a C₃-C₆₀ heteroarylalkyl group, a monovalent non-aromatic condensed polycyclic group and a monovalent non-aromatic condensed heteropolycyclic group, each substituted with at least one selected from a deuterium, -CD₃, -CD₂H, -CDH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₂-C₆₀ heteroaryloxy group, a C₂-C₆₀ heteroarylthio group, a C₃-C₆₀ heteroarylalkyl group, a monovalent non-aromatic condensed polycyclic group, a monovalent non-aromatic condensed heteropolycyclic group, -Si(Q₂₁)(Q₂₂)(Q₂₃), -N(Q₂₄)(Q₂₅) and-B(Q₂₆)(Q₂₇); and
-Si(Q₃₁)(Q₃₂)(Q₃₃), -N(Q₃₄)(Q₃₅) and -B(Q₃₆)(Q₃₇), and
Q₁ to Q₇, Q₁₁ to Q₁₇, Q₂₁ to Q₂₇ and Q₃₁ to Q₃₇ may be each independently selected from a hydrogen, a deuterium, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₂-C₆₀ heteroaryloxy group, a substituted or unsubstituted C₂-C₆₀ heteroarylthio group, a substituted or unsubstituted C₃-C₆₀ heteroarylalkyl group, a substituted or unsubstituted monovalent non-aromatic condensed polycyclic group and a substituted or unsubstituted monovalent non-aromatic condensed heteropolycyclic group.

When a group containing a specified number of carbon atoms is substituted with any of the groups listed in the preceding paragraph, the number of carbon atoms in the resulting "substituted" group is defined as the sum of the carbon atoms contained in the original (unsubstituted) group and the carbon atoms (if any) contained in the substituent. For example, when the term "substituted C₁-C₆₀ alkyl" refers to a C₁-C₆₀ alkyl group substituted with C₆-C₆₀ aryl group, the total number of carbon atoms in the resulting aryl substituted alkyl group is C₇-C₁₂₀.

The term "a biphenyl group" as used herein refers to a monovalent group in which two benzene rings are linked to each other by a single bond.

The term "a terphenyl group" as used herein refers to a monovalent group in which three benzene rings are linked to each other by a single bond.

Hereinafter, a compound and an organic light-emitting device according to an embodiment of the present disclosure will be described in detail with reference to Synthesis Examples and Examples. However, the present disclosure is not limited thereto. The expression "B was used instead of A" used in describing Synthesis Examples means that an amount of B used was identical to an amount of A used based on molar equivalence.

### Example

### Synthesis Example 1: Synthesis of Compound 86

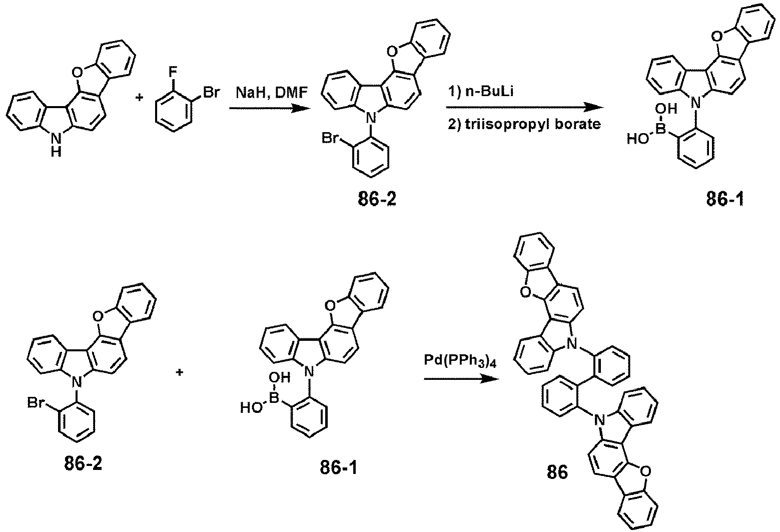

### Synthesis of Intermediate 86-2

6.24 grams (g) (24.26 millimoles (mmol)) of 5H-benzofuro[3, 2-c]carbazole was added to 80 milliliters (ml) of DMF and then 1.16 g (29.11 mmol) of NaH (60% dispersion in mineral oil) was added thereto at 0°C. The resulting mixture was stirred at room temperature for an hour. Then, 5.52 g (31.53 mmol) of 1-bromo-2-fluorobenzene was added thereto and the mixture stirred for 18 hours while being heated at 150°C. 100 ml of ethyl acetate was added thereto, and then the result was washed with 100 ml of water twice and dried with MgSO₄. Then, ethyl acetate was removed therefrom using a rotavapor to obtain a viscous product. The product was purified by silica gel column chromatography to obtain 7.6 g of Intermediate 86-2. The identity of obtained Intermediate 86-2 was confirmed by LC-MS.

### Synthesis of Intermediate 86-1

11.35 g (27.53 mmol) of Intermediate 86-2 was dissolved in 92 ml of anhydrous THF and then 1.6 molar (M) (20.6 ml) of n-BuLi was slowly added thereto at -78°C over the course of one hour. After a 3-hour reaction, 8.80 g (46.80 mmol) of triisopropyl borate was added thereto and a reaction temperature was slowly increased to room temperature. Then, 1 normal (N) (10 ml) of HCl was added thereto, and the resulting mixture was stirred for 2 hours. The solvent was removed using a rotavapor, and the resulting residue was dissolved by adding 200 ml of methylene chloride thereto and washed with 100 ml of water twice, thereby obtaining a white solid. Slurry treatments using 1 liter (L) of hexane and 1 L of MeOH were respectively performed on the white solid to obtain 6.5 g of Intermediate 86-1. The identity of obtained Intermediate 86-1 was confirmed by LC-MS.
C₂₄H₁₆BNO₃: [M+H] = 377.09

### Synthesis of Compound 86

3.92 g of Intermediate 86-2, 4.34 g of Intermediate 86-1, 0.41 g (0.44 mmol) of Pd(PPh₃)₄ and 2.45 g (17.71 mmol) of K₂CO₃ were mixed with 35 ml/5 ml of THF/H₂O, and the resulting mixture was stirred for 18 hours while being heated at 80°C. The resulting product was precipitated by adding 500 ml of MeOH, stirred and collected by filtration to obtain a solid compound. The solid compound was purified by silica gel column chromatography to obtain 3.9 g of Compound 86. The identity of obtained Compound 86 was confirmed by LC-MS.
C₄₈H₂₈N₂O₂: [M+H] = 664.54

### Synthesis Example 2: Synthesis of Compound 87

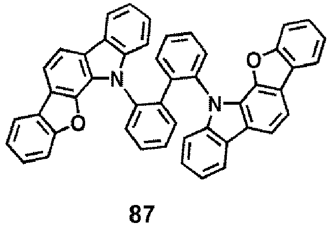

Compound 87 was synthesized in the same manner as in Synthesis Example 1, except that, when synthesizing Intermediate 86-2, 12H-benzofuro[2,3-a]carbazole was used instead of 5H-benzofuro[3,2-c]carbazole. The synthesized Compound was confirmed by LC-MS.

### Synthesis Example 3: Synthesis of Compound 88

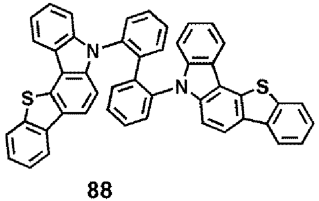

Compound 88 was synthesized in the same manner as in Synthesis Example 1, except that, when synthesizing Intermediate 86-2, 5H-benzo[4,5]thieno[3,2-c]carbazole was used instead of 5H-benzofuro[3,2-c]carbazole. The synthesized Compound was confirmed by LC-MS.
C₄₈H₂₈N₂S₂: [M+H] = 696.12

### Synthesis Example 4: Synthesis of Compound 89

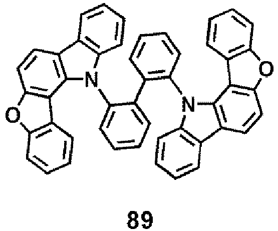

Compound 89 was synthesized in the same manner as in Synthesis Example 1, except that, when synthesizing Intermediate 86-2, 12H-benzofuro[3,2-a]carbazole was used instead of 5H-benzofuro[3,2-c]carbazole. The synthesized Compound was confirmed by LC-MS.
C₄₈H₂₈N₂O₂: [M+H] = 664.62

### Synthesis Example 5: Synthesis of Compound 27

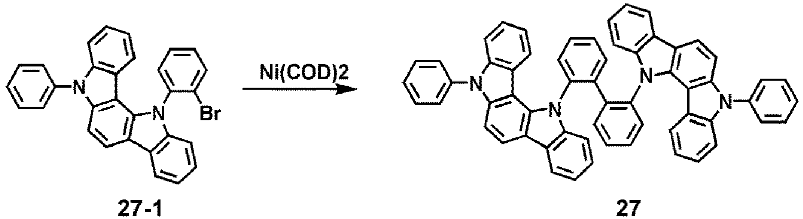

4.05 g of Ni(COD)₂, 1.59 g of COD, and 2.30 g of bi-pyridyl were mixed with 24 ml of DMF and the resulting mixture was heated to 85°C and stirred for 30 minutes. Then, a mixture of 5 ml of toluene and 4.78 g of Intermediate 27-1 was slowly added thereto and the resulting mixture was heated and stirred for 3 hours. Then, a mixture of conc. HCI / MeOH / acetone (1/1/1) was slowly added thereto to form a precipitate, the precipitate was filtered using a paper and washed with MeOH to obtain a solid compound, and the solid compound was subjected to silica hot filtering using xylene and recrystallization with dichloromethane / xylene to obtain 5.50 g of Compound 27. The obtained Compound 27 was confirmed by LC-MS.
C₆₀H₃₈N₄: [M+H] = 814.30

### Synthesis Example 6: Synthesis of Compound 32

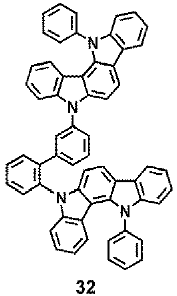

Compound 32 was synthesized in the same manner as in Synthesis Example 5, except that 5-(2-bromophenyl)-12-phenyl-5,12-dihydroindolo[3,2-a]carbazole was used instead of Intermediate 27-1. The synthesized Compound was confirmed by LC-MS.
C₆₀H₃₈N₄: [M+H] = 814.34

### Synthesis Example 7: Synthesis of Compound 80

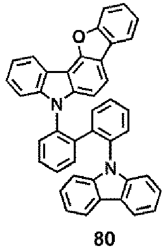

Compound 80 was synthesized in the same manner as in Synthesis Example 1, except that, when synthesizing Compound 86, (2-(9H-carbazol-9-yl)phenyl)boronic acid was used instead of Intermediate 86-1. The synthesized Compound was confirmed by LC-MS.
C₄₂H₂₆N₂O: [M+H] = 574.18

### Synthesis Example 8: Synthesis of Compound 91

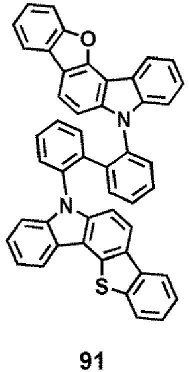

Compound 91 was synthesized in the same manner as in Synthesis Example 1, except that, when synthesizing Compound 86, (2-(5H-benzo[4,5]thieno[3,2-c]carbazol-5-yl)phenyl)boronic acid was used instead of Intermediate 86-1. The synthesized Compound was confirmed by LC-MS.
C₄₈H₂₈N₂OS: [M+H] = 680.14

### Synthesis Example 9: Synthesis of Compound 97

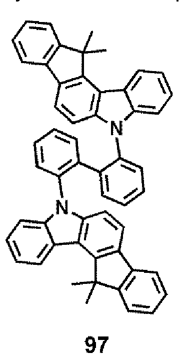

Compound 97 was synthesized in the same manner as in Synthesis Example 5, except that 5-(2-bromophenyl)-12,12-dimethyl-5,12-dihydroindeno[1,2-c]carbazole was used instead of Intermediate 27-1. The synthesized Compound was confirmed by LC-MS.
C₅₄H₄₀N₂: [M+H] = 716.33

### Example 1

A glass substrate having a thickness of 150 nm (1,500 Å) and an indium tin oxide (ITO) electrode as a first electrode (anode) thereon was sonicated with distilled water, and then further sonicated with iso-propyl alcohol, acetone, and methanol, and dried to be placed in a plasma cleaner. Next, the glass substrate was cleaned for 5 minutes by using oxygen plasma and then mounted on a vacuum deposition apparatus.

Compound HT3 and Compound HP-1 were co-deposited on the ITO electrode of the glass substrate to form a hole injection layer having a thickness of 10 nm (100 Å), and Compound HT3 was deposited on the hole injection layer to form a hole transport layer having a thickness of 130 nm (1,300 Å). mCP was deposited on the hole transport layer to form an electron blocking layer having a thickness of 15 nm (150 Å), thereby forming a hole transport region.

Compound 86 (host) and Flr6 (dopant, 10 percent by weight (wt%)) were co-deposited on the hole transport region to form an emission layer having a thickness of 30 nm (300 Å).

BCP was vacuum-deposited on the emission layer to form a hole blocking layer having a thickness of 10 nm (100 Å), and Compound ET3 and Liq were vacuum-deposited on the hole blocking layer to form an electron transport layer having a thickness of 25 nm (250 Å). Liq was deposited on the electron transport layer to form an electron injection layer having a thickness of 0.5 nm (5 Å) and an Al second electrode (cathode) having a thickness of 100 nm (1,000 Å) was formed on the electron injection layer, thereby manufacturing an organic light-emitting device.

### Examples 2 to 9 and Comparative Example 1

Organic light-emitting devices were manufactured in the same manner as in Example 1, except that when forming an emission layer, compounds shown in Table 2 were used instead of Compound 86.

### Evaluation Example 1: Characteristic evaluation of organic light-emitting device

Driving voltage, current density, luminous efficiency, power efficiency, quantum luminance efficiency and lifespan of each of the organic light-emitting devices manufactured in Examples 1 to 9 and Comparative Example 1 were measured by using a current-voltage meter (Keithley 2400) and a luminance meter (Minolta Cs-1000A). The results thereof are shown in Table 2. T₉₅ (at 500 candelas per meter, cd/m²) in Table 2 refers to an amount of time until luminance was decreased to 95% of its initial luminance. In Table 2, the driving voltage, luminous efficiency, power efficiency, quantum luminance efficiency and lifespan of Examples 1 to 9 were expressed in a relative value compared to "100", which denotes the driving voltage, luminous efficiency, power efficiency, quantum luminance efficiency and lifespan of Comparative Example 1.

**Table 2**

| | host | driving voltage (V) | luminous efficiency (cd/A) | Power efficiency (Im/W) | Quantum luminance efficiency (%) | T₉₅ (hr) |
|---|---|---|---|---|---|---|
| Example 1 | 86 | 89% | 114% | 128% | 113% | 256% |
| Example 2 | 87 | 105% | 105% | 108% | 104% | 142% |
| Example 3 | 88 | 101% | 129% | 128% | 129% | 167% |
| Example 4 | 89 | 98% | 109% | 116% | 111% | 120% |
| Example 5 | 27 | 107% | 104% | 108% | 103% | 103% |
| Example 6 | 32 | 101% | 120% | 118% | 117% | 123% |
| Example 7 | 80 | 87% | 117% | 115% | 112% | 208% |
| Example 8 | 91 | 95% | 122% | 128% | 121% | 212% |
| Example 9 | 97 | 99% | 117% | 114% | 115% | 93% |
| Comparative Example 1 | Compound B | 100% | 100% | 100% | 100% | 100% |

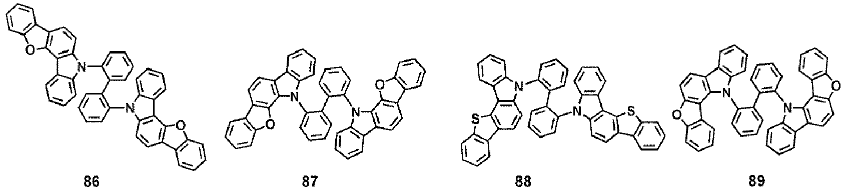

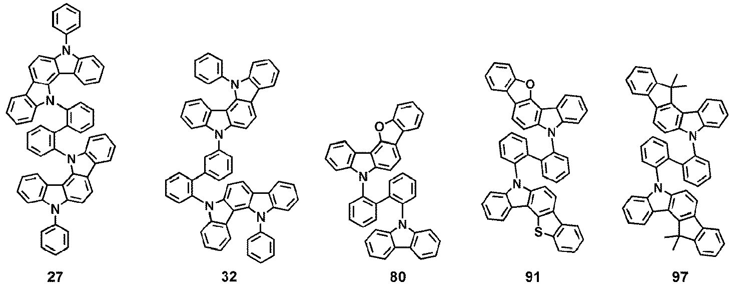

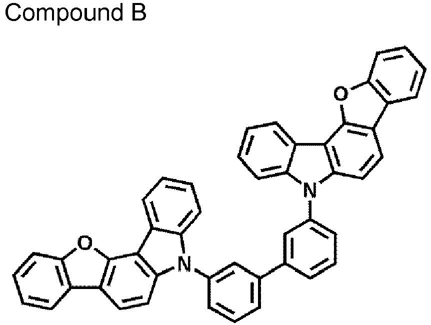

Table 2 shows that the organic light-emitting devices of Examples 1 to 9 have lower of comparable driving voltage, higher luminous efficiency, high power efficiency, higher quantum luminance efficiency and longer or comparable lifespan, compared to the organic light-emitting device of Comparative Example 1.

It should be understood that exemplary embodiments described herein should be considered in a descriptive sense only and not for purposes of limitation. Descriptions of features or aspects within each exemplary embodiment should typically be considered as available for other similar features or aspects in other exemplary embodiments.

While one or more exemplary embodiments have been described with reference to the figures, it will be understood by those of ordinary skill in the art that various changes in form and details may be made therein without departing from the scope of the present inventive concept as defined by the following claims.

## Claims

1. A condensed cyclic compound
represented by one of Formulae 1-1 to 1-3: 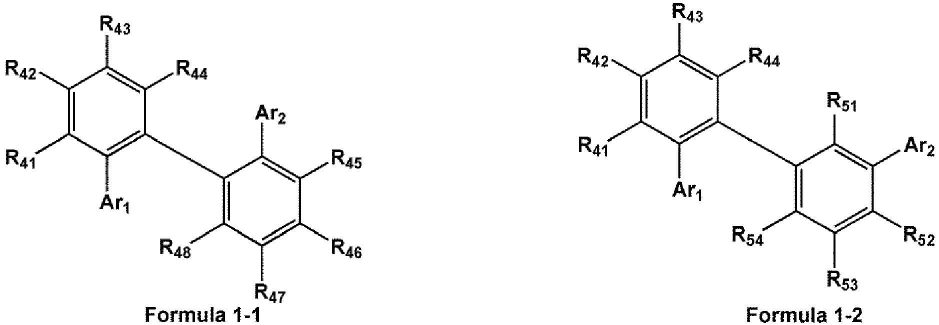 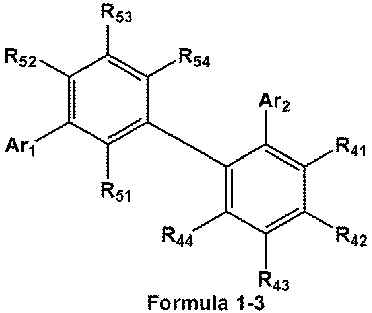 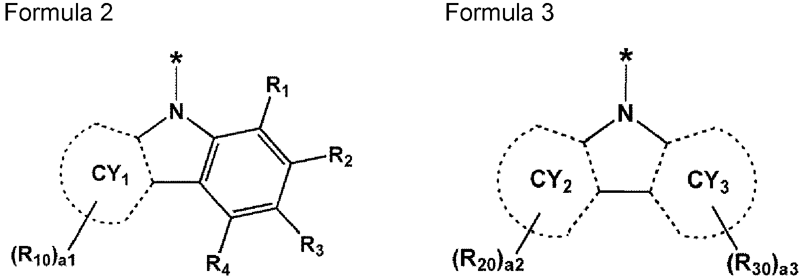 wherein
Ar₁ is a group represented by Formula 2,
Ar₂ is a group represented by Formula 3,
CY₁ is selected from a fluorene group, a carbazole group, a dibenzofuran group, and a dibenzothiophene group,
CY₂ and CY₃ are each independently selected from a benzene group, a fluorene group, a carbazole group, a dibenzofuran group, and a dibenzothiophene group,
R₁ to R₄, R₁₀, R₂₀ and R₃₀ are each independently selected from a hydrogen, a deuterium, a hydroxyl group, a cyano group (CN), a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₂-C₆₀ heteroaryloxy group, a substituted or unsubstituted C₂-C₆₀ heteroarylthio group, a substituted or unsubstituted C₃-C₆₀ heteroarylalkyl group, a substituted or unsubstituted monovalent non-aromatic condensed polycyclic group, a substituted or unsubstituted monovalent non-aromatic condensed heteropolycyclic group, -Si(Q₁)(Q₂)(Q₃), - N(Q₄)(Q₅), and -B(Q₆)(Q₇),
R₄₁₋₄₈ and R₅₁₋₅₄ are each independently selected from a hydrogen, a deuterium, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₂-C₆₀ heteroaryloxy group, a substituted or unsubstituted C₂-C₆₀ heteroarylthio group, a substituted or unsubstituted C₃-C₆₀ heteroarylalkyl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -N(Q₄)(Q₅), and -B(Q₆)(Q₇),
a1 to a3 are each independently an integer of 0 to 10,
* is a binding site to a neighboring atom,
at least one substituent of the substituted C₁-C₆₀ alkyl group, substituted C₂-C₆₀ alkenyl group, substituted C₂-C₆₀ alkynyl group, substituted C₃-C₁₀ cycloalkyl group, substituted C₁-C₁₀ heterocycloalkyl group, substituted C₃-C₁₀ cycloalkenyl group, substituted C₁-C₁₀ heterocycloalkenyl group, substituted C₆-C₆₀ aryl group, substituted C₆-C₆₀ aryloxy group, substituted C₆-C₆₀ arylthio group, substituted C₇-C₆₀ arylalkyl group, substituted C₁-C₆₀ heteroaryl group, substituted C₂-C₆₀ heteroaryloxy group, substituted C₂-C₆₀ heteroarylthio group, substituted C₃-C₆₀ heteroarylalkyl group, substituted monovalent non-aromatic condensed polycyclic group and substituted monovalent non-aromatic condensed heteropolycyclic group is selected from :
a deuterium, -CD₃, -CD₂H, -CDH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group and a C₁-C₆₀ alkoxy group;
a C₁-C₆₀ alkyl group, a C₂-C₆₀ alkenyl group, a C₂-C₆₀ alkynyl group and a C₁-C₆₀ alkoxy group, each substituted with at least one selected from a deuterium, -CD₃, -CD₂H, -CDH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₂-C₆₀ heteroaryloxy group, a C₂-C₆₀ heteroarylthio group, a C₃-C₆₀ heteroarylalkyl group, a monovalent non-aromatic condensed polycyclic group, a monovalent non-aromatic condensed heteropolycyclic group, -Si(Q₁₁)(Q₁₂)(Q₁₃), -N(Q₁₄)(Q₁₅) and -B(Q₁₆)(Q₁₇);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₂-C₆₀ heteroaryloxy group, a C₂-C₆₀ heteroarylthio group, a C₃-C₆₀ heteroarylalkyl group, a monovalent non-aromatic condensed polycyclic group and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₂-C₆₀ heteroaryloxy group, a C₂-C₆₀ heteroarylthio group, a C₃-C₆₀ heteroarylalkyl group, a monovalent non-aromatic condensed polycyclic group and a monovalent non-aromatic condensed heteropolycyclic group, each substituted with at least one selected from a deuterium, -CD₃, -CD₂H, -CDH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₂-C₆₀ heteroaryloxy group, a C₂-C₆₀ heteroarylthio group, a C₃-C₆₀ heteroarylalkyl group, a monovalent non-aromatic condensed polycyclic group, a monovalent non-aromatic condensed heteropolycyclic group, -Si(Q₂₁)(Q₂₂)(Q₂₃),-N(Q₂₄)(Q₂₅) and -B(Q₂₆)(Q₂₇); and
-Si(Q₃₁)(Q₃₂)(Q₃₃), -N(Q₃₄)(Q₃₅) and -B(Q₃₆)(Q₃₇), and
Q₁ to Q₇, Q₁₁ to Q₁₇, Q₂₁ to Q₂₇ and Q₃₁ to Q₃₇ are each independently selected from a hydrogen, a deuterium, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₂-C₆₀ heteroaryloxy group, a substituted or unsubstituted C₂-C₆₀ heteroarylthio group, a substituted or unsubstituted C₃-C₆₀ heteroarylalkyl group, a substituted or unsubstituted monovalent non-aromatic condensed polycyclic group and a substituted or unsubstituted monovalent non-aromatic condensed heteropolycyclic group.

2. The condensed cyclic compound of claim 1, wherein
Ar₁ is selected from groups represented by Formulae 2-1 to 2-6, and
Ar₂ is selected from groups represented by Formulae 3-1 to 3-7: 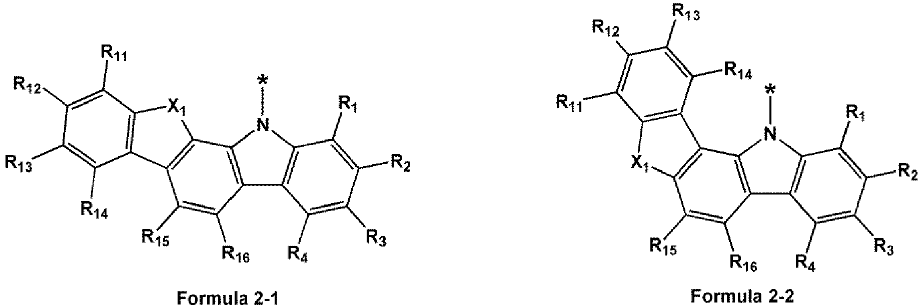 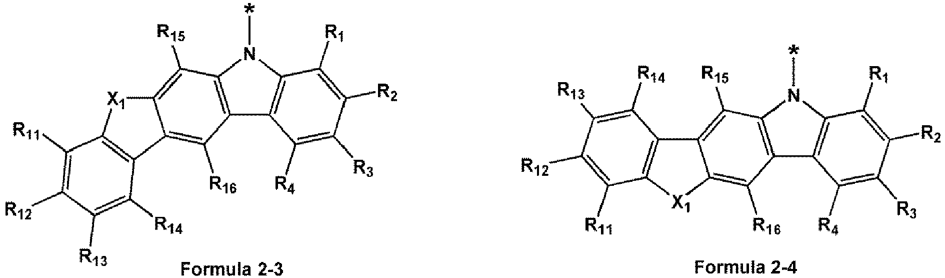 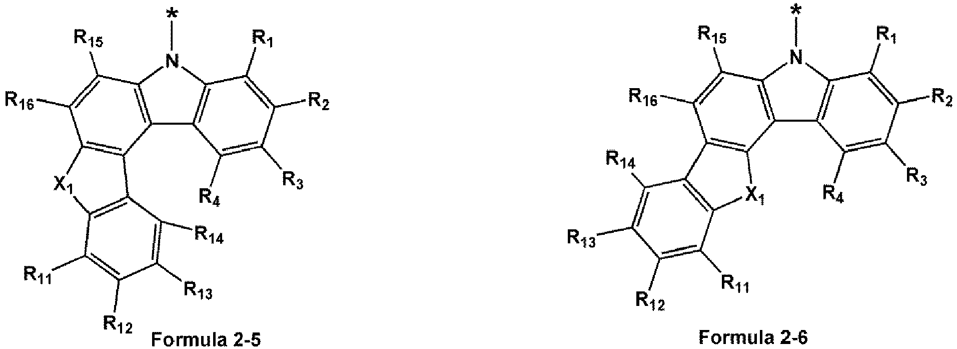 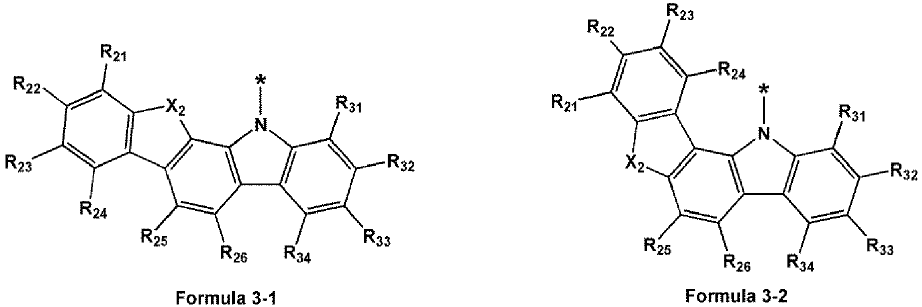 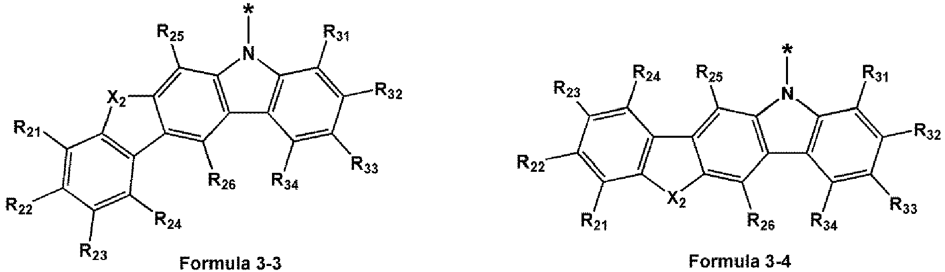 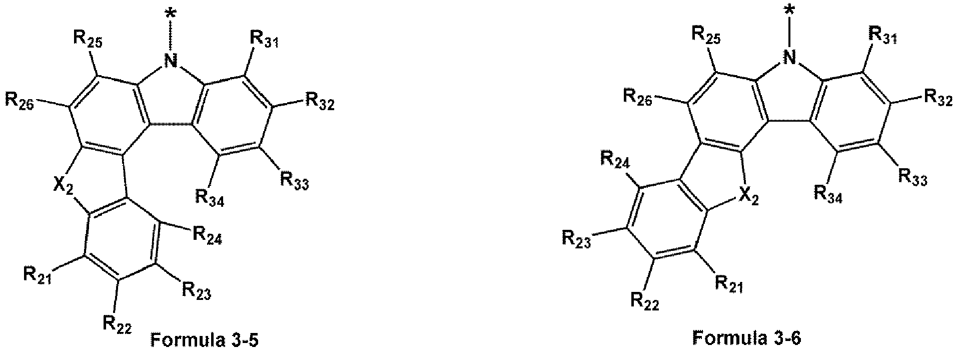 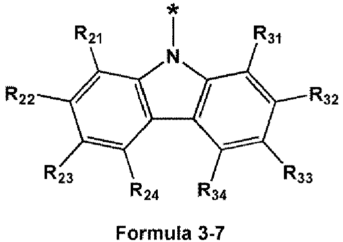 in Formulae 2-1 to 2-6 and 3-1 to 3-7,
X₁ is C(R₁₇)(R₁₈), N(R₁₉), O or S,
X₂ is C(R₂₇)(R₂₈), N(R₂₉), O or S,
R₁ to R₄, R₁₁ to R₁₉, R₂₁ to R₂₉ and R₃₁ to R₃₄ are each independently selected from
a hydrogen, a deuterium, a hydroxyl group, a cyano group (CN), a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group and a C₁-C₂₀ alkoxy group;
a C₁-C₂₀ alkyl group and a C₁-C₂₀ alkoxy group, each substituted with at least one selected from a deuterium, a hydroxyl group, a cyano group (CN), a nitro group, an amino group, an amidino group, a hydrazine group, a hydrazone group, a carboxylic acid group or a salt thereof, a sulfonic acid group or a salt thereof, a phosphoric acid group or a salt thereof, a phenyl group, a biphenyl group, a terphenyl group and a naphthyl group;
a cyclopentyl group, a cyclohexyl group, a cyclopentenyl group, a cyclohexenyl group, a cycloheptenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spirobifluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzooxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyrimidinyl group, an imidazopyridinyl group, a pyridoindolyl group, a benzofuropyridinyl group, a benzothienopyridinyl group, a pyrimidoindolyl group, a benzofuropyrimidinyl group, a benzothienopyrimidinyl group, a phenoxazinyl group, a pyridobenzooxazinyl group and a pyridobenzothiazinyl group;
a cyclopentyl group, a cyclohexyl group, a cyclopentenyl group, a cyclohexenyl group, a cycloheptenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzoxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyrimidinyl group, an imidazopyridinyl group, a pyridoindolyl group, a benzofuropyridinyl group, a benzothienopyridinyl group, a pyrimidoindolyl group, a benzofuropyrimidinyl group, a benzothienopyrimidinyl group, a phenoxazinyl group, a pyridobenzooxazinyl group and a pyridobenzothiazinyl group, each substituted with at least one selected from a deuterium, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a biphenyl group, a terphenyl group, a naphthyl group, an anthracenyl group, a pyrenyl group, a phenanthrenyl group, a fluorenyl group, a carbazolyl group, a benzocarbazolyl group, a dibenzocarbazolyl group, a pyridinyl group, a pyrimidinyl group, a pyrazinyl group, a pyridazinyl group, a triazinyl group, a quinolinyl group, an isoquinolinyl group, a phthalazinyl group, a quinoxalinyl group, a cinnolinyl group, a quinazolinyl group, -Si(Q₃₁)(Q₃₂)(Q₃₃), - N(Q₃₄)(Q₃₅) and -B(Q₃₆)(Q₃₇); and
-Si(Q₁)(Q₂)(Q₃), -N(Q₄)(Q₅), and -B(Q₆)(Q₇),
wherein Q₁ to Q₇ and Q₃₁ to Q₃₇ are each independently selected from a hydrogen, a C₁-C₂₀ alkyl group, a C₁-C₂₀ alkoxy group, a phenyl group, a biphenyl group, a terphenyl group, a naphthyl group, a fluorenyl group, a carbazolyl group, a dibenzofuranyl group and a dibenzothiophenyl group, and
* is a binding site to a neighboring atom, preferably wherein
R₁ to R₄, R₁₁ to R₁₉, R₂₁ to R₂₉ and R₃₁ to R₃₄ are each independently selected from
a hydrogen, a deuterium, a cyano group (CN), a C₁-C₂₀ alkyl group and a C₁-C₂₀ alkoxy group;
a C₁-C₂₀ alkyl group and a C₁-C₂₀ alkoxy group, each substituted with at least one selected from a deuterium, a cyano group (CN), a phenyl group, a biphenyl group, a terphenyl group and a naphthyl group;
a cyclopentyl group, a cyclohexyl group, a cyclopentenyl group, a cyclohexenyl group, a cycloheptenyl group, a phenyl group, a biphenyl group, a terphenyl group, a naphthyl group, a fluorenyl group, a carbazolyl group, a dibenzofuranyl group and a dibenzothiophenyl group;
a cyclopentyl group, a cyclohexyl group, a cyclopentenyl group, a cyclohexenyl group, a cycloheptenyl group, a phenyl group, a biphenyl group, a terphenyl group, a naphthyl group, a fluorenyl group, a carbazolyl group, a dibenzofuranyl group and a dibenzothiophenyl group, each substituted with at least one selected from a deuterium, a cyano group, a C₁-C₂₀ alkyl group, a C₁-C₂₀ alkoxy group, a phenyl group, a biphenyl group, a terphenyl group, a naphthyl group and -Si(Q₃₁)(Q₃₂)(Q₃₃); and
-Si(Q₁)(Q₂)(Q₃), and
Q₁ to Q₃ and Q₃₁ to Q₃₃ are each independently selected from a hydrogen, a C₁-C₂₀ alkyl group, a C₁-C₂₀ alkoxy group, a phenyl group, a biphenyl group, a terphenyl group and a naphthyl group, and/or preferablywherein
X₁ is O or S.

3. The condensed cyclic compound of claim 1 or 2, wherein
Ar₁ is the same as Ar₂ in Formulae 1-1 to 1-3; or
CY₂ and CY₃ in Formula 3 are each independently a benzene group.

4. The condensed cyclic compound of claim 2, wherein
Ar₁ is represented by Formula 2-1, and Ar₂ is represented by Formula 3-1;
Ar₁ is represented by Formula 2-2, and Ar₂ is represented by Formula 3-2; or
Ar₁ is represented by Formula 2-6, and Ar₂ is represented by Formula 3-6.

5. The condensed cyclic compound of any of claims 1 to 4, wherein
the condensed cyclic compound is represented by Formula 1-1, and/or wherein
R₄₁ to R₄₈ and R₅₁ to R₅₄ are each independently selected from
a hydrogen, a deuterium, a C₁-C₂₀ alkyl group and a C₁-C₂₀ alkoxy group;
a C₁-C₂₀ alkyl group and a C₁-C₂₀ alkoxy group, each substituted with at least one selected from a deuterium, a phenyl group, a biphenyl group, a terphenyl group and a naphthyl group;
a cyclopentyl group, a cyclohexyl group, a cyclopentenyl group, a cyclohexenyl group, a cycloheptenyl group, a phenyl group, a biphenyl group, a terphenyl group, a naphthyl group, a fluorenyl group, a carbazolyl group, a dibenzofuranyl group and a dibenzothiophenyl group;
a cyclopentyl group, a cyclohexyl group, a cyclopentenyl group, a cyclohexenyl group, a cycloheptenyl group, a phenyl group, a biphenyl group, a terphenyl group, a naphthyl group, a fluorenyl group, a carbazolyl group, a dibenzofuranyl group and a dibenzothiophenyl group, each substituted with at least one selected from a deuterium, a C₁-C₂₀ alkyl group, a C₁-C₂₀ alkoxy group, a phenyl group, a biphenyl group, a terphenyl group, a naphthyl group and -Si(Q₃₁)(Q₃₂)(Q₃₃); and
-Si(Q₁)(Q₂)(Q₃), and
Q₁ to Q₃ and Q₃₁ to Q₃₃ are each independently selected from a hydrogen, a C₁-C₂₀ alkyl group, a C₁-C₂₀ alkoxy group, a phenyl group, a biphenyl group, a terphenyl group and a naphthyl group.

6. The condensed cyclic compound of claim 5, wherein
Ar₁ is selected from groups represented by Formulae 2-1 to 2-6,
Ar₂ is selected from groups represented by Formulae 3-1 to 3-7: 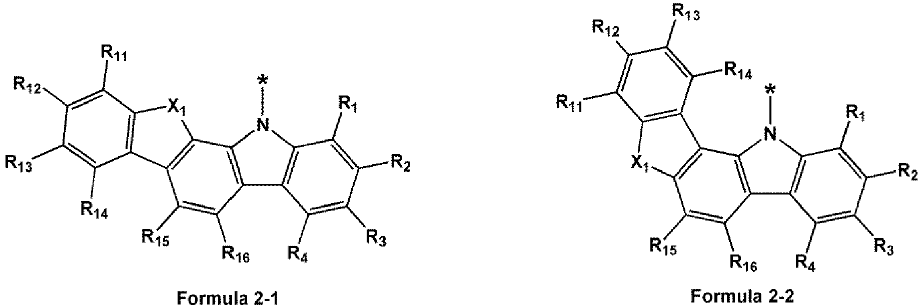 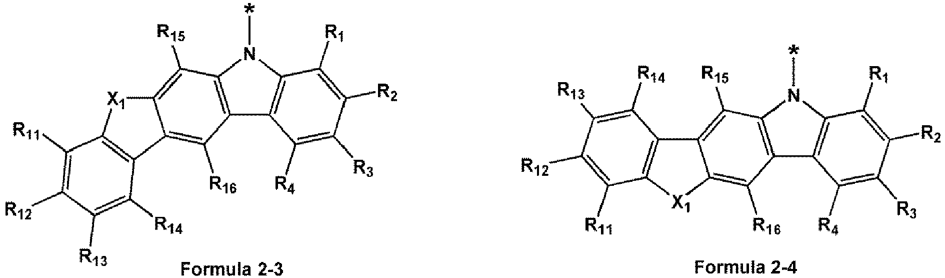 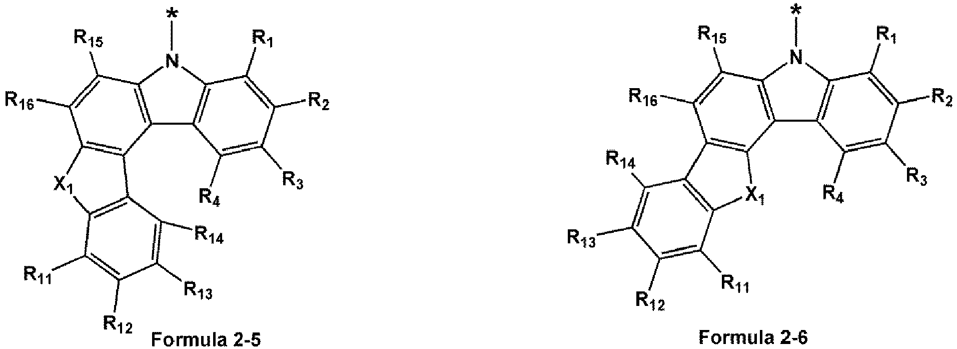 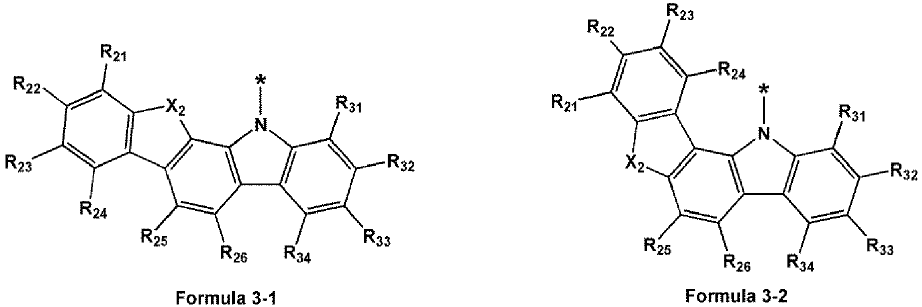 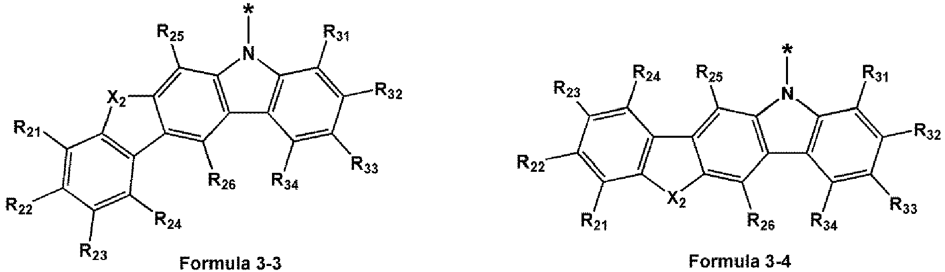 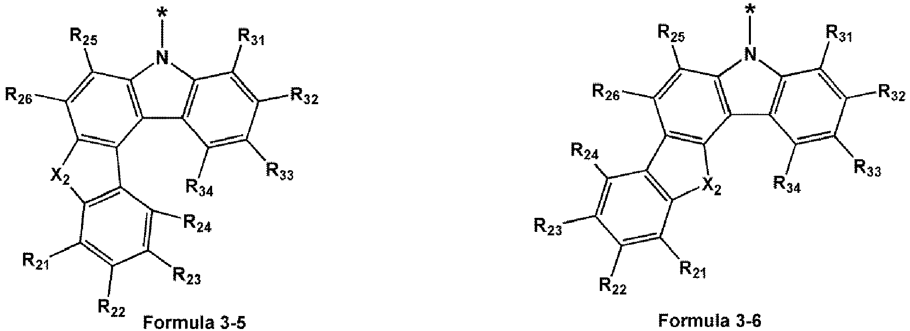 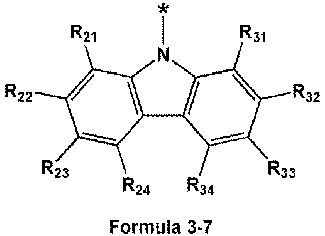 in Formulae 2-1 to 2-6 and 3-1 to 3-7,
X₁ is C(R₁₇)(R₁₈), N(R₁₉), O or S,
X₂ is C(R₂₇)(R₂₈), N(R₂₉), O or S,
R₁ to R₄, R₁₁ to R₁₉, R₂₁ to R₂₉ and R₃₁ to R₃₄ are each independently selected from
a hydrogen, a deuterium, a cyano group (CN), a C₁-C₂₀ alkyl group and a C₁-C₂₀ alkoxy group;
a C₁-C₂₀ alkyl group and a C₁-C₂₀ alkoxy group, each substituted with at least one selected from a deuterium, a cyano group (CN), a phenyl group, a biphenyl group, a terphenyl group and a naphthyl group;
a cyclopentyl group, a cyclohexyl group, a cyclopentenyl group, a cyclohexenyl group, a cycloheptenyl group, a phenyl group, a biphenyl group, a terphenyl group, a naphthyl group, a fluorenyl group, a carbazolyl group, a dibenzofuranyl group and a dibenzothiophenyl group;
a cyclopentyl group, a cyclohexyl group, a cyclopentenyl group, a cyclohexenyl group, a cycloheptenyl group, a phenyl group, a biphenyl group, a terphenyl group, a naphthyl group, a fluorenyl group, a carbazolyl group, a dibenzofuranyl group and a dibenzothiophenyl group, each substituted with at least one selected from a deuterium, a cyano group, a C₁-C₂₀ alkyl group, a C₁-C₂₀ alkoxy group, a phenyl group, a biphenyl group, a terphenyl group, a naphthyl group and -Si(Q₃₁)(Q₃₂)(Q₃₃); and
-Si(Q₁)(Q₂)(Q₃),
wherein Q₁ to Q₃ and Q₃₁ to Q₃₃ are each independently selected from a hydrogen, a C₁-C₂₀ alkyl group, a C₁-C₂₀ alkoxy group, a phenyl group, a biphenyl group, a terphenyl group and a naphthyl group, preferably wherein
the condensed cyclic compound is represented by Formula 1-1, and
X₁ in Formulae 2-1 to 2-6 is O or S.

7. The condensed cyclic compound of claim 1, wherein
the condensed cyclic compound is selected from Compounds 1 to 108:

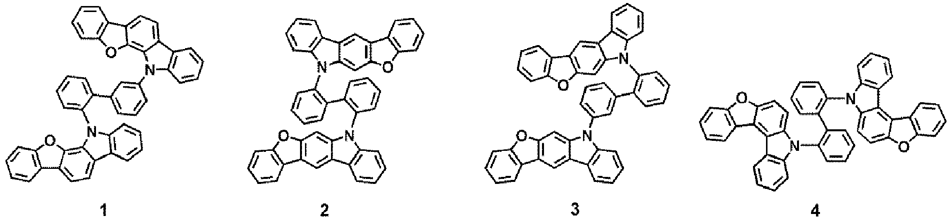

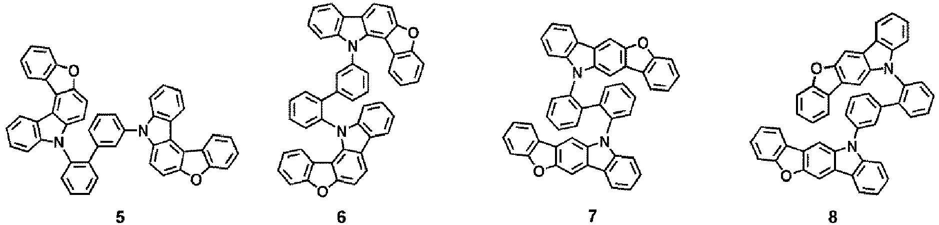

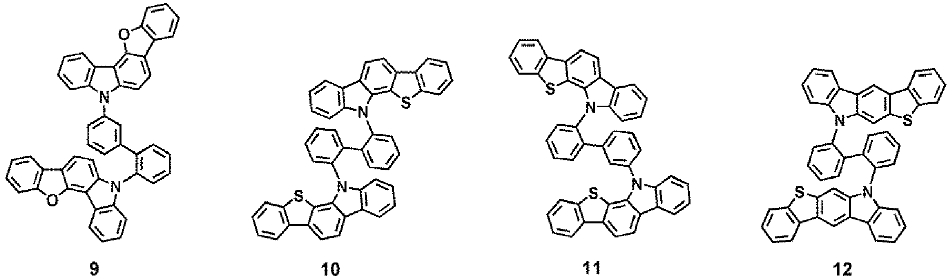

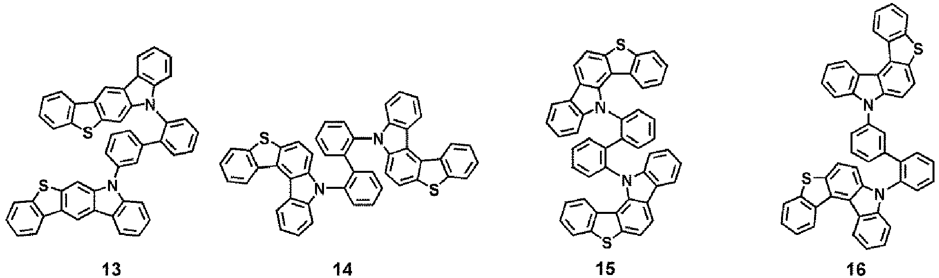

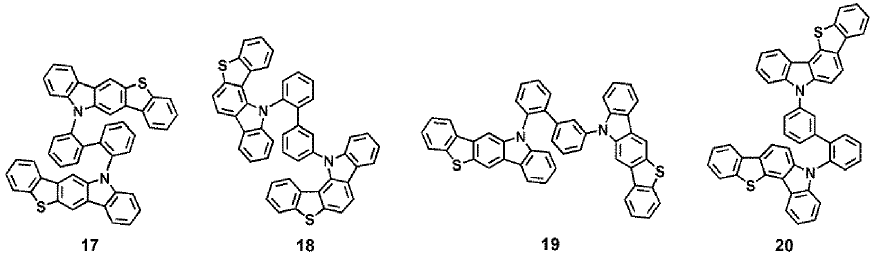

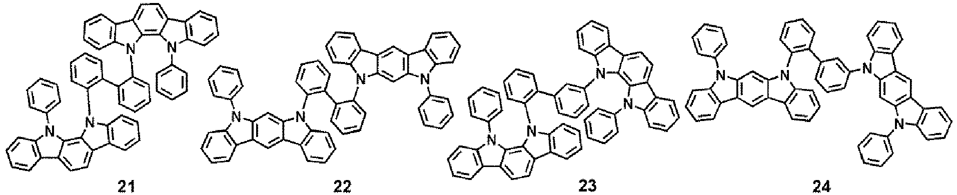

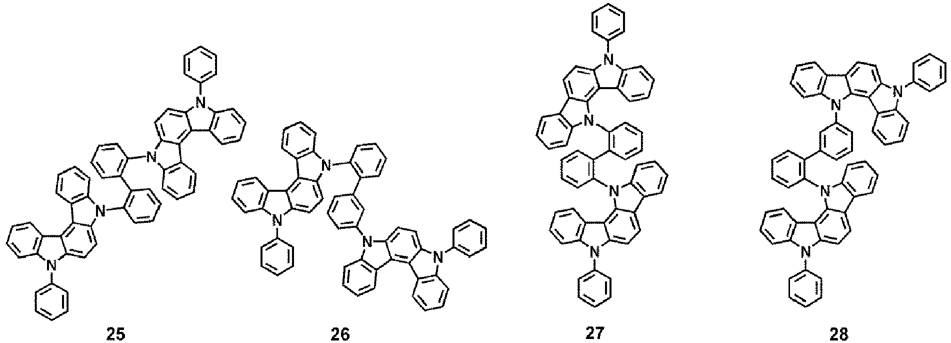

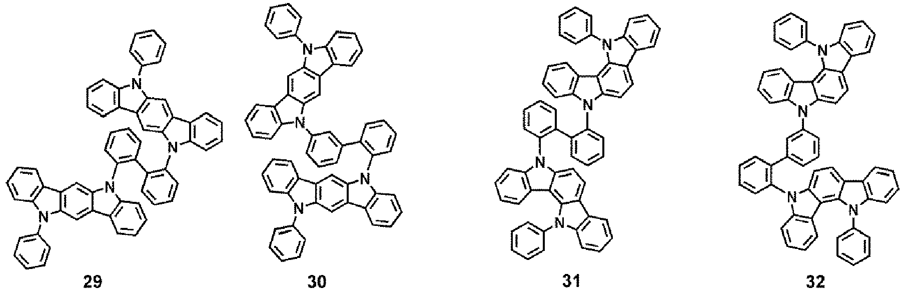

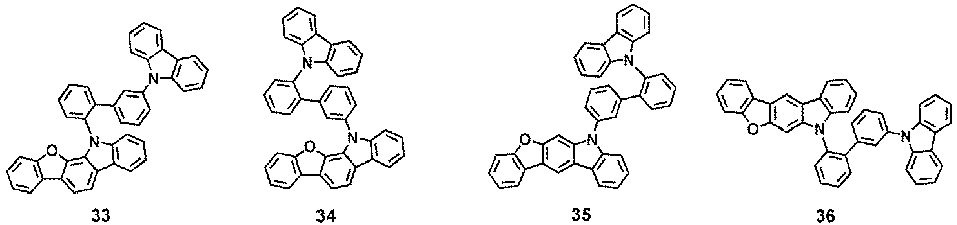

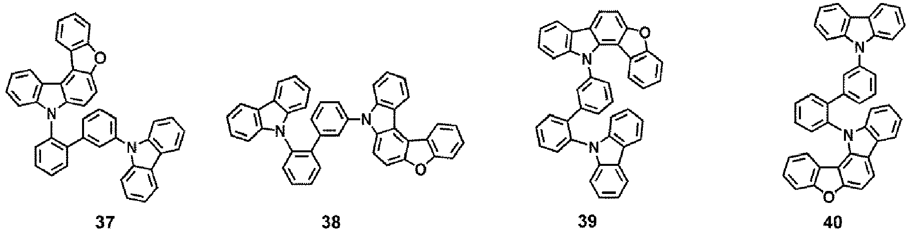

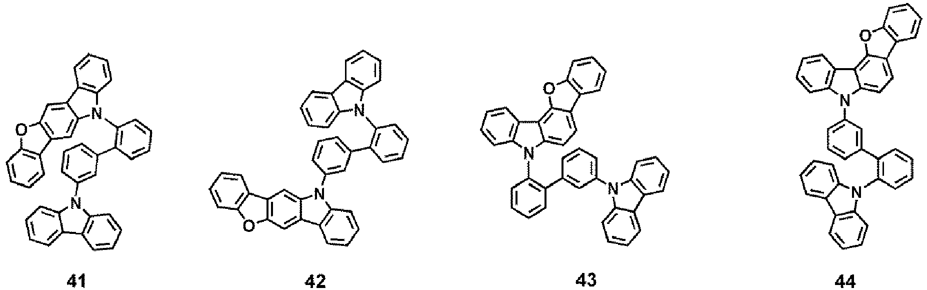

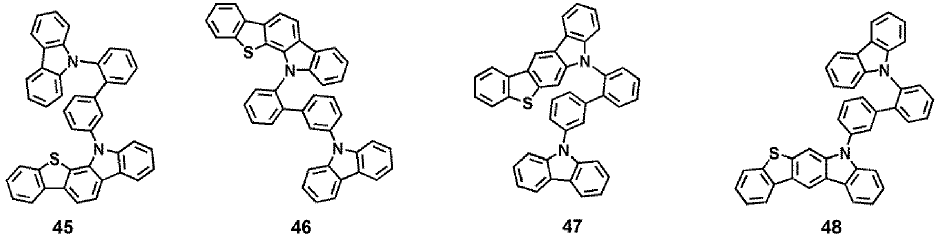

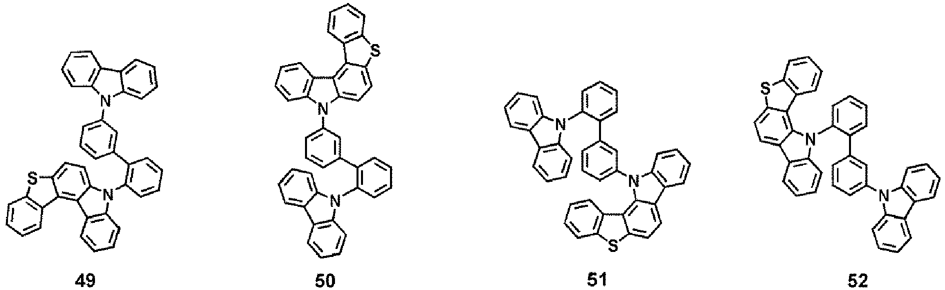

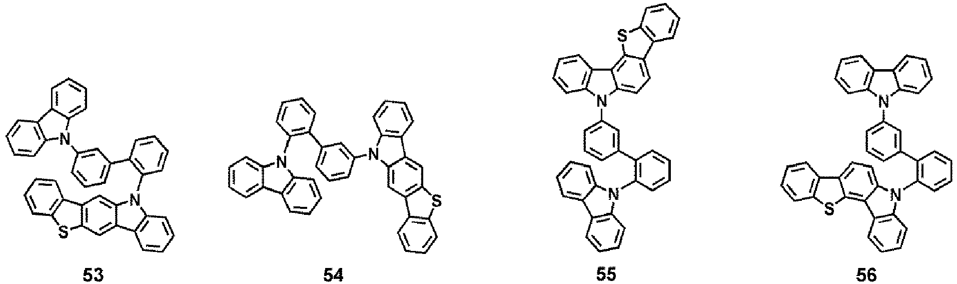

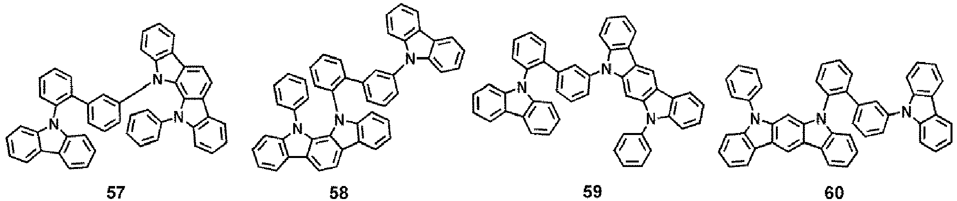

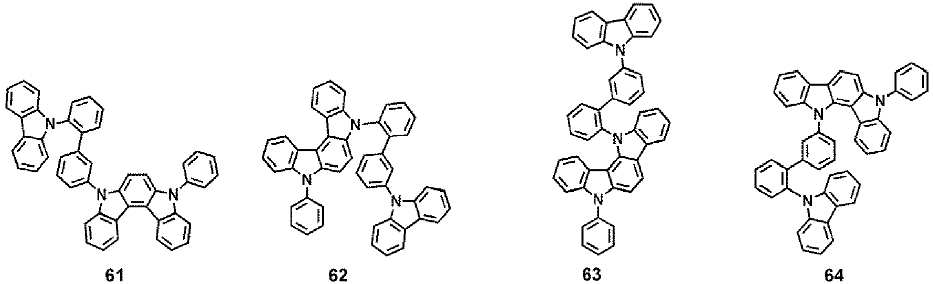

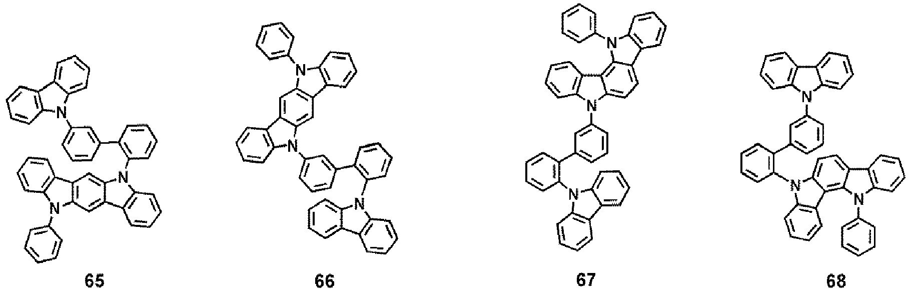

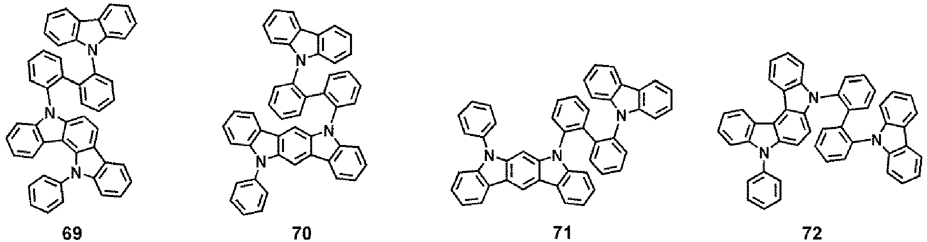

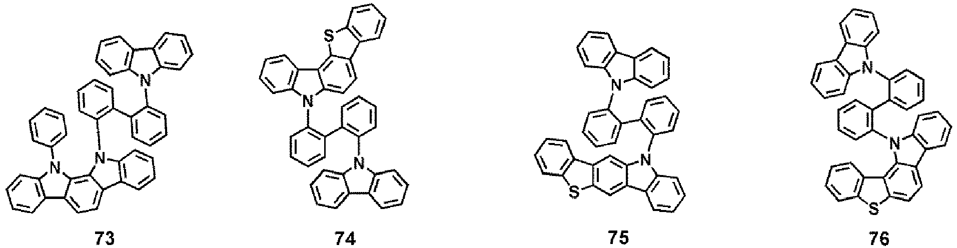

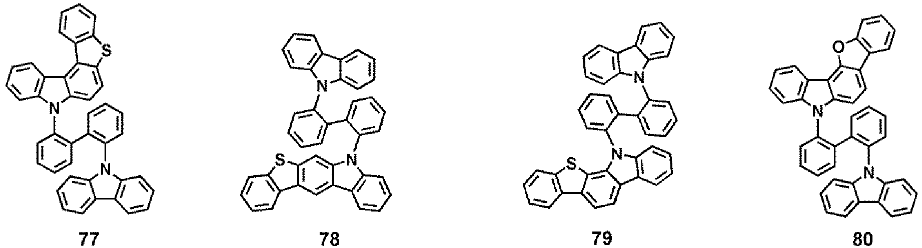

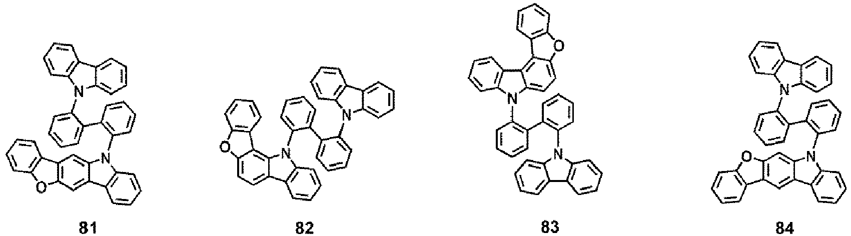

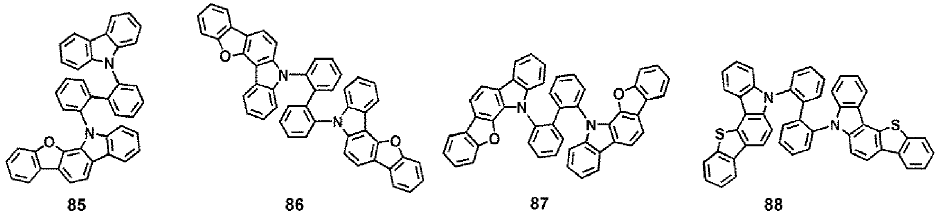

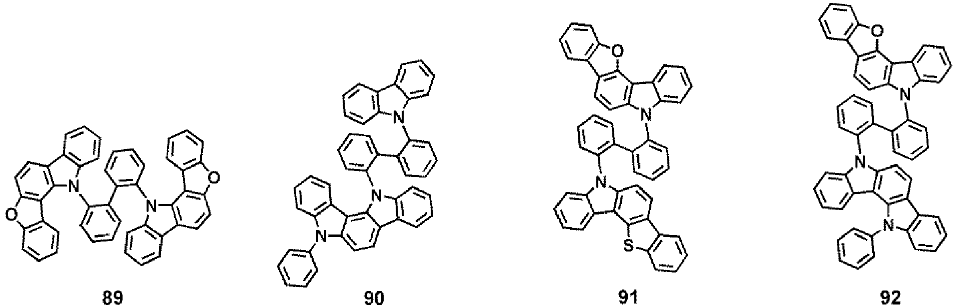

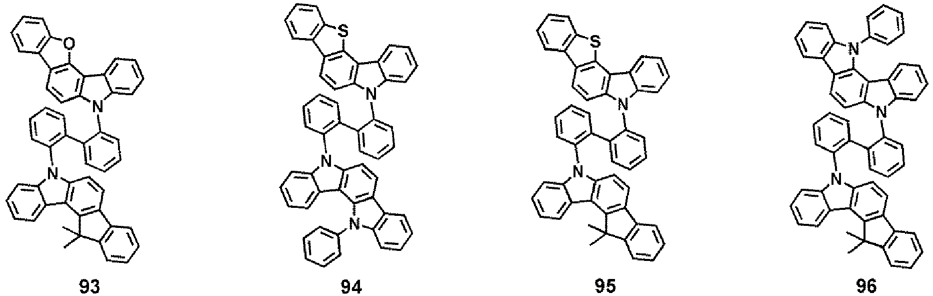

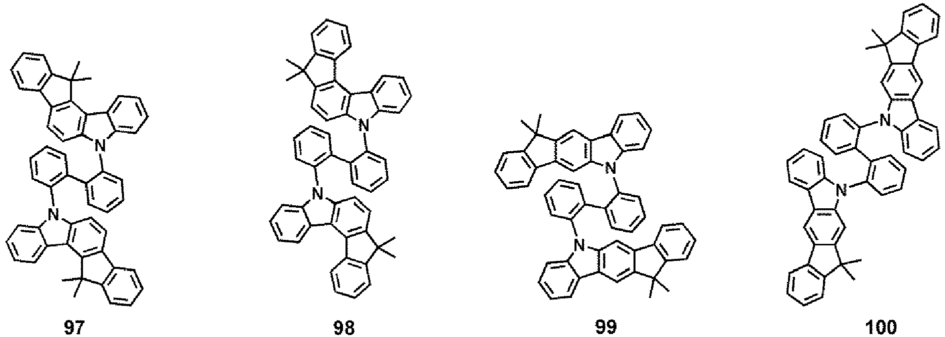

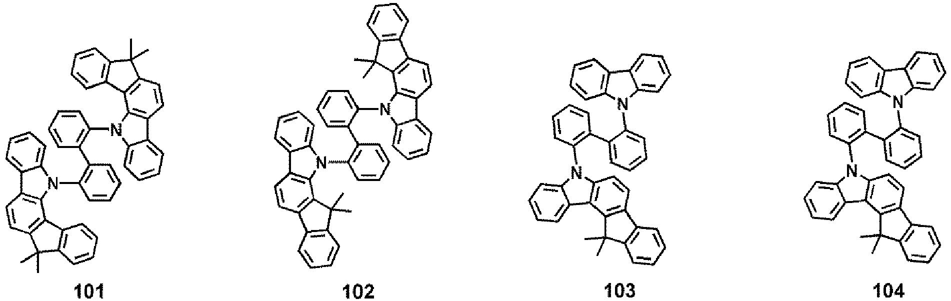

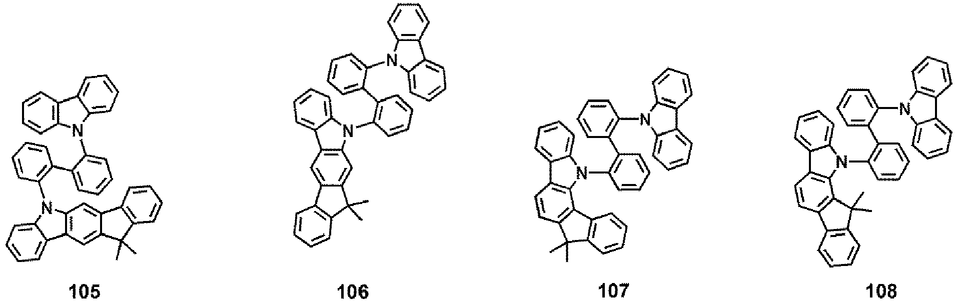

8. An organic light-emitting device comprising:
a first electrode;
a second electrode; and
an organic layer disposed between the first electrode and the second electrode and comprising an emission layer,
wherein the organic layer comprises one or more of the condensed cyclic compounds represented by Formulae 1-1 to 1-3 of any of claims 1 to 7.

9. The organic light-emitting device of claim 8, wherein
the first electrode is an anode,
the second electrode is a cathode; and
the organic layer comprises a hole transport region disposed between the first electrode and the emission layer and an electron transport region disposed between the emission layer and the second electrode;
wherein the hole transport region comprises a hole injection layer, a hole transport layer, an electron blocking layer, or any combination thereof; and
wherein the electron transport region comprises a hole blocking layer, an electron transport layer, an electron injection layer, or any combination thereof.

10. The organic light-emitting device of claim 8 or 9, wherein
the emission layer comprises the condensed cyclic compound represented by Formulae 1-1 to 1-3.

11. The organic light-emitting device of any of claims 8 to 10, wherein
the emission layer comprises a host and a dopant,
the host comprises the condensed cyclic compound represented by Formulae 1-1 to 1-3, and
an amount of the host is greater than an amount of the dopant.

12. The organic light-emitting device of any of claims 9 to 11, wherein
the hole transport region comprises a hole transport layer, and
the hole transport layer comprises the condensed cyclic compound represented by Formulae 1-1 to 1-3.

13. The organic light-emitting device of any of claims 9 to 11, wherein
the hole transport region comprises the electron blocking layer, and
the electron blocking layer comprises the condensed cyclic compound represented by Formulae 1-1 to 1-3.

## Patentansprüche

1. Kondensierte zyklische Verbindung, dargestellt durch eine der Formeln 1-1 bis 1-3: 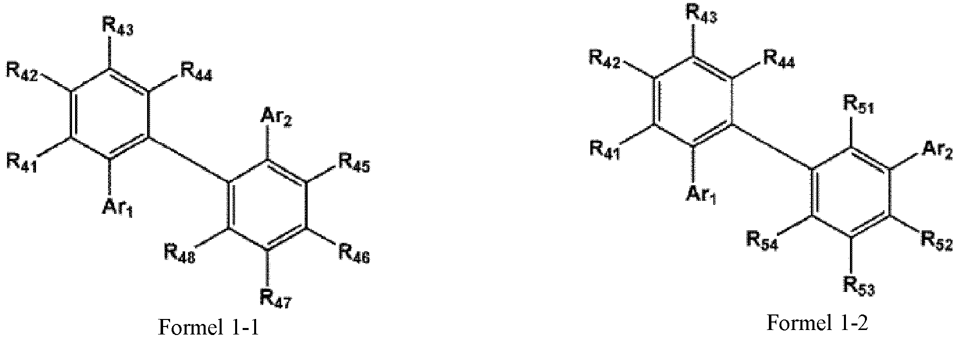 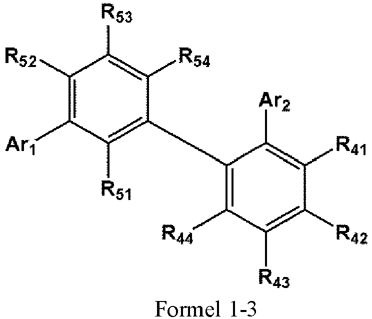 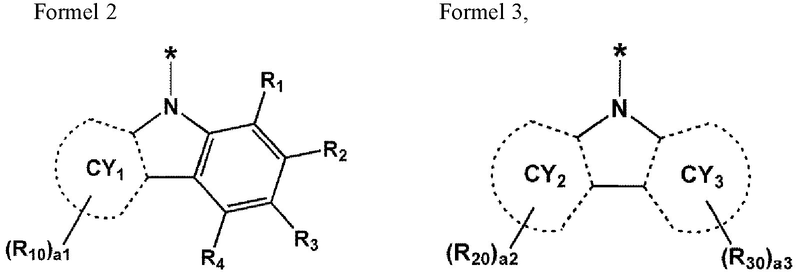 wobei
Ar₁ eine durch die Formel 2 dargestellte Gruppe ist,
Ar₂ eine durch die Formel 3 dargestellte Gruppe ist,
CY₁ ausgewählt ist aus einer Fluorengruppe, einer Carbazolgruppe, einer Dibenzofurangruppe und einer Dibenzothiophengruppe,
CY₂ und CY₃ jeweils unabhängig voneinander ausgewählt sind aus einer Benzolgruppe, einer Fluorengruppe, einer Carbazolgruppe, einer Dibenzofurangruppe und einer Dibenzothiophengruppe,
R₁ bis R₄, R₁₀, R₂₀ und R₃₀ jeweils unabhängig ausgewählt sind aus einem Wasserstoff, einem Deuterium, einer Hydroxylgruppe, einer Cyangruppe (CN), einer Nitrogruppe, einer Aminogruppe, einer Amidinogruppe, einer Hydrazingruppe, einer Hydrazongruppe, einer Carbonsäuregruppe oder einem Salz davon, eine Sulfonsäuregruppe oder einem Salz davon, einer Phosphorsäuregruppe oder einem Salz davon, einer substituierten oder unsubstituierten C₁-C₆₀-Alkylgruppe, einer substituierten oder unsubstituierten C₂-C₆₀-Alkenylgruppe, einer substituierten oder unsubstituierten C₂-C₆₀-Alkynylgruppe, einer substituierten oder unsubstituierten C₁-C₆₀-Alkoxygruppe, einer substituierten oder unsubstituierten C₃-C₁₀-Cycloalkylgruppe, einer substituierten oder unsubstituierten C₁-C₁₀-Heterocycloalkylgruppe, einer substituierten oder unsubstituierten C₃-C₁₀-Cycloalkenylgruppe, einer substituierten oder unsubstituierten Ci-Cio-Heterocycloalkenylgruppe, einer substituierten oder unsubstituierten C₆-C₆₀-Arylgruppe, einer substituierten oder unsubstituierten C₆-C₆₀-Aryloxygruppe, einer substituierten oder unsubstituierten C₆-C₆₀-Arylthiogruppe, einer substituierten oder unsubstituierten C₇-C₆₀-Arylalkylgruppe, einer substituierten oder unsubstituierten C₁-C₆₀-Heteroarylgruppe, einer substituierten oder unsubstituierten C₂-C₆₀-Heteroaryloxygruppe, einer substituierten oder unsubstituierten C₂-C₆₀-Heteroarylthiogruppe, einer substituierten oder unsubstituierten C₃-C₆₀-Heteroarylalkylgruppe, einer substituierten oder unsubstituierten einwertigen nichtaromatischen kondensierten polyzyklischen Gruppe, einer substituierten oder unsubstituierten einwertigen monovalent nichtaromatischen kondensierten heteropolyzyklischen Gruppe, -Si(Q₁)(Q₂)(Q₃), - N(Q₄)(Q₅) und -B(Q₆)(Q₇),
R₄₁₋₄₈ und R₅₁₋₅₄ jeweils unabhängig ausgewählt sind aus einem Wasserstoff, einem Deuterium, einer Hydroxylgruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer substituierten oder unsubstituierten C₁-C₆₀Alkylgruppe, einer substituierten oder unsubstituierten C₂-C₆₀Alkenylgruppe, einer substituierten oder unsubstituierten C₂-C₆₀Alkynylgruppe, einer substituierten oder unsubstituierten C₁-C₆₀-Alkoxygruppe, einer substituierten oder unsubstituierten C₃-C₁₀-Cycloalkylgruppe, einer substituierten oder unsubstituierten C₁-C₁₀-Heterocycloalkylgruppe, einer substituierten oder unsubstituierten C₃-C₁₀-Cycloalkenylgruppe, einer substituierten oder unsubstituierten C₁-C₁₀-Heterocycloalkenylgruppe, einer substituierten oder unsubstituierten C₆-C₆₀-Arylgruppe, einer substituierten oder unsubstituierten C₆-C₆₀-Aryloxygruppe, einer substituierten oder unsubstituierten C₆-C₆₀-Arylthiogruppe, einer substituierten oder unsubstituierten C₇-C₆₀-Arylalkylgruppe, einer substituierten oder unsubstituierten C₁-C₆₀-Heteroarylgruppe, einer substituierten oder unsubstituierten C₂-C₆₀-Heteroaryloxygruppe, einer substituierten oder unsubstituierten C₂-C₆₀-Heteroarylthiogruppe, einer substituierten oder unsubstituierten C₃-C₆₀-Heteroarylalkylgruppe, einer substituierten oder unsubstituierten einwertigen nichtaromatischen kondensierten polyzyklischen Gruppe, einer substituierten oder unsubstituierten einwertigen nichtaromatischen kondensierten heteropolyzyklischen Gruppe, -Si(Q₁)(Q₂)(Q₃), -N(Q₄)(Q₅) und -B(Q₆)(Q₇),
a1 bis a3 jeweils unabhängig eine ganze Zahl von 0 bis 10 sind,
* eine Bindungsstelle an ein benachbartes Atom ist,
mindestens ein Substituent der substituierten C₁-C₆₀-Alkylgruppe, substituierten C₂-C₆₀-Alkenylgruppe, substituierten C₂-C₆₀-Alkynylgruppe, substituierten C₃-C₁₀-Cycloalkylgruppe, substituierten C₁-C₁₀-Heterocycloalkylgruppe, substituierten C₃-C₁₀-Cycloalkenylgruppe, substituierten C₁-C₁₀-Heterocycloalkenylgruppe, substituierten C₆-C₆₀-Arylgruppe, substituierten C₆-C₆₀-Aryloxygruppe, substituierten C₆-C₆₀-Arylthiogruppe, substituierten C₇-C₆₀-Arylalkylgruppe, substituierten C₁-C₆₀-Heteroarylgruppe, substituierten C₂-C₆₀-Heteroaryloxygruppe, substituierten C₂-C₆₀-Heteroarylthiogruppe, substituierten C₃-C₆₀-Heteroarylalkylgruppe, substituierten einwertigen nichtaromatischen kondensierten polyzyklischen Gruppe und substituierten einwertigen nichtaromatischen kondensierten heteropolyzyklischen Gruppe ausgewählt ist aus:
einem Deuterium, CD₃, -CD₂H, -CDH₂, einer Hydroxylgruppe, einer Cyangruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₆₀-Alkylgruppe, einer C₂-C₆₀-Akenylgruppe, einer C₂-C₆₀-Alkynylgruppe und einer C₁-C₆₀-Alkoxygruppe;
einer C₁-C₆₀-Alkylgruppe, einer C₂-C₆₀-Alkenylgruppe, einer C₂-C₆₀-Alkynylgruppe und einer C₁-C₆₀-Alkoxygruppe, jeweils substituiert mit mindestens einem, ausgewählt aus einem Deuterium, -CD₃, -CD₂H, -CDH₂, einer Hydroxylgruppe, einer Cyangruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₇-C₆₀-Arylalkylgruppe, einer C₁-C₆₀-Heteroarylgruppe, einer C₂-C₆₀-Heteroaryloxygruppe, einer C₂-C₆₀-Heteroarylthiogruppe, einer C₃-C₆₀-Heteroarylalkylgruppe, einer einwertigen nichtaromatischen kondensierten polyzyklischen Gruppe, einer einwertigen nichtaromatischen kondensierten heteropolyzyklischen Gruppe, -Si(Q₁₁)(Q₁₂)(Q₁₃), -N(Q₁₄)(Q₁₅) und -B(Q₁₆)(Q₁₇);
einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₇-C₆₀-Arylalkylgruppe, einer C₁-C₆₀-Heteroarylgruppe, einer C₂-C₆₀-Heteroaryloxygruppe, einer C₂-C₆₀-Heteroarylthiogruppe, einer C₃-C₆₀-Heteroarylalkylgruppe, einer einwertigen nichtaromatischen kondensierten polyzyklischen Gruppe, einer einwertigen nichtaromatischen kondensierten heteropolyzyklischen Gruppe;
einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₇-C₆₀-Arylalkylgruppe, einer C₁-C₆₀-Heteroarylgruppe, einer C₂-C₆₀-Heteroaryloxygruppe, einer C₂-C₆₀-Heteroarylthiogruppe, einer C₃-C₆₀-Heteroarylalkylgruppe einer einwertigen nichtaromatischen kondensierten polyzyklischen Gruppe und einer einwertigen nichtaromatischen kondensierten heteropolyzyklischen, jeweils substituiert mit mindestens einem, ausgewählt aus Deuterium, -CD₃, -CD₂H, -CDH₂, einer Hydroxylgruppe, einer Cyangruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₆₀-Alkylgruppe, einer C₂-C₆₀-Alkenylgruppe, einer C₂-C₆₀-Alkynylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₇-C₆₀-Arylalkylgruppe, einer C₁-C₆₀-Heteroarylgruppe, einer C₂-C₆₀-Heteroaryloxygruppe, einer C₂-C₆₀-Heteroarylthiogruppe, einer C₃-C₆₀-Heteroarylalkylgruppe, einer einwertigen nichtaromatischen kondensierten polyzyklischen Gruppe, einer einwertigen nichtaromatischen kondensierten heteropolyzyklischen Gruppe, -Si(Q₂₁)(Q₂₂)(Q₂₃), -N(Q₂₄)(Q₂₅) und -B(Q₂₆)(Q₂₇); und
-Si(Q₃₁)(Q₃₂)(Q₃₃), -N(Q₃₄)(Q₃₅) und -B(Q₃₆)(Q₃₇), und
Q₁ bis Q₇, Q₁₁ bis Q₁₇, Q₂₁ bis Q₂₇ und Q₃₁ bis Q₃₇ jeweils unabhängig ausgewählt sind aus einem Wasserstoff, einem Deuterium, einer Hydroxylgruppe, einer Cyangruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer substituierten oder unsubstituierten C₁-C₆₀-Alkylgruppe, einer substituierten oder unsubstituierten C₂-C₆₀-Alkenylgruppe, einer substituierten oder unsubstituierten C₂-C₆₀-Alkynylgruppe, einer substituierten oder unsubstituierten C₁-C₆₀-Alkoxygruppe, einer substituierten oder unsubstituierten C₃-C₁₀-Cycloalkylgruppe, einer substituierten oder unsubstituierten C₁-C₁₀-Heterocycloalkylgruppe, einer substituierten oder unsubstituierten C₃-C₁₀-Cycloalkenylgruppe, einer substituierten oder unsubstituierten C₁-C₁₀-Heterocycloalkenylgruppe, einer substituierten oder unsubstituierten C₆-C₆₀-Arylgruppe,einer substituierten oder unsubstituierten C₆-C₆₀-Aryloxygruppe, einer substituierten oder unsubstituierten C₆-C₆₀-Arylthiogruppe, einer substituierten oder unsubstituierten C₇-C₆₀-Arylalkylgruppe, einer substituierten oder unsubstituierten C₁-C₆₀-Heteroarylgruppe, einer substituierten oder unsubstituierten C₂-C₆₀-Heteroaryloxygruppe, einer substituierten oder unsubstituierten C₂-C₆₀-Heteroarylthiogruppe, einer substituierten oder unsubstituierten C₃-C₆₀-Heteroarylalkylgruppe, einer substituierten oder unsubstituierten einwertigen nichtaromatischen kondensierten polyzyklischen Gruppe, einer substituierten oder unsubstituierten einwertigen nichtaromatischen kondensierten heteropolyzyklischen Gruppe.

2. Kondensierte zyklische Verbindung nach Anspruch 1, wobei
Ar₁ ausgewählt ist aus Gruppen, die durch die Formeln 2-1 bis 2-6 dargestellt werden, und
Ar₂ ausgewählt ist aus Gruppen, die durch die Formeln 3-1 bis 3-7 dargestellt werden: 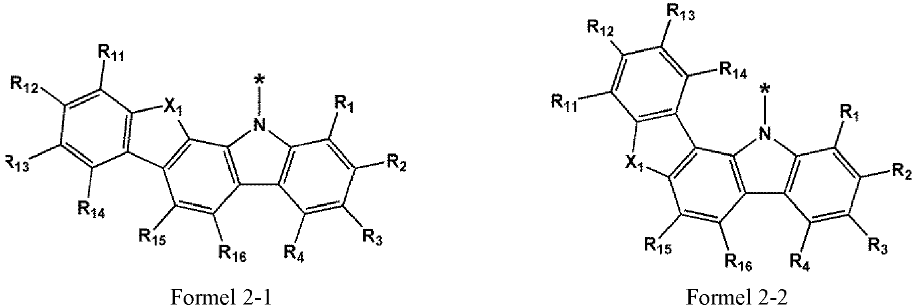 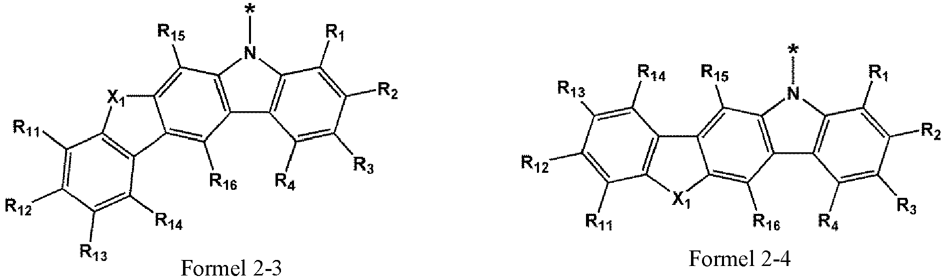 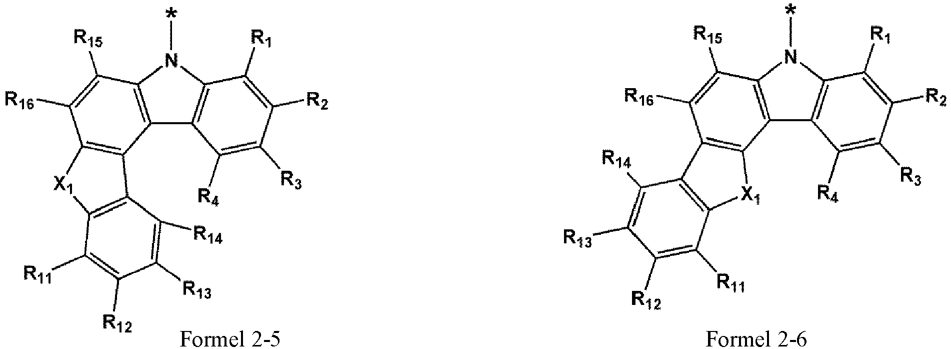 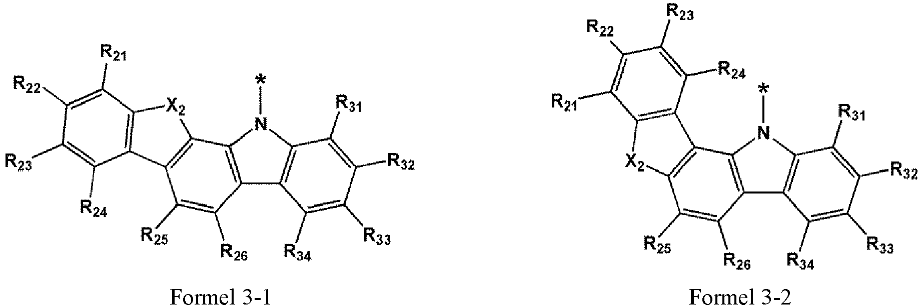 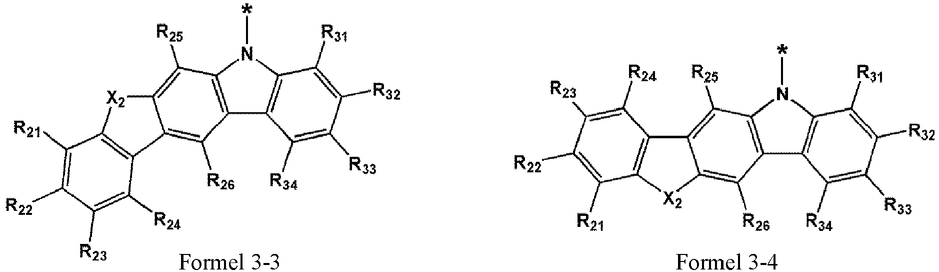 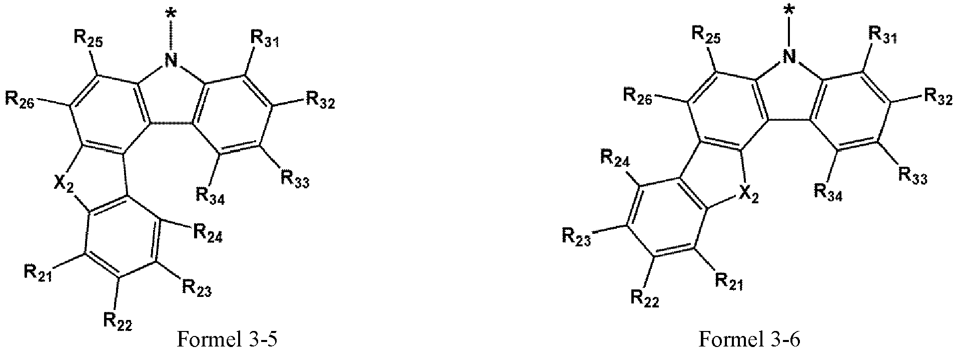 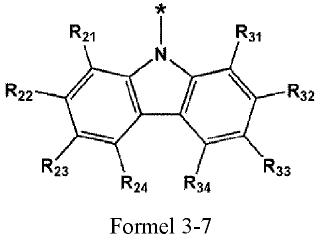 in den Formeln 2-1 bis 2-6 und 3-1 bis 3-7
X₁ C(R₁₇)(R₁₈), N(R₁₉), O oder S ist,
X₂ C(R₂₇)(R₂₈), N(R₂₉), O oder S ist,
R₁ bis R₄, R₁₁ bis R₁₉, R₂₁ bis R₂₉ und R₃₁ bis R₃₄ jeweils unabhängig ausgewählt sind aus
einem Wasserstoff, einem Deuterium, einer Hydroxylgruppe, einer Cyangruppe (CN), einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₂₀-Alkylgruppe, und einer C₁-C₂₀-Alkoxygruppe;
einer C₁-C₂₀-Alkylgruppe und einer C₁-C₂₀-Alkoxygruppe, jeweils substituiert mit mindestens einem, ausgewählt aus einem Deuterium, einer Hydroxylgruppe, einer Cyangruppe (CN), einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer Phenylgruppe, einer Biphenylgruppe, einer Terphenylgruppe und einer Naphthylgruppe;
einer Cyclopentylgruppe, einer Cyclohexylgruppe, einer Cyclopentenylgruppe, einer Cyclohexenylgruppe, einer Cycloheptenylgruppe, einer Phenylgruppe, einer Biphenylgruppe, einer Terphenylgruppe, einer Pentalenylgruppe, einer Indenylgruppe, einer Naphthylgruppe, einer Azulenylgruppe, einer Heptalenylgruppe, einer Indacenylgruppe, einer Acenaphthylgruppe, einer Fluorenylgruppe, einer Spirobifluorenylgruppe, einer Phenalenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Naphthacenylgruppe, einer Picenylgruppe, einer Perylenylgruppe, einer Pentaphenylgruppe, einer Hexacenylgruppe, einer Pyrrolylgruppe, einer Imidazolylgruppe, einer Pyrazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Quinolinylgruppe, einer Isoquinolinylgruppe, einer Benzoquinolinylgruppe, einer Phthalazinylgruppe, einer Naphthyridinylgruppe, einer Quinoxalinylgruppe, einer Quinazolinylgruppe, einer Cinnolinylgruppe, einer Carbazolylgruppe, einer Phenanthridinylgruppe, einer Acridinylgruppe, einer Phenanthrolinylgruppe, einer Phenazinylgruppe, einer Benzooxazolylgruppe, einer Benzoimidazolylgruppe, einer Furanylgruppe, einer Benzofuranylgruppe, einer Thiophenylgruppe, einer Benzothiophenylgruppe, einer Thiazolylgruppe, einer Isothiazolylgruppe, einer Benzothiazolylgruppe, einer Isoxazolylgruppe, einer Oxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Benzocarbazolylgruppe, einer Dibenzocarbazolylgruppe, einer Imidazopyrimidinylgruppe, einer Imidazopyridinylgruppe, einer Pyridoindolylgruppe, einer Benzofuropyridinylgruppe, einer Benzothienopyridinylgruppe, einer Pyrimidoindolylgruppe, einer Benzofuropyrimidinylgruppe, einer Benzothienopyrimidinylgruppe, einer Phenoxazinylgruppe, einer Pyridobenzooxazinylgruppe und einer Pyridobenzothiazinylgruppe;
einer Cyclopentylgruppe, einer Cyclohexylgruppe, einer Cyclopentenylgruppe, einer Cyclohexenylgruppe, einer Cycloheptenylgruppe, einer Phenylgruppe, einer Biphenylgruppe, einer Terphenylgruppe, einer Pentalenylgruppe, einer Indenylgruppe, einer Naphthylgruppe, einer Azulenylgruppe, einer Heptalenylgruppe, einer Indacenylgruppe, einer Acenaphthylgruppe, einer Fluorenylgruppe, einer Spiro-Fluorenylgruppe, einer Phenalenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Naphthacenylgruppe, einer Picenylgruppe, einer Perylenylgruppe, einer Pentaphenylgruppe, einer Hexacenylgruppe, einer Pyrrolylgruppe, einer Imidazolylgruppe, einer Pyrazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Quinolinylgruppe, einer Isoquinolinylgruppe, einer Benzoquinolinylgruppe, einer Phthalazinylgruppe, einer Naphthyridinylgruppe, einer Quinoxalinylgruppe, einer Quinazolinylgruppe, einer Cinnolinylgruppe, einer Carbazolylgruppe, einer Phenanthridinylgruppe, einer Acridinylgruppe, einer Phenanthrolinylgruppe, einer Phenazinylgruppe, einer Benzoxazolylgruppe, einer Benzoimidazolylgruppe, einer Furanylgruppe, einer Benzofuranylgruppe, einer Thiophenylgruppe, einer Benzothiophenylgruppe, einer Thiazolylgruppe, einer Isothiazolylgruppe, einer Benzothiazolylgruppe, einer Isoxazolylgruppe, einer Oxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Benzocarbazolylgruppe, einer Dibenzocarbazolylgruppe, einer Imidazopyrimidinylgruppe, einer Imidazopyridinylgruppe, einer Pyridoindolylgruppe, einer Benzofuropyridinylgruppe, einer Benzothienopyridinylgruppe, einer Pyrimidoindolylgruppe, einer Benzofuropyrimidinylgruppe, einer Benzothienopyrimidinylgruppe, einer Phenoxazinylgruppe, einer Pyridobenzooxazinylgruppe und einer Pyridobenzothiazinylgruppe, jeweils substituiert mit mindestens einem, ausgewählt aus einem Deuterium, einer Hydroxylgruppe, einer Cyangruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₂₀-Alkylgruppe, einer C₂-C₂₀-Alkenylgruppe, einer C₂-C₂₀-Akynylgruppe, einer C₁-C₂₀-Alkoxygruppe, einer Phenylgruppe, einer Biphenylgruppe, einer Terphenylgruppe, einer Naphthylgruppe, einer Anthracenylgruppe, einer Pyrenylgruppe, einer Phenanthrenylgruppe, einer Fluorenylgruppe, einer Carbazolylgruppe, einer Benzocarbazolylgruppe, einer Dibenzocarbazolylgruppe, einer Pyridinylgruppe, einer Pyrimidinylgruppe, einer Pyrazinylgruppe, einer Pyridazinylgruppe, einer Triazinylgruppe, einer Quinolinylgruppe, einer Isoquinolinylgruppe, einer Phthalazinylgruppe, einer Quinoxalinylgruppe, einer Cinnolinylgruppe, einer Quinazolinylgruppe, -Si(Q₃₁)(Q₃₂)(Q₃₃), - N(Q₃₄)(Q₃₅) und -B(Q₃₆)(Q₃₇); und
-Si(Q₁)(Q₂)(Q₃), -N(Q₄)(Q₅) und -B(Q₆)(Q₇),
wobei Q₁ bis Q₇ und Q₃₁ bis Q₃₇ jeweils unabhängig ausgewählt sind aus einem Wasserstoff, einer C₁-C₂₀-Alkylgruppe, einer C₁-C₂₀-Alkoxygruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe und einer Dibenzothiophenylgruppe und
* eine Bindungsstelle an ein benachbartes Atom ist, wobei vorzugsweise
R₁ bis R₄, R₁₁ bis R₁₉, R₂₁ bis R₂₉ und R₃₁ bis R₃₄ jeweils unabhängig ausgewählt sind aus
einem Wasserstoff, einem Deuterium, einer Cyangruppe (CN), einer C₁-C₂₀-Alkylgruppe und einer C₁-C₂₀-Alkoxygruppe;
einer C₁-C₂₀-Alkylgruppe und einer C₁-C₂₀-Alkoxygruppe, jeweils substituiert mit mindestens einem, ausgewählt aus einem Deuterium, einer Cyangruppe (CN), einer Phenylgruppe, einer Biphenylgruppe, einer Terphenylgruppe und einer Naphthylgruppe;
einer Cyclopentylgruppe, einer Cyclohexylgruppe, einer Cyclopentenylgruppe, einer Cyclohexenylgruppe, eine Cycloheptenylgruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe und einer Dibenzothiophenylgruppe;
einer Cyclopentylgruppe, einer Cyclohexylgruppe, einer Cyclopentenylgruppe, einer Cyclohexenylgruppe, einer Cycloheptenylgruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe und einer Dibenzothiophenylgruppe, jeweils substituiert mit mindestens einem, ausgewählt aus einem Deuterium, einer Cyangruppe, einer C₁-C₂₀-Alkylgruppe, einer C₁-C₂₀-Alkoxygruppe, einer Phenylgruppe, einer Biphenylgruppe, einer Terphenylgruppe, einer Naphthylgruppe und -Si(Q₃₁)(Q₃₂)(Q₃₃); und
-Si(Q₁)(Q₂)(Q₃) und
Q₁ bis Q₃ und Q₃₁ bis Q₃₃ jeweils unabhängig ausgewählt sind aus einem Wasserstoffatom, einer C₁-C₂₀-Alkylgruppe, einer C₁-C₂₀-Alkoxygruppe, einer Phenylgruppe, einer Biphenylgruppe, einer Terphenylgruppe und einer Naphthylgruppe und/oder wobei vorzugsweise
X₁ O oder S ist.

3. Kondensierte zyklische Verbindung nach Anspruch 1 oder 2, wobei
Ar₁ dasselbe wie Ar₂ in den Formeln 1-1 bis 1-3 ist; oder
CY₂ und CY₃ in der Formel 3 jeweils unabhängig eine Benzolgruppe sind.

4. Kondensierte zyklische Verbindung nach Anspruch 2, wobei
Ar₁ durch die Formel 2-1 dargestellt wird und Ar₂ durch die Formel 3-1 dargestellt wird;
Ar₁ durch die Formel 2-2 dargestellt wird und Ar₂ durch die Formel 3-2 dargestellt wird; oder
Ar₁ durch die Formel 2-6 dargestellt wird und Ar₂ durch die Formel 3-6 dargestellt wird.

5. Kondensierte zyklische Verbindung nach einem der Ansprüche 1 bis 4, wobei
die kondensierte zyklische Verbindung durch die Formel 1-1 dargestellt wird und/oder wobei R₄₁ bis R₄₈ und R₅₁ bis R₅₄ jeweils unabhängig ausgewählt sind aus einem Wasserstoff, einem Deuterium, einer C₁-C₂₀-Alkylgruppe und einer C₁-C₂₀-Alkoxygruppe; einer C₁-C₂₀-Alkylgruppe und einer C₁-C₂₀-Alkoxygruppe, jeweils substituiert mit mindestens einem, ausgewählt aus einem Deuterium, einer Phenylgruppe, einer Biphenylgruppe, einer Terphenylgruppe und einer Naphthylgruppe;
einer Cyclopentylgruppe, einer Cyclohexylgruppe, einer Cyclopentenylgruppe, einer Cyclohexenylgruppe, eine Cycloheptenylgruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe und einer Dibenzothiophenylgruppe;
einer Cyclopentylgruppe, einer Cyclohexylgruppe, einer Cyclopentenylgruppe, einer Cyclohexenylgruppe, einer Cycloheptenylgruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe und einer Dibenzothiophenylgruppe, jeweils substituiert mit mindestens einem, ausgewählt aus einem Deuterium, einer C₁-C₂₀-Alkylgruppe, einer C₁-C₂₀-Alkoxygruppe, einer Phenylgruppe, einer Biphenylgruppe, einer Terphenylgruppe, einer Naphthylgruppe und -Si(Q₃₁)(Q₃₂)(Q₃₃); und -Si(Q₁)(Q₂)(Q₃) und
Q₁ bis Q₃ und Q₃₁ bis Q₃₃ jeweils unabhängig ausgewählt sind aus einem Wasserstoff, einer C₁-C₂₀-Alkylgruppe, einer C₁-C₂₀-Alkoxygruppe, einer Phenylgruppe, einer Biphenylgruppe, einer Terphenylgruppe und einer Naphthylgruppe.

6. Kondensierte zyklische Verbindung nach Anspruch 5, wobei
Ar₁ ausgewählt ist aus Gruppen, die durch die Formeln 2-1 bis 2-6 dargestellt werden,
Ar₂ ausgewählt ist aus Gruppen, die durch die Formeln 3-1 bis 3-7 dargestellt werden: 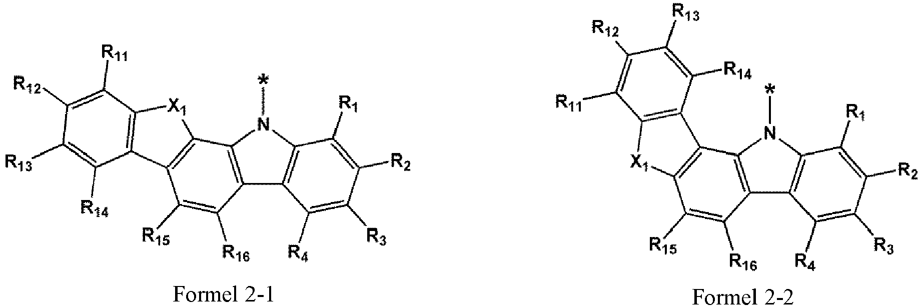 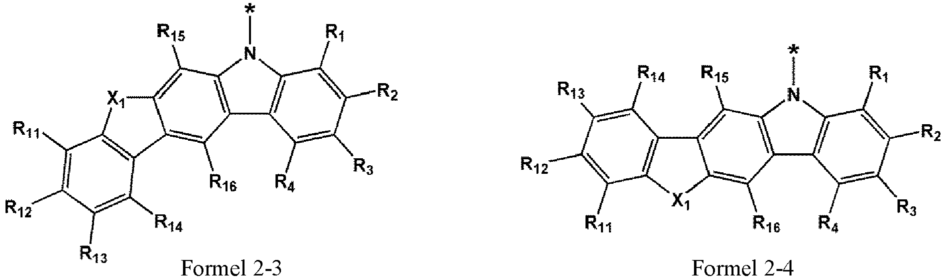 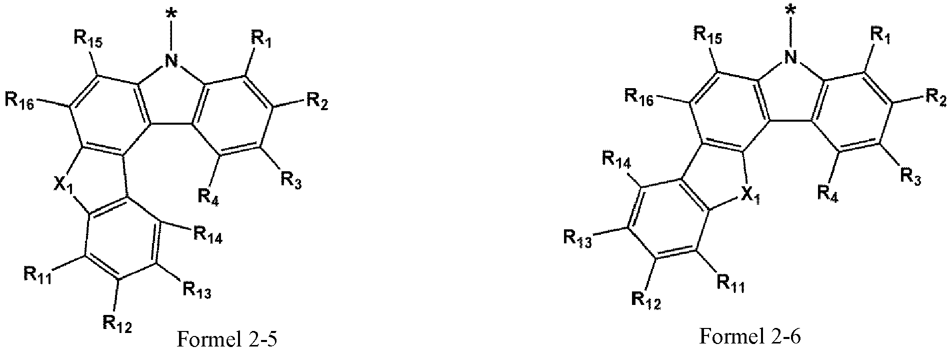 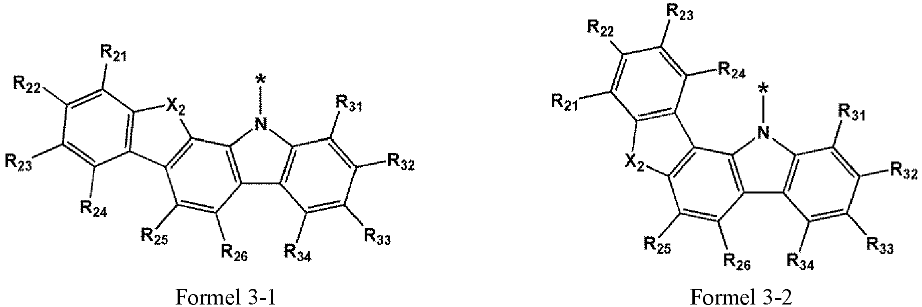 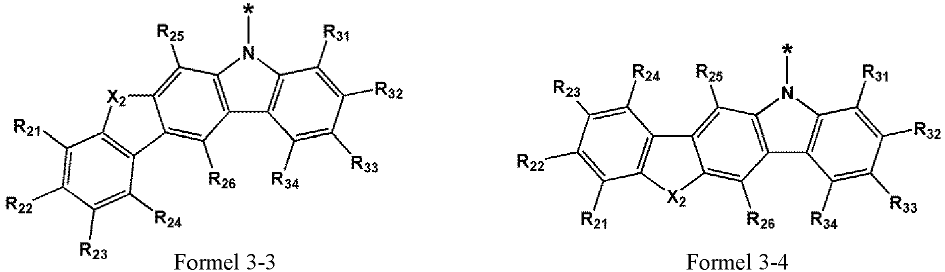 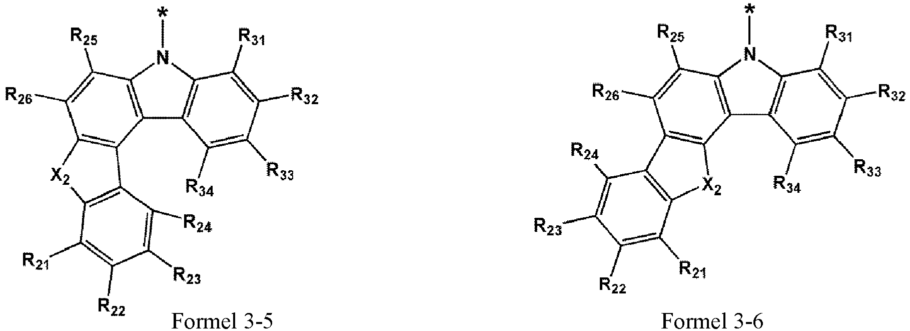 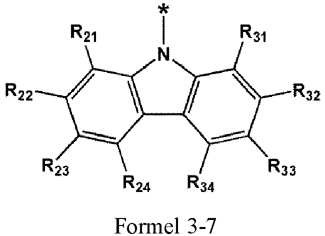 in den Formeln 2-1 bis 2-6 und 3-1 bis 3-7
X₁ C(R₁₇)(R₁₈), N(R₁₉), O oder S ist,
X₂ C(R₂₇)(R₂₈), N(R₂₉), O oder S ist,
R₁ bis R₄, R₁₁ bis R₁₉, R₂₁ bis R₂₉ und R₃₁ bis R₃₄ jeweils unabhängig ausgewählt sind aus
einem Wasserstoff, einem Deuterium, einer Cyangruppe (CN), einer C₁-C₂₀-Alkylgruppe und einer C₁-C₂₀-Alkoxygruppe;
einer C₁-C₂₀-Alkylgruppe und einer C₁-C₂₀-Alkoxygruppe, jeweils substituiert mit mindestens einem, ausgewählt aus einem Deuterium, einer Cyangruppe (CN), einer Phenylgruppe, einer Biphenylgruppe, einer Terphenylgruppe und einer Naphthylgruppe;
einer Cyclopentylgruppe, einer Cyclohexylgruppe, einer Cyclopentenylgruppe, einer Cyclohexenylgruppe, eine Cycloheptenylgruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe und einer Dibenzothiophenylgruppe;
einer Cyclopentylgruppe, einer Cyclohexylgruppe, einer Cyclopentenylgruppe, einer Cyclohexenylgruppe, einer Cycloheptenylgruppe, einer Phenylgruppe, einer Biphenylgruppe, einer Terphenylgruppe, einer Naphthylgruppe, einer Fluorenylgruppe, einer Carbazolylgruppe, einer Dibenzofuranylgruppe und einer Dibenzothiophenylgruppe, jeweils substituiert mit mindestens einem, ausgewählt aus einem Deuterium, einer Cyangruppe, einer C₁-C₂₀-Alkylgruppe, einer C₁-C₂₀-Alkoxygruppe, einer Phenylgruppe, einer Biphenylgruppe, einer Terphenylgruppe, einer Naphthylgruppe und -Si(Q₃₁)(Q₃₂)(Q₃₃); und -Si(Q₁)(Q₂)(Q₃),
wobei Q₁ bis Q₃ und Q₃₁ bis Q₃₃ jeweils unabhängig ausgewählt sind aus einem Wasserstoff, einer C₁-C₂₀-Alkylgruppe, einer C₁-C₂₀-Alkoxygruppe, einer Phenylgruppe, einer Biphenylgruppe, einer Terphenylgruppe und einer Naphthylgruppe, wobei vorzugsweise
die kondensierte zyklische Verbindung durch die Formel 1-1 dargestellt wird und X₁ in den Formeln 2-1 bis 2-6 ist O oder S ist.

7. Kondensierte zyklische Verbindung nach Anspruch 1, wobei
die kondensierte zyklische Verbindung ausgewählt ist aus den Verbindungen 1 bis 108:

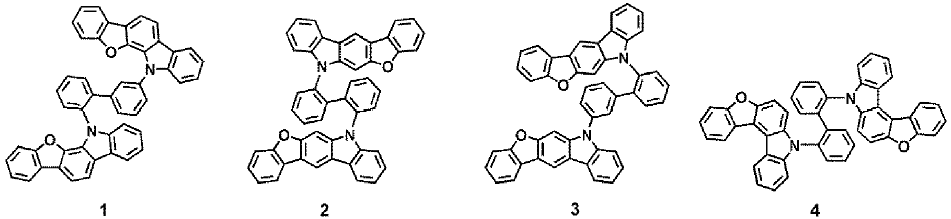

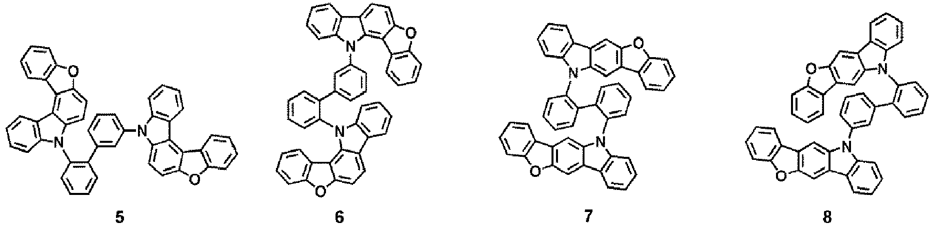

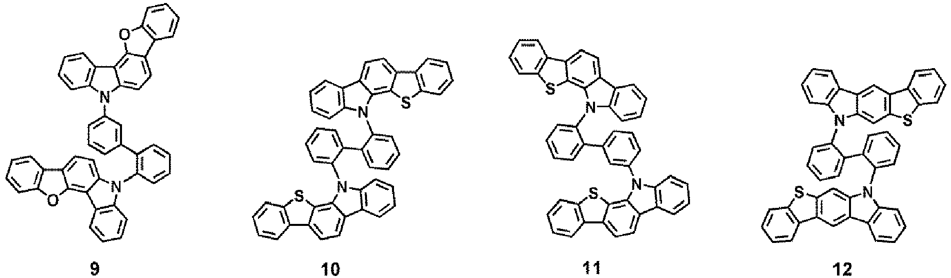

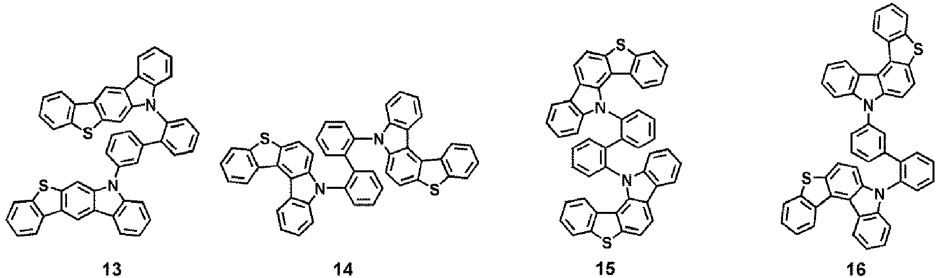

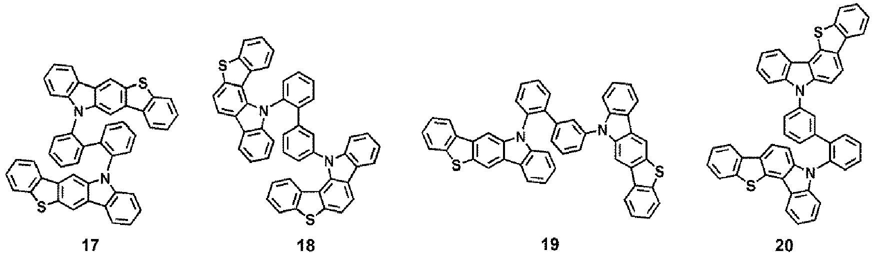

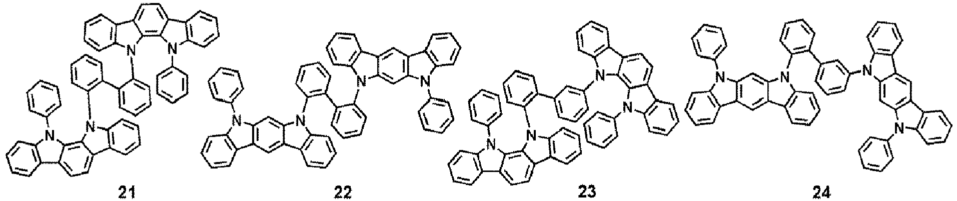

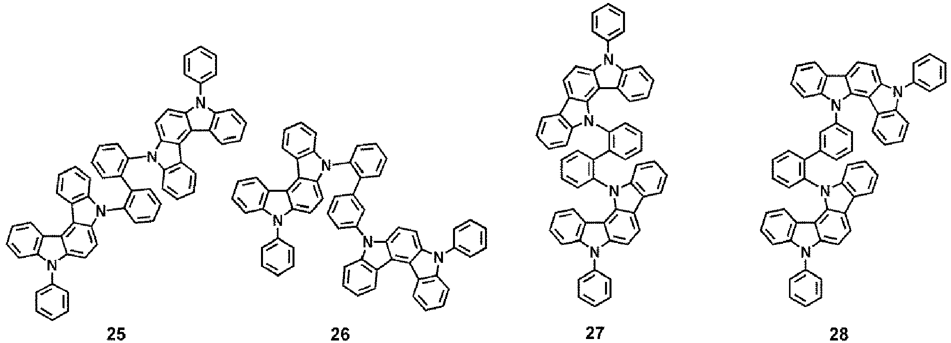

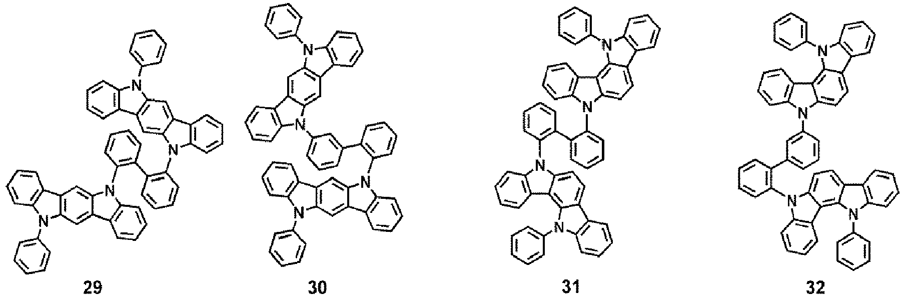

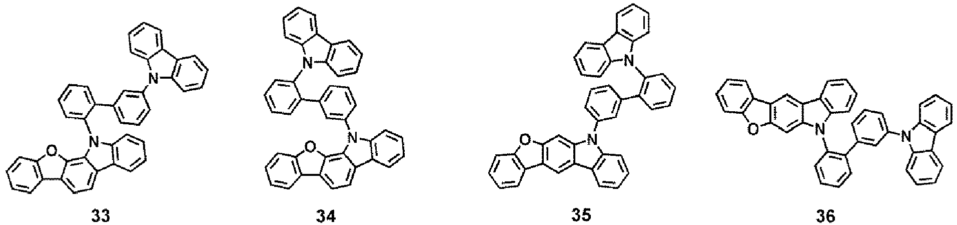

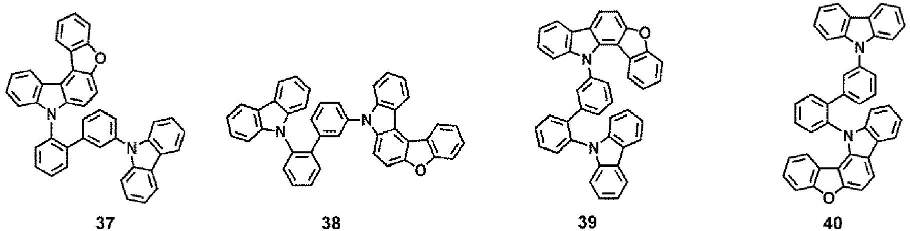

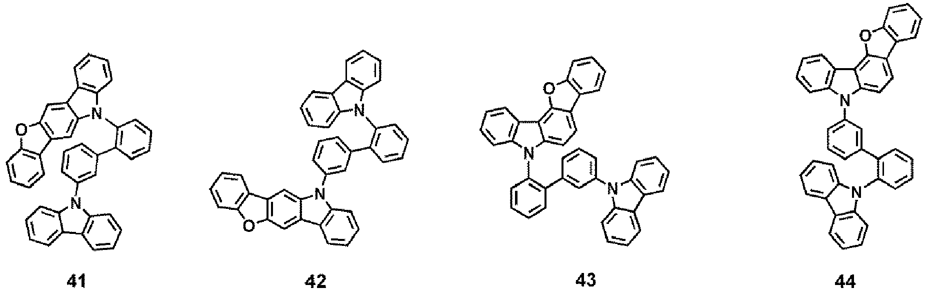

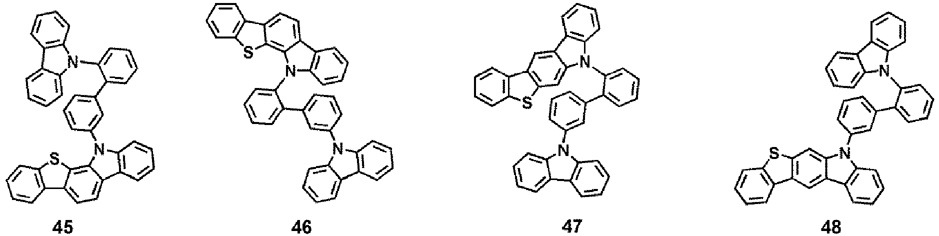

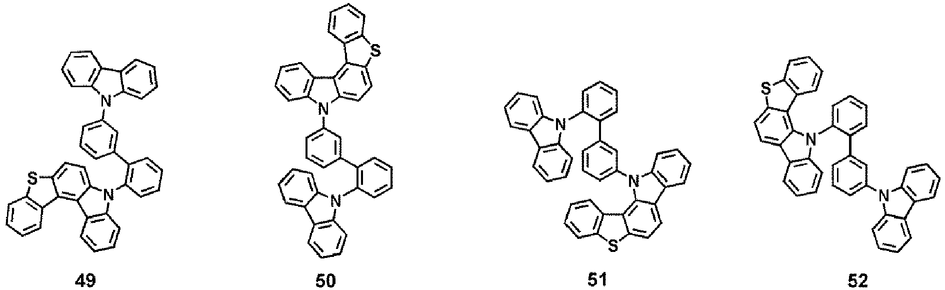

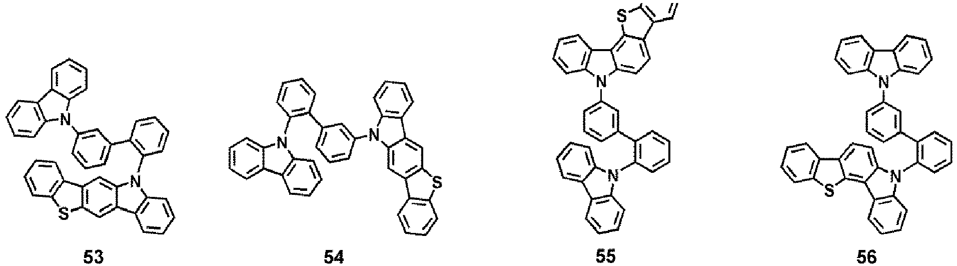

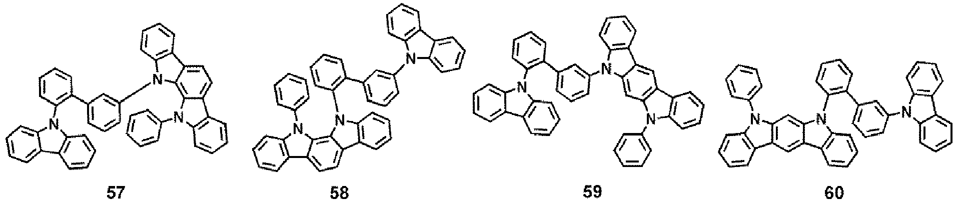

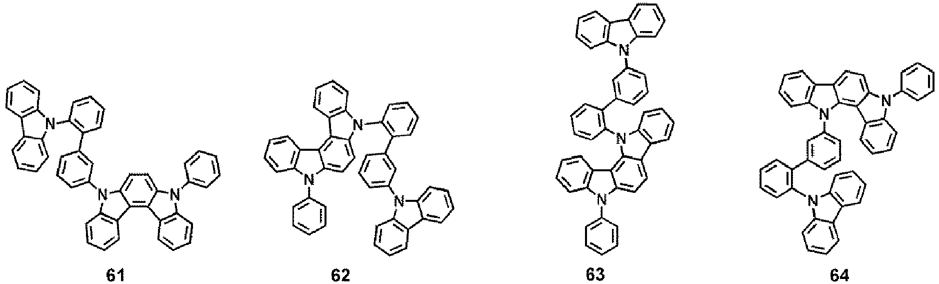

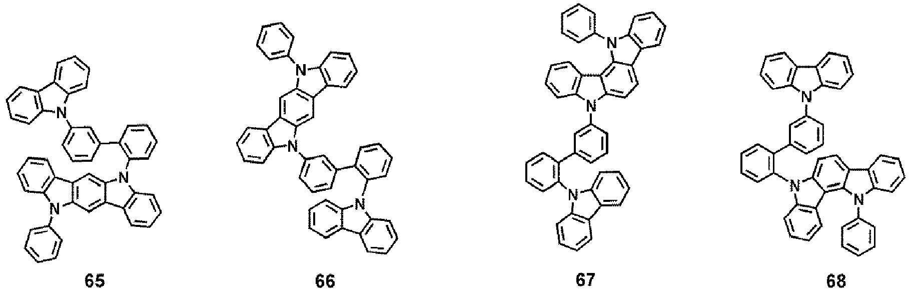

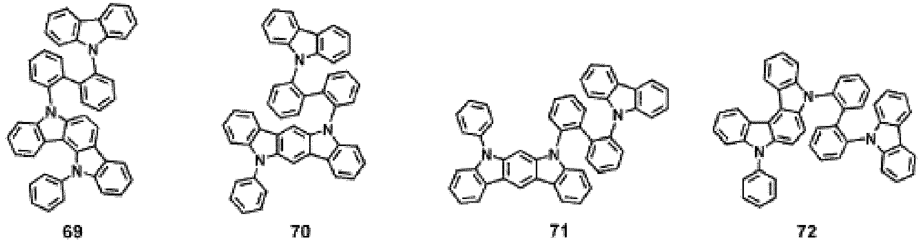

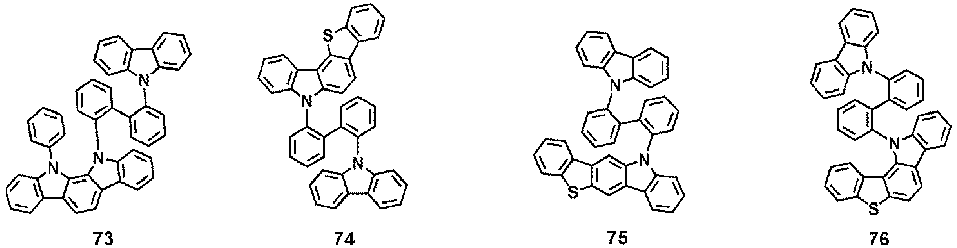

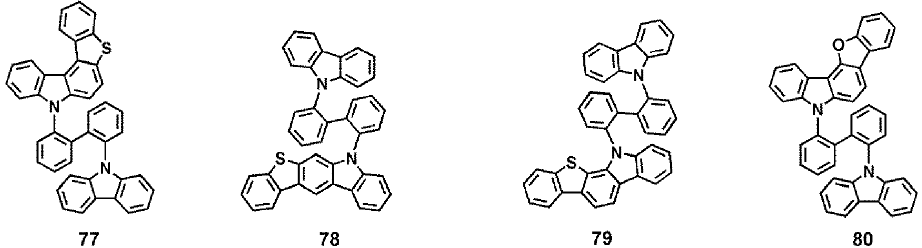

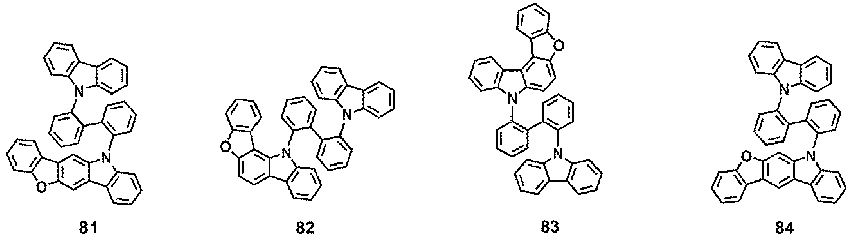

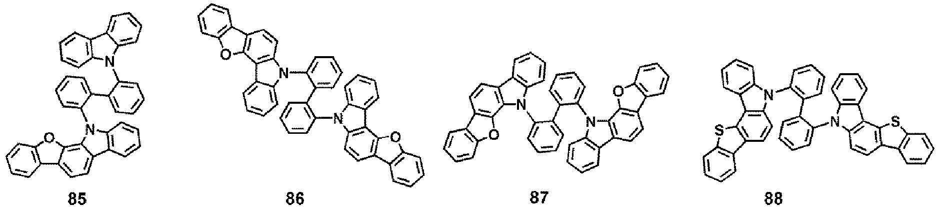

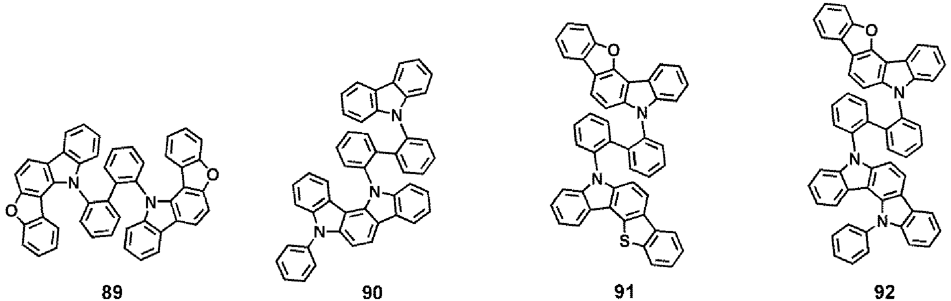

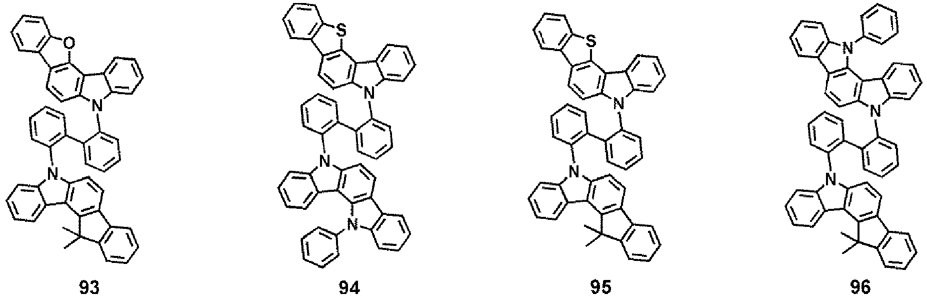

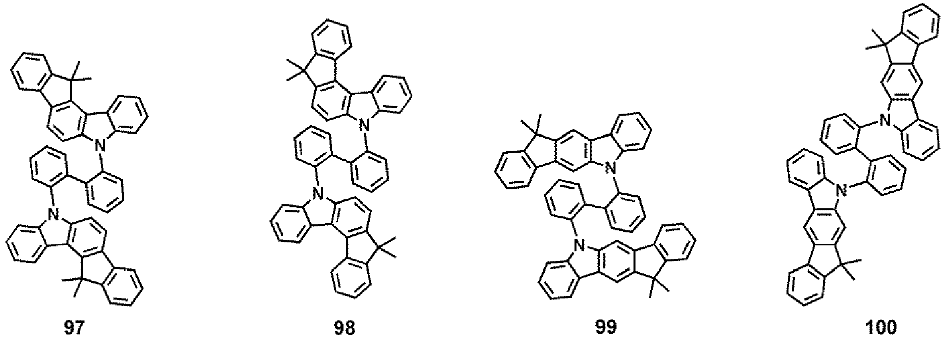

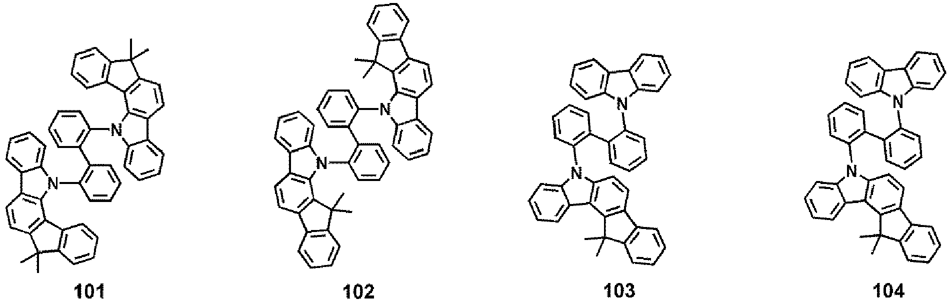

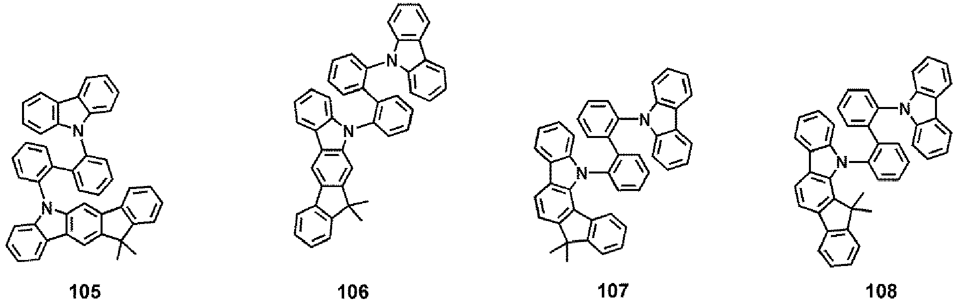

8. Organische lichtemittierende Vorrichtung, umfassend: eine erste Elektrode; eine zweite Elektrode; und
eine organische Schicht, die zwischen der ersten Elektrode und der zweiten Elektrode angeordnet ist und eine Emissionsschicht umfasst,
wobei die organische Schicht eine oder mehrere der durch die Formeln 1-1 bis 1-3 dargestellten kondensierten zyklischen Verbindungen nach einem der Ansprüche 1 bis 7 umfasst.

9. Organische lichtemittierende Vorrichtung nach Anspruch 8, wobei die erste Elektrode eine Anode ist,
die zweite Elektrode eine Kathode ist; und
die organische Schicht einen Lochtransportbereich, der zwischen der ersten Elektrode und der Emissionsschicht angeordnet ist, und einen Elektronentransportbereich, der zwischen der Emissionsschicht und der zweiten Elektrode angeordnet ist, umfasst;
wobei der Lochtransportbereich eine Lochinjektionsschicht, eine Lochtransportschicht, eine Elektronenblockierschicht oder eine beliebige Kombination davon umfasst; und
wobei der Elektronentransportbereich eine Lochblockierschicht, eine Elektronentransportschicht, eine Elektroneninjektionsschicht oder eine beliebige Kombination davon umfasst.

10. Organische lichtemittierende Vorrichtung nach Anspruch 8 oder 9, wobei
die Emissionsschicht die durch die Formeln 1-1 bis 1-3 dargestellte kondensierte zyklische Verbindung umfasst.

11. Organische lichtemittierende Vorrichtung nach einem der Ansprüche 8 bis 10, wobei die Emissionsschicht einen Wirt und ein Dotierungsmittel umfasst,
der Wirt die durch die Formeln 1-1 bis 1-3 dargestellte kondensierte zyklische Verbindung umfasst und
eine Menge des Wirts größer ist als eine Menge des Dotierungsmittels.

12. Organische lichtemittierende Vorrichtung nach einem der Ansprüche 9 bis 11, wobei der Lochtransportbereich eine Lochtransportschicht umfasst und
die Lochtransportschicht die durch die Formeln 1-1 bis 1-3 dargestellte kondensierte zyklische Verbindung umfasst.

13. Organische lichtemittierende Vorrichtung nach einem der Ansprüche 9 bis 11, wobei der Lochtransportbereich die Elektronenblockierschicht umfasst und
die Elektronenblockierschicht die durch die Formeln 1-1 bis 1-3 dargestellte kondensierte zyklische Verbindung umfasst.

## Revendications

1. Composé cyclique condensé représenté par l'une des Formules 1-1 à 1-3 : 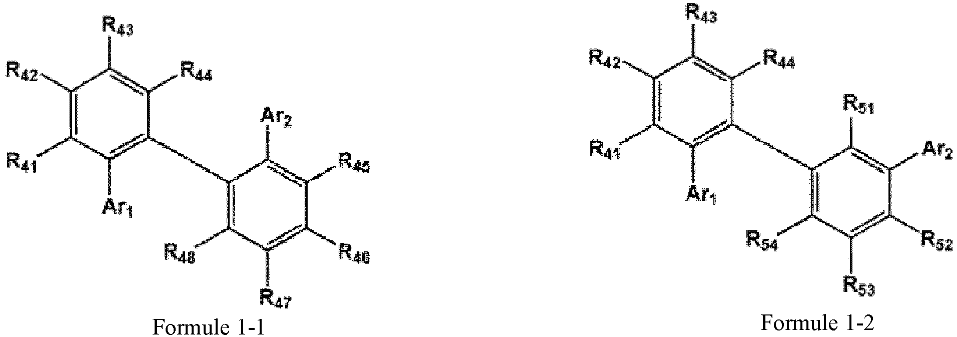 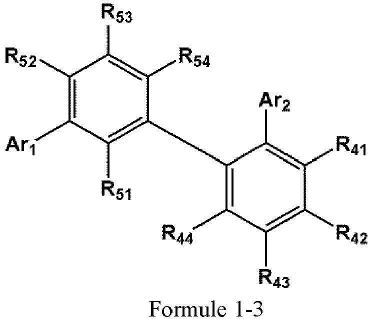 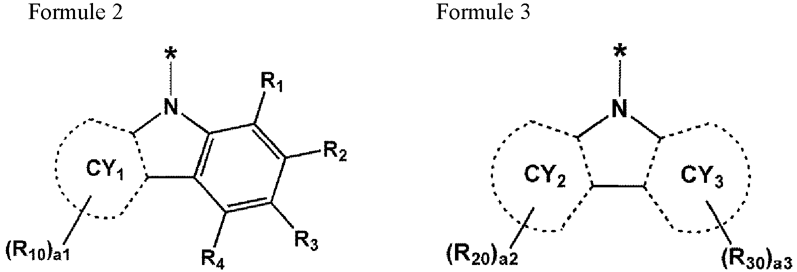 dans lesquelles
Ar₁ est un groupe représenté par la Formule 2,
Ar₂ est un groupe représenté par la Formule 3,
CY₁ est choisi parmi un groupe fluorène, un groupe carbazole, un groupe dibenzofurane, et un groupe dibenzothiophène,
CY₂ et CY₃ sont chacun indépendamment choisis parmi un groupe benzène, un groupe fluorène, un groupe carbazole, un groupe dibenzofurane, et un groupe dibenzothiophène,
R₁ à R₄, R₁₀, R₂₀ et R₃₀ sont chacun indépendamment choisis parmi un hydrogène, un deutérium, un groupe hydroxyle, un groupe cyano (CN), un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₆₀ substitué ou non substitué, un groupe alcényle en C₂-C₆₀ substitué ou non substitué, un groupe alcynyle en C₂-C₆₀ substitué ou non substitué, un groupe alcoxy en C₁-C₆₀ substitué ou non substitué, un groupe cycloalkyle en C₃-C₁₀ substitué ou non substitué, un groupe hétérocycloalkyle en C₁-C₁₀ substitué ou non substitué, un groupe cycloalcényle en C₃-C₁₀ substitué ou non substitué, un groupe hétérocycloalcényle en C₁-C₁₀ substitué ou non substitué, un groupe aryle en C₆-C₆₀ substitué ou non substitué, un groupe aryloxy en C₆-C₆₀ substitué ou non substitué, un groupe arylthio en C₆-C₆₀ substitué ou non substitué, un groupe arylalkyle en C₇-C₆₀ substitué ou non substitué, un groupe hétéroaryle en C₁-C₆₀ substitué ou non substitué, un groupe hétéroaryloxy en C₂-C₆₀ substitué ou non substitué, un groupe hétéroarylthio en C₂-C₆₀ substitué ou non substitué, un groupe hétéroarylalkyle en C₃-C₆₀ substitué ou non substitué, un groupe polycyclique condensé non aromatique monovalent substitué ou non substitué, un groupe hétéropolycyclique condensé non aromatique monovalent substitué ou non substitué, -Si(Q₁)(Q₂)(Q₃), -N(Q₄)(Q₅), et -B(Q₆)(Q₇),
R₄₁₋₄₈ et R₅₁₋₅₄ sont chacun indépendamment choisis parmi un hydrogène, un deutérium, un groupe hydroxyle, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₆₀ substitué ou non substitué, un groupe alcényle en C₂-C₆₀ substitué ou non substitué, un groupe alcynyle en C₂-C₆₀ substitué ou non substitué, un groupe alcoxy en C₁-C₆₀ substitué ou non substitué, un groupe cycloalkyle en C₃-C₁₀ substitué ou non substitué, un groupe hétérocycloalkyle en C₁-C₁₀ substitué ou non substitué, un groupe cycloalcényle en C₃-C₁₀ substitué ou non substitué, un groupe hétérocycloalcényle en C₁-C₁₀ substitué ou non substitué, un groupe aryle en C₆-C₆₀ substitué ou non substitué, un groupe aryloxy en C₆-C₆₀ substitué ou non substitué, un groupe arylthio en C₆-C₆₀ substitué ou non substitué, un groupe arylalkyle en C₇-C₆₀ substitué ou non substitué, un groupe hétéroaryle en C₁-C₆₀ substitué ou non substitué, un groupe hétéroaryloxy en C₂-C₆₀ substitué ou non substitué, un groupe hétéroarylthio en C₂-C₆₀ substitué ou non substitué, un groupe hétéroarylalkyle en C₃-C₆₀ substitué ou non substitué, un groupe polycyclique condensé non aromatique monovalent substitué ou non substitué, un groupe hétéropolycyclique condensé non aromatique monovalent substitué ou non substitué,
-Si(Q₁)(Q₂)(Q₃), -N(Q₄)(Q₅), et -B(Q₆)(Q₇),
a1 à a3 représentent chacun indépendamment un nombre entier valant de 0 à 10,
* est un site de liaison à un atome voisin,
au moins un substituant du groupe alkyle en C₁-C₆₀ substitué, du groupe alcényle en C₂-C₆₀ substitué, du groupe alcynyle en C₂-C₆₀ substitué, du groupe cycloalkyle en C₃-C₁₀ substitué, du groupe hétérocycloalkyle en C₁-C₁₀ substitué, du groupe cycloalcényle en C₃-C₁₀ substitué, du groupe hétérocycloalcényle en C₁-C₁₀ substitué, du groupe aryle en C₆-C₆₀ substitué, du groupe aryloxy en C₆-C₆₀ substitué, du groupe arylthio en C₆-C₆₀ substitué, du groupe arylalkyle en C₇-C₆₀ substitué, du groupe hétéroaryle en C₁-C₆₀ substitué, du groupe hétéroaryloxy en C₂-C₆₀ substitué, du groupe hétéroarylthio en C₂-C₆₀ substitué, du groupe hétéroarylalkyle en C₃-C₆₀ substitué, du groupe polycyclique condensé non aromatique monovalent substitué et groupe hétéropolycyclique condensé non aromatique monovalent substitué est choisi parmi :
un deutérium, -CD₃, -CD₂H, -CDH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₆₀, un groupe alcényle en C₂-C₆₀, un groupe alcynyle en C₂-C₆₀ et un groupe alcoxy en C₁-C₆₀ ;
un groupe alkyle en C₁-C₆₀, un groupe alcényle en C₂-C₆₀, un groupe alcynyle en C₂-C₆₀ et un groupe alcoxy en C₁-C₆₀, chacun étant substitué par au moins un groupe choisi parmi un deutérium, -CD₃, -CD₂H, -CDH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe arylalkyle en C₇-C₆₀, un groupe hétéroaryle en C₁-C₆₀, un groupe hétéroaryloxy en C₂-C₆₀, un groupe hétéroarylthio en C₂-C₆₀, un groupe hétéroarylalkyle en C₃-C₆₀, un groupe polycyclique condensé non aromatique monovalent, un groupe hétéropolycyclique condensé non aromatique monovalent, -Si(Q₁₁)(Q₁₂)(Q₁₃), -N(Q₁₄)(Q₁₅) et -B(Q₁₆)(Q₁₇) ;
un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe arylalkyle en C₇-C₆₀, un groupe hétéroaryle en C₁-C₆₀, un groupe hétéroaryloxy en C₂-C₆₀, un groupe hétéroarylthio en C₂-C₆₀, un groupe hétéroarylalkyle en C₃-C₆₀, un groupe polycyclique condensé non aromatique monovalent et un groupe hétéropolycyclique condensé non aromatique monovalent ;
un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe arylalkyle en C₇-C₆₀, un groupe hétéroaryle en C₁-C₆₀, un groupe hétéroaryloxy en C₂-C₆₀, un hétéroarylthio en C₂-C₆₀, un groupe hétéroarylalkyle en C₃-C₆₀, un groupe polycyclique condensé non aromatique monovalent et un groupe hétéropolycyclique condensé non aromatique monovalent, chacun étant substitué par au moins un groupe choisi parmi un deutérium, -CD₃, -CD₂H, -CDH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₆₀, un groupe alcényle en C₂-C₆₀, un groupe alcynyle en C₂-C₆₀, un groupe alcoxy en C₁-C₆₀, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe arylalkyle en C₇-C₆₀, un groupe hétéroaryle en C₁-C₆₀, un groupe hétéroaryloxy en C₂-C₆₀, un groupe hétéroarylthio en C₂-C₆₀, un groupe hétéroarylalkyle en C₃-C₆₀, un groupe polycyclique condensé non aromatique monovalent, un groupe hétéropolycyclique condensé non aromatique monovalent, -Si(Q₂₁)(Q₂₂)(Q₂₃), - N(Q₂₄)(Q₂₅) et -B(Q₂₆)(Q₂₇) ; et
-Si(Q₃₁)(Q₃₂)(Q₃₃), -N(Q₃₄)(Q₃₅) et -B(Q₃₆)(Q₃₇), et
Q₁ à Q₇, Q₁₁ à Q₁₇, Q₂₁ à Q₂₇ et Q₃₁ à Q₃₇ sont chacun indépendamment choisis parmi un hydrogène, un deutérium, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₆₀ substitué ou non substitué, un groupe alcényle en C₂-C₆₀ substitué ou non substitué, un groupe alcynyle en C₂-C₆₀ substitué ou non substitué, un groupe alcoxy en C₁-C₆₀ substitué ou non substitué, un groupe cycloalkyle en C₃-C₁₀ substitué ou non substitué, un groupe hétérocycloalkyle en C₁-C₁₀ substitué ou non substitué, un groupe cycloalcényle en C₃-C₁₀ substitué ou non substitué, un groupe hétérocycloalcényle en C₁-C₁₀ substitué ou non substitué, un groupe aryle en C₆-C₆₀ substitué ou non substitué, un groupe aryloxy en C₆-C₆₀ substitué ou non substitué, un groupe arylthio en C₆-C₆₀ substitué ou non substitué, un groupe arylalkyle en C₇-C₆₀ substitué ou non substitué, un groupe hétéroaryle en C₁-C₆₀ substitué ou non substitué, un groupe hétéroaryloxy en C₂-C₆₀ substitué ou non substitué, un groupe hétéroarylthio en C₂-C₆₀ substitué ou non substitué, un groupe hétéroarylalkyle en C₃-C₆₀ substitué ou non substitué, un groupe polycyclique condensé non aromatique monovalent substitué ou non substitué et un groupe hétéropolycyclique condensé non aromatique monovalent substitué ou non substitué.

2. Composé cyclique condensé selon la revendication 1, dans lequel
Ar₁ est choisi parmi des groupes représentés par les Formules 2-1 à 2-6, et
Ar₂ est choisi parmi des groupes représentés par les Formules 3-1 à 3-7 : 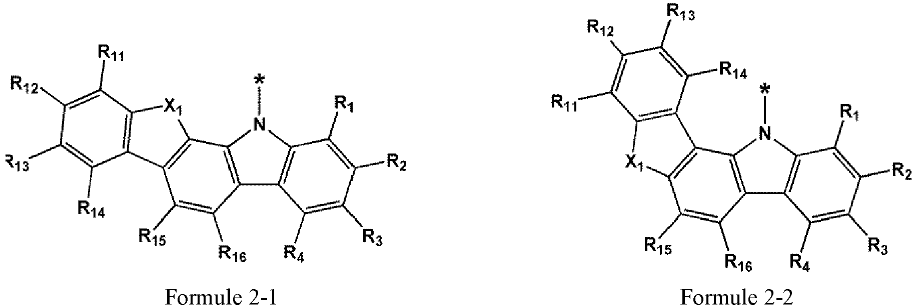 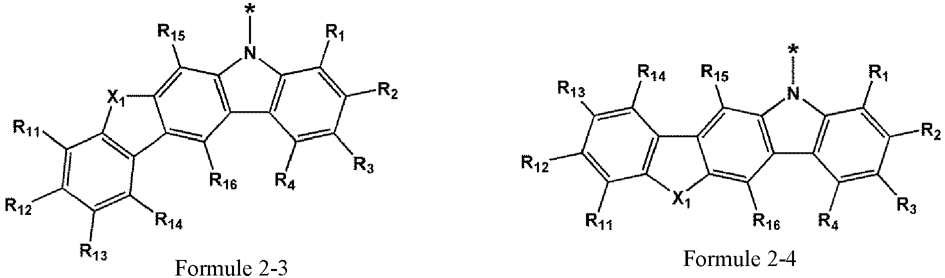 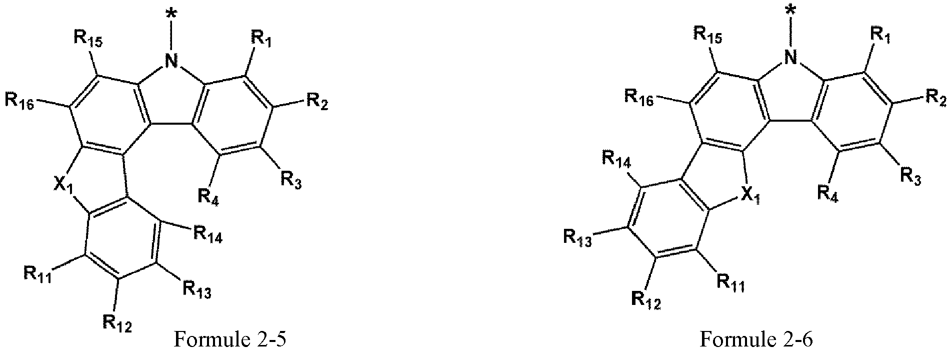 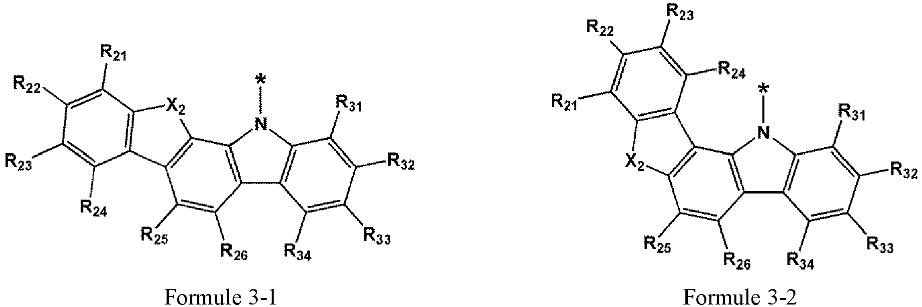 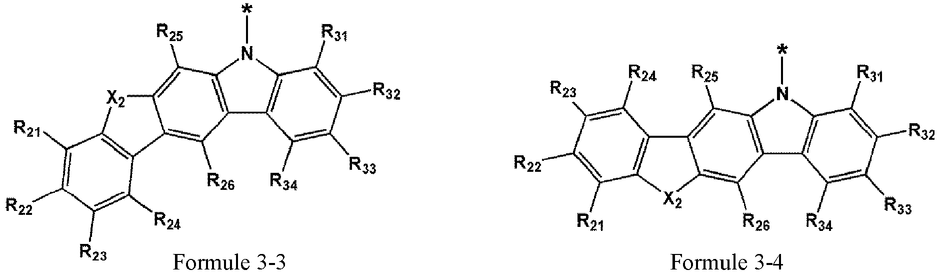 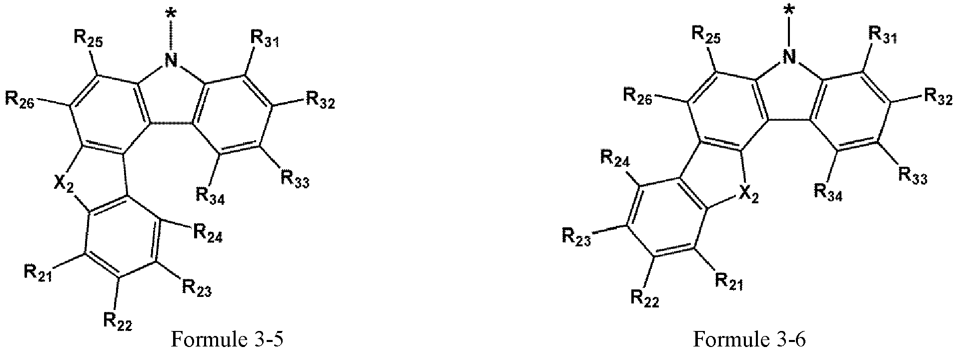 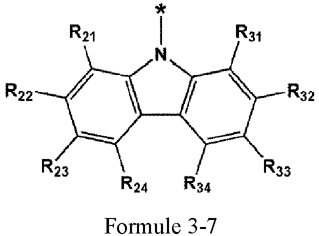 dans les Formules 2-1 à 2-6 et 3-1 à 3-7,
X₁ représente C(R₁₇)(R₁₈), N(R₁₉), O ou S,
X₂ représente C(R₂₇)(R₂₈), N(R₂₉), O ou S,
R₁ à R₄, R₁₁ à R₁₉, R₂₁ à R₂₉ et R₃₁ à R₃₄ sont chacun indépendamment choisis parmi
un hydrogène, un deutérium, un groupe hydroxyle, un groupe cyano (CN), un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₂₀ et un groupe alcoxy en C₁-C₂₀ ;
un groupe alkyle en C₁-C₂₀ et un groupe alcoxy en C₁-C₂₀, chacun étant substitué par au moins un groupe choisi parmi un deutérium, un groupe hydroxyle, un groupe cyano (CN), un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe phényle, un groupe biphényle, un groupe terphényle et un groupe naphtyle ;
un groupe cyclopentyle, un groupe cyclohexyle, un groupe cyclopentényle, un groupe cyclohexényle, un groupe cycloheptényle, un groupe phényle, un groupe biphényle, un groupe terphényle, un groupe pentalényle, un groupe indényle, un groupe naphtyle, un groupe azulényle, un groupe heptalényle, un groupe indacényle, un groupe acénaphtyle, un groupe fluorényle, un groupe spiro-bifluorényle, un groupe phénalényle, un groupe phénanthrényle, un groupe anthracényle, un groupe fluoranthényle, un groupe triphénylényle, un groupe pyrényle, un groupe chrysényle, un groupe naphtacényle, un groupe picényle, un groupe perylényle, un groupe pentaphényle, un groupe hexacényle, un groupe pyrrolyle, un groupe imidazolyle, un groupe pyrazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe phtalazinyle, un groupe naphtyridinyle, un groupe quinoxalinyle, un groupe quinazolinyle, un groupe cinnolinyle, un groupe carbazolyle, un groupe phénanthridinyle, un groupe acridinyle, un groupe phénanthrolinyle, un groupe phénazinyle, un groupe benzooxazolyle, un groupe benzoimidazolyle, un groupe furanyle, un groupe benzofuranyle, un groupe thiophényle, un groupe benzothiophényle, un thiazolyle, un groupe isothiazolyle, un groupe benzothiazolyle, un groupe isoxazolyle, un groupe oxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyrimidinyle, un groupe imidazopyridinyle, un groupe pyridoindolyle, un groupe benzofuropyridinyle, un groupe benzothiénopyridinyle, un groupe pyrimidoindolyle, un groupe benzofuropyrimidinyle, un groupe benzothiénopyrimidinyle, un groupe phénoxazinyle, un groupe pyridobenzooxazinyle et un groupe pyridobenzothiazinyle ;
un groupe cyclopentyle, un groupe cyclohexyle, un groupe cyclopentényle, un groupe cyclohexényle, un groupe cycloheptényle, un groupe phényle, un groupe biphényle, un groupe terphényle, un groupe pentalényle, un groupe indényle, un groupe naphtyle, un groupe azulényle, un groupe heptalényle, un groupe indacényle, un groupe acénaphtyle, un groupe fluorényle, un groupe spiro-fluorényle, un groupe phénalényle, un groupe phénanthrényle, un groupe anthracényle, un groupe fluoranthényle, un groupe triphénylényle, un groupe pyrényle, un groupe chrysényle, un groupe naphtacényle, un groupe picényle, un groupe perylényle, un groupe pentaphényle, un groupe hexacényle, un groupe pyrrolyle, un groupe imidazolyle, un groupe pyrazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe phtalazinyle, un groupe naphtyridinyle, un groupe quinoxalinyle, un groupe quinazolinyle, un groupe cinnolinyle, un groupe carbazolyle, un groupe phénanthridinyle, un groupe acridinyle, un groupe phénanthrolinyle, un groupe phénazinyle, un groupe benzoxazolyle, un groupe benzoimidazolyle, un groupe furanyle, un groupe benzofuranyle, un groupe thiophényle, un groupe benzothiophényle, un thiazolyle, un groupe isothiazolyle, un groupe benzothiazolyle, un groupe isoxazolyle, un groupe oxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyrimidinyle, un groupe imidazopyridinyle, un groupe pyridoindolyle, un groupe benzofuropyridinyle, un groupe benzothiénopyridinyle, un groupe pyrimidoindolyle, un groupe benzofuropyrimidinyle, un groupe benzothiénopyrimidinyle, un groupe phénoxazinyle, un groupe pyridobenzooxazinyle et un groupe pyridobenzothiazinyle, chacun étant substitué par au moins un groupe choisi parmi un deutérium, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₂₀, un groupe alcényle en C₂-C₂₀, un groupe alcynyle en C₂-C₂₀, un groupe alcoxy en C₁-C₂₀, un groupe phényle, un groupe biphényle, un groupe terphényle, un groupe naphtyle, un groupe anthracényle, un groupe pyrényle, un groupe phénanthrényle, un groupe fluorényle, un groupe carbazolyle, un groupe benzocarbazolyle, un groupe dibenzocarbazolyle, un groupe pyridinyle, un groupe pyrimidinyle, un groupe pyrazinyle, un groupe pyridazinyle, un groupe triazinyle, un groupe quinolinyle, un groupe isoquinolinyle, un groupe phtalazinyle, un groupe quinoxalinyle, un groupe cinnolinyle, un groupe quinazolinyle,
-Si(Q₃₁)(Q₃₂)(Q₃₃), -N(Q₃₄)(Q₃₅) et -B(Q₃₆)(Q₃₇) ; et
-Si(Q₁)(Q₂)(Q₃), -N(Q₄)(Q₅), et -B(Q₆)(Q₇),
dans lequel Q₁ à Q₇ et Q₃₁ à Q₃₇ sont chacun indépendamment choisis parmi un hydrogène, un groupe alkyle en C₁-C₂₀, un groupe alcoxy en C₁-C₂₀, un groupe phényle, un groupe biphényle, un groupe terphényle, un groupe naphtyle, un groupe fluorényle, un groupe carbazolyle, un groupe dibenzofuranyle et un groupe dibenzothiophényle, et
* est un site de liaison à un atome voisin, de préférence dans lequel
R₁ à R₄, R₁₁ à R₁₉, R₂₁ à R₂₉ et R₃₁ à R₃₄ sont chacun indépendamment choisis parmi
un hydrogène, un deutérium, un groupe cyano (CN), un groupe alkyle en C₁-C₂₀ et un groupe alcoxy en C₁-C₂₀ ;
un groupe alkyle en C₁-C₂₀ et un groupe alcoxy en C₁-C₂₀, chacun étant substitué par au moins un groupe choisi parmi un deutérium, un groupe cyano (CN), un groupe phényle, un groupe biphényle, un groupe terphényle et un groupe naphtyle ;
un groupe cyclopentyle, un groupe cyclohexyle, un groupe cyclopentényle, un groupe cyclohexényle, un groupe cycloheptényle, un groupe phényle, un groupe biphényle, un groupe terphényle, un groupe naphtyle, un groupe fluorényle, un groupe carbazolyle, un groupe dibenzofuranyle et un groupe dibenzothiophényle ;
un groupe cyclopentyle, un groupe cyclohexyle, un groupe cyclopentényle, un groupe cyclohexényle, un groupe cycloheptényle, un groupe phényle, un groupe biphényle, un groupe terphényle, un groupe naphtyle, un groupe fluorényle, un groupe carbazolyle, un groupe dibenzofuranyle et un groupe dibenzothiophényle, chacun étant substitué par au moins un groupe choisi parmi un deutérium, un groupe cyano, un groupe alkyle en C₁-C₂₀, un groupe alcoxy en C₁-C₂₀, un groupe phényle, un groupe biphényle, un groupe terphényle, un groupe naphtyle et
-Si(Q₃₁)(Q₃₂)(Q₃₃) ; et -Si(Q₁)(Q₂)(Q₃), et
Q₁ à Q₃ et Q₃₁ à Q₃₃ sont chacun indépendamment choisis parmi un hydrogène, un groupe alkyle en C₁-C₂₀, un groupe alcoxy en C₁-C₂₀, un groupe phényle, un groupe biphényle, un groupe terphényle et un groupe naphtyle, et/ou de préférence dans lequel
X₁ représente O ou S.

3. Composé cyclique condensé selon la revendication 1 ou la revendication 2, dans lequel Ar₁ est le même que Ar₂ dans les Formules 1-1 à 1-3 ; ou
CY₂ et CY₃ dans la Formule 3 représentent chacun indépendamment un groupe benzène.

4. Composé cyclique condensé selon la revendication 2, dans lequel
Ar₁ est représenté par la Formule 2-1, et Ar₂ est représenté par la Formule 3-1 ;
Ar₁ est représenté par la Formule 2-2, et Ar₂ est représenté par la Formule 3-2 ; ou
Ar₁ est représenté par la Formule 2-6, et Ar₂ est représenté par la Formule 3-6.

5. Composé cyclique condensé selon l'une quelconque des revendications 1 à 4, dans lequel
le composé cyclique condensé est représenté par la Formule 1-1, et/ou dans lequel R₄₁ à R₄₈ et R₅₁ à R₅₄ sont chacun indépendamment choisis parmi un hydrogène, un deutérium, un groupe alkyle en C₁-C₂₀ et un groupe alcoxy en C₁-C₂₀; un groupe alkyle en C₁-C₂₀ et un groupe alcoxy en C₁-C₂₀, chacun étant substitué par au moins un groupe choisi parmi un deutérium, un groupe phényle, un groupe biphényle, un groupe terphényle et un groupe naphtyle ;
un groupe cyclopentyle, un groupe cyclohexyle, un groupe cyclopentényle, un groupe cyclohexényle, un groupe cycloheptényle, un groupe phényle, un groupe biphényle, un groupe terphényle, un groupe naphtyle, un groupe fluorényle, un groupe carbazolyle, un groupe dibenzofuranyle et un groupe dibenzothiophényle ;
un groupe cyclopentyle, un groupe cyclohexyle, un groupe cyclopentényle, un groupe cyclohexényle, un groupe cycloheptényle, un groupe phényle, un groupe biphényle, un groupe terphényle, un groupe naphtyle, un groupe fluorényle, un groupe carbazolyle, un groupe dibenzofuranyle et un groupe dibenzothiophényle, chacun étant substitué par au moins un groupe choisi parmi un deutérium, un groupe alkyle en C₁-C₂₀, un groupe alcoxy en C₁-C₂₀, un groupe phényle, un groupe biphényle, un groupe terphényle, un groupe naphtyle et -Si(Q₃₁)(Q₃₂)(Q₃₃) ; et -Si(Q₁)(Q₂)(Q₃), et
Q₁ à Q₃ et Q₃₁ à Q₃₃ sont chacun indépendamment choisis parmi un hydrogène, un groupe alkyle en C₁-C₂₀, un groupe alcoxy en C₁-C₂₀, un groupe phényle, un groupe biphényle, un groupe terphényle et un groupe naphtyle.

6. Composé cyclique condensé selon la revendication 5, dans lequel
Ar₁ est choisi parmi des groupes représentés par les Formules 2-1 à 2-6,
Ar₂ est choisi parmi des groupes représentés par les Formules 3-1 à 3-7 : 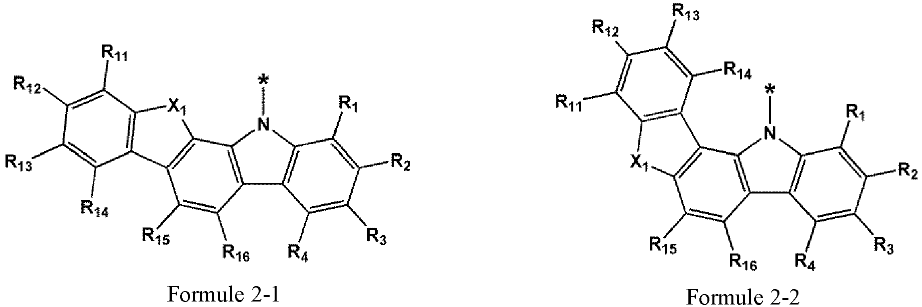 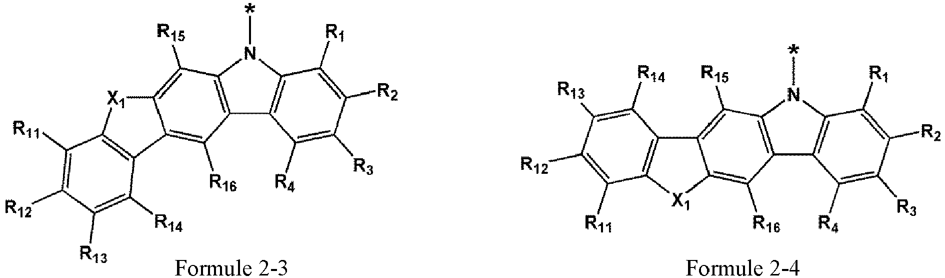 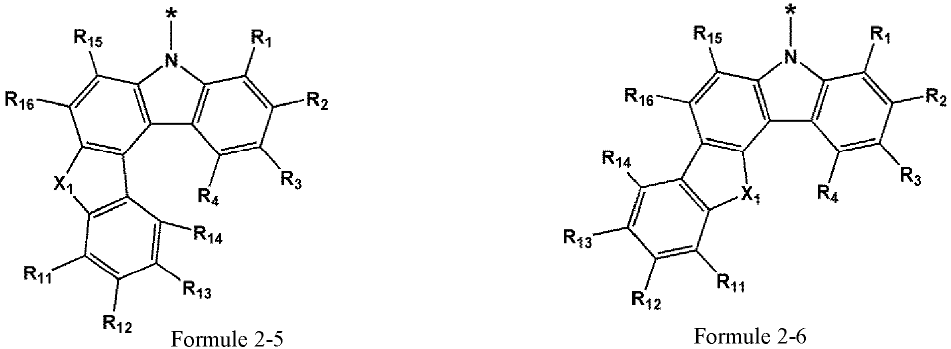 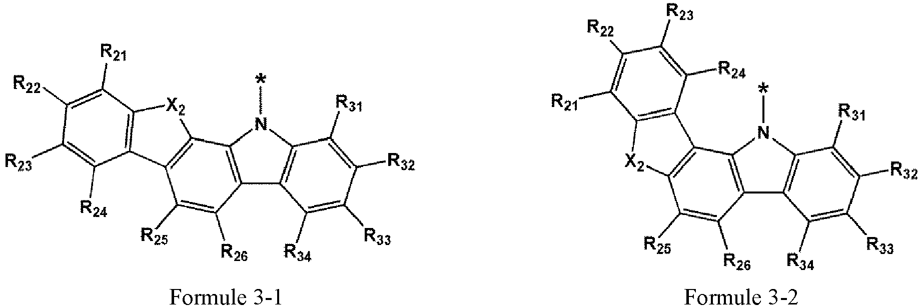 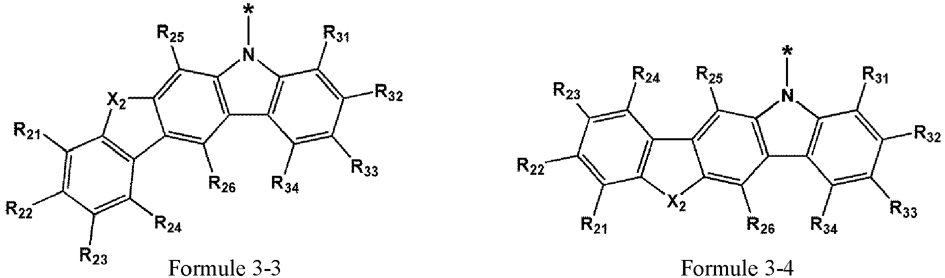 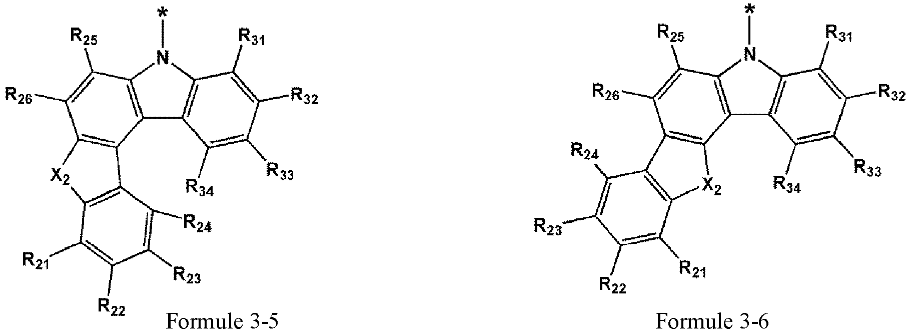 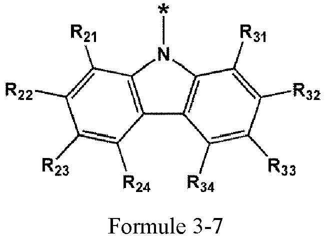 dans les Formules 2-1 à 2-6 et 3-1 à 3-7,
X₁ représente C(R₁₇)(R₁₈), N(R₁₉), O ou S,
X₂ représente C(R₂₇)(R₂₈), N(R₂₉), O ou S,
R₁ à R₄, R₁₁ à R₁₉, R₂₁ à R₂₉ et R₃₁ à R₃₄ sont chacun indépendamment choisis parmi
un hydrogène, un deutérium, un groupe cyano (CN), un groupe alkyle en C₁-C₂₀ et un groupe alcoxy en C₁-C₂₀ ;
un groupe alkyle en C₁-C₂₀ et un groupe alcoxy en C₁-C₂₀, chacun étant substitué par au moins un groupe choisi parmi un deutérium, un groupe cyano (CN), un groupe phényle, un groupe biphényle, un groupe terphényle et un groupe naphtyle ;
un groupe cyclopentyle, un groupe cyclohexyle, un groupe cyclopentényle, un groupe cyclohexényle, un groupe cycloheptényle, un groupe phényle, un groupe biphényle, un groupe terphényle, un groupe naphtyle, un groupe fluorényle, un groupe carbazolyle, un groupe dibenzofuranyle et un groupe dibenzothiophényle ;
un groupe cyclopentyle, un groupe cyclohexyle, un groupe cyclopentényle, un groupe cyclohexényle, un groupe cycloheptényle, un groupe phényle, un groupe biphényle, un groupe terphényle, un groupe naphtyle, un groupe fluorényle, un groupe carbazolyle, un groupe dibenzofuranyle et un groupe dibenzothiophényle, chacun étant substitué par au moins un groupe choisi parmi un deutérium, un groupe cyano, un groupe alkyle en C₁-C₂₀, un groupe alcoxy en C₁-C₂₀, un groupe phényle, un groupe biphényle, un groupe terphényle, un groupe naphtyle et
-Si(Q₃₁)(Q₃₂)(Q₃₃) ; et -Si(Q₁)(Q₂)(Q₃),
dans lequel Q₁ à Q₃ et Q₃₁ à Q₃₃ sont chacun indépendamment choisis parmi un hydrogène, un groupe alkyle en C₁-C₂₀, un groupe alcoxy en, C₁-C₂₀ un groupe phényle, un groupe biphényle, un groupe terphényle et un groupe naphtyle, de préférence dans lequel
le composé cyclique condensé est représenté par la Formule 1-1, et X₁ dans les Formules 2-1 à 2-6 représente O ou S.

7. Composé cyclique condensé selon la revendication 1, dans lequel
le composé cyclique condensé est choisi parmi les Composés 1 à 108 :

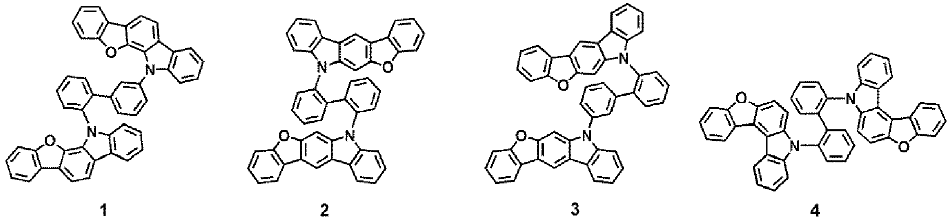

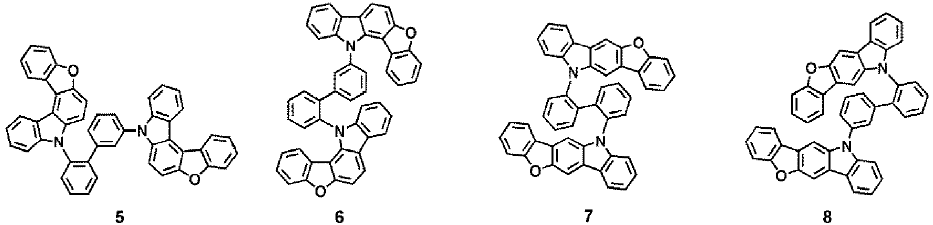

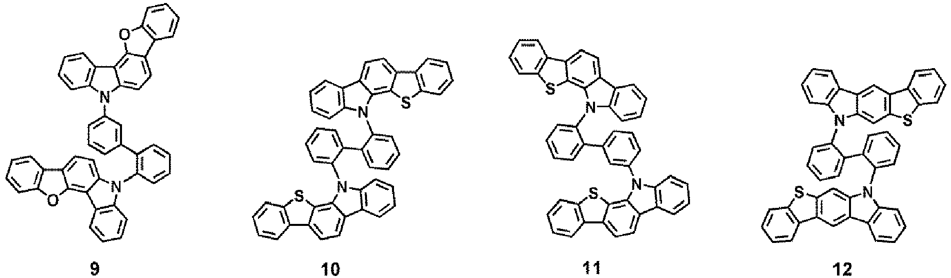

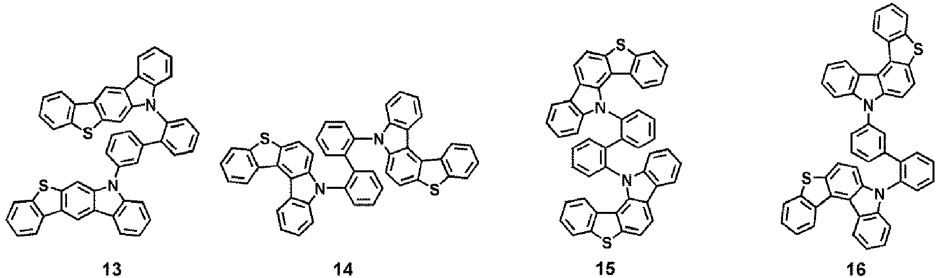

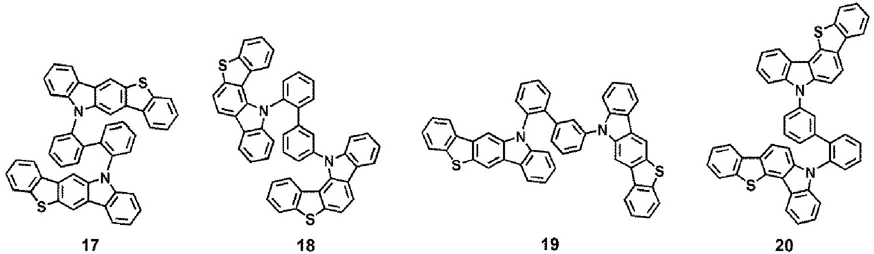

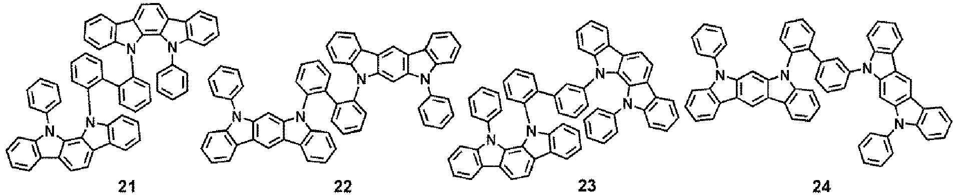

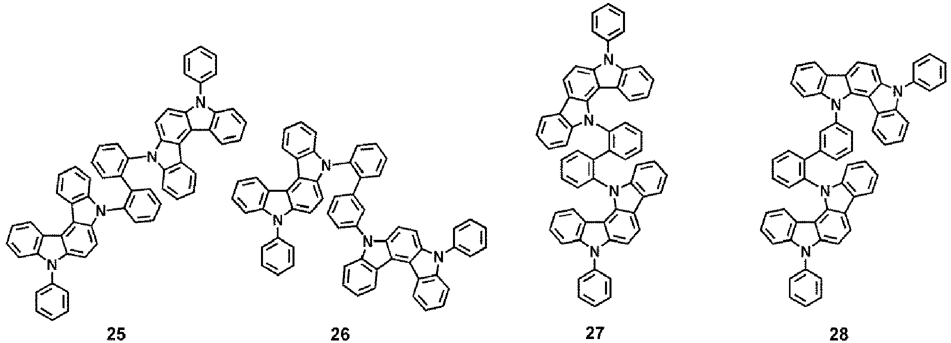

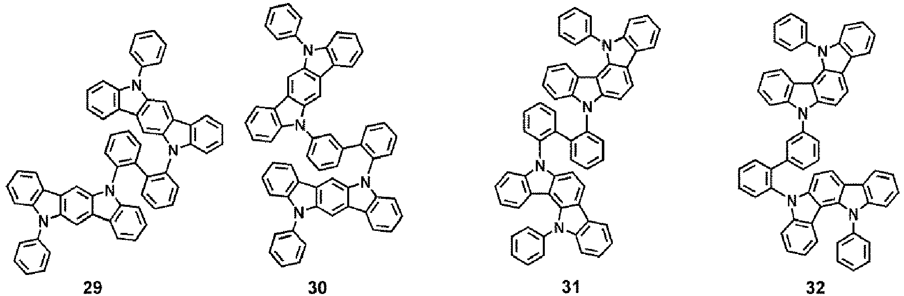

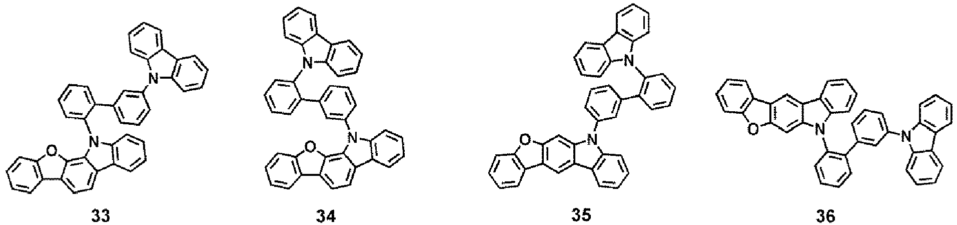

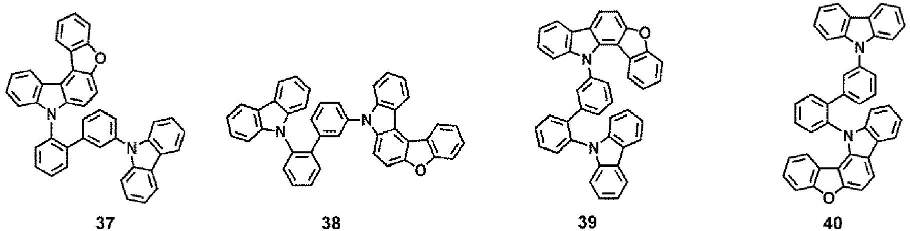

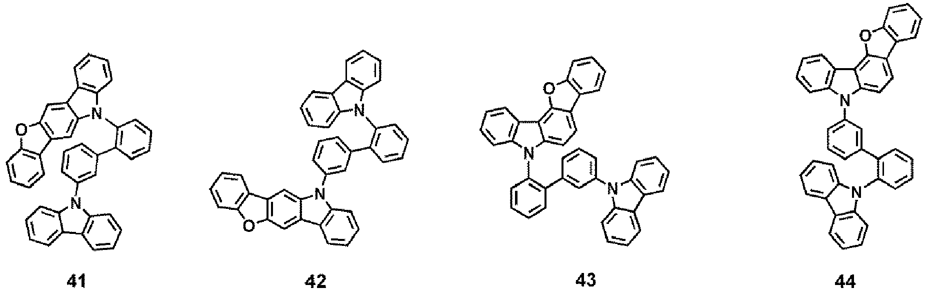

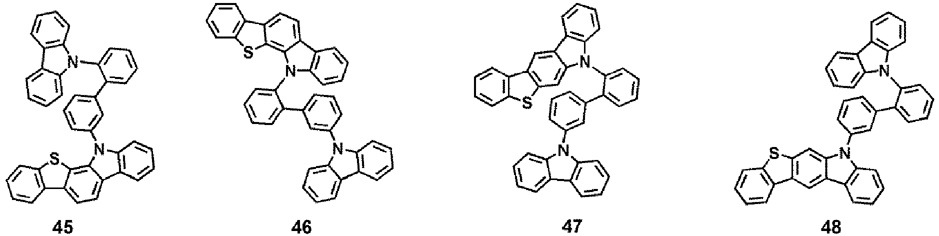

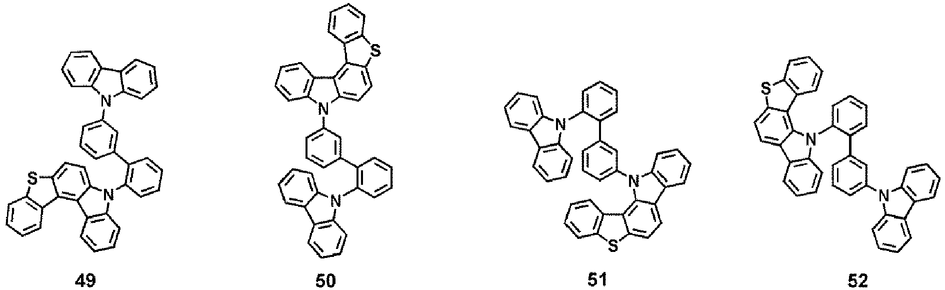

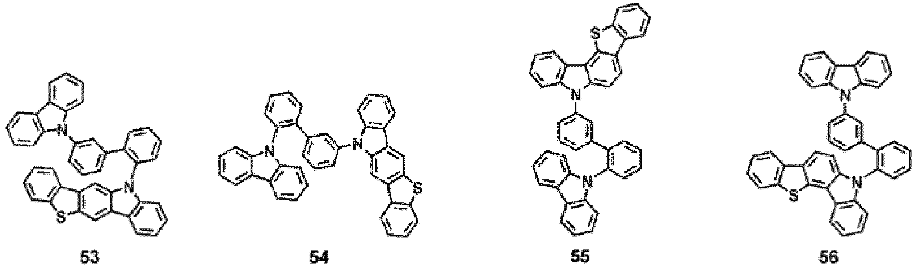

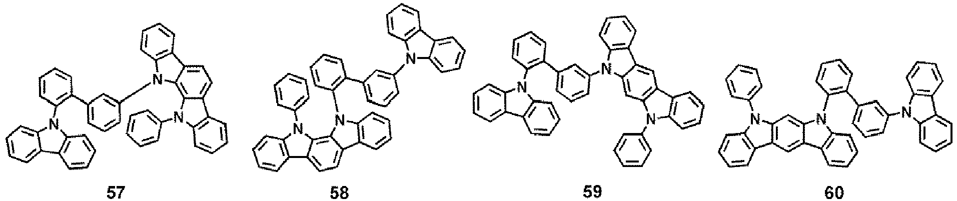

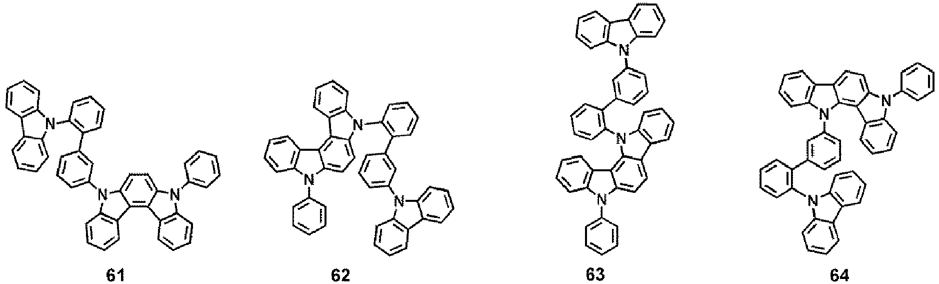

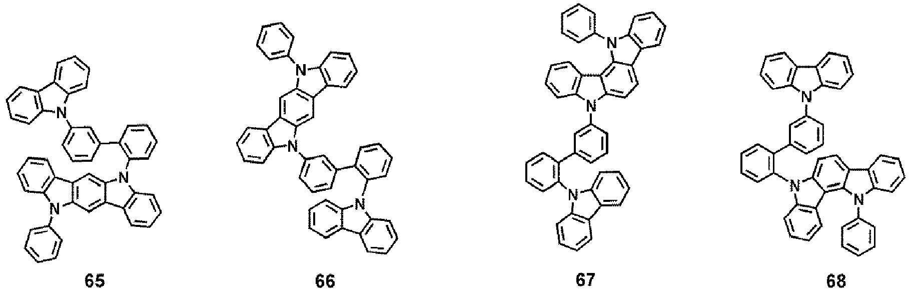

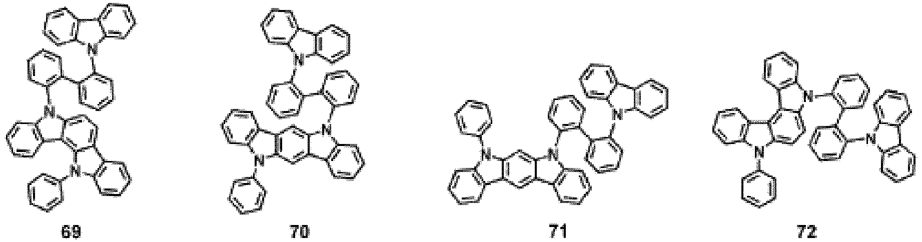

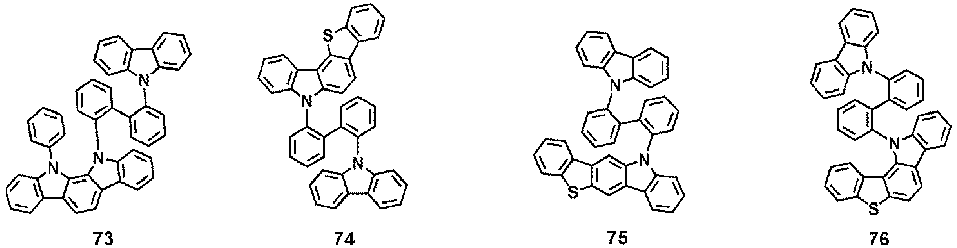

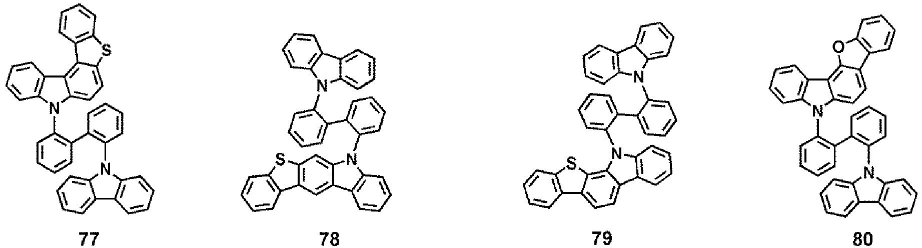

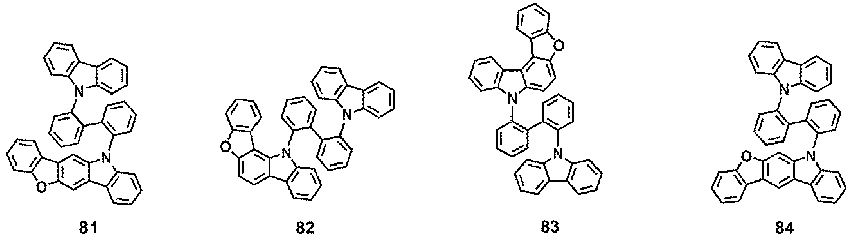

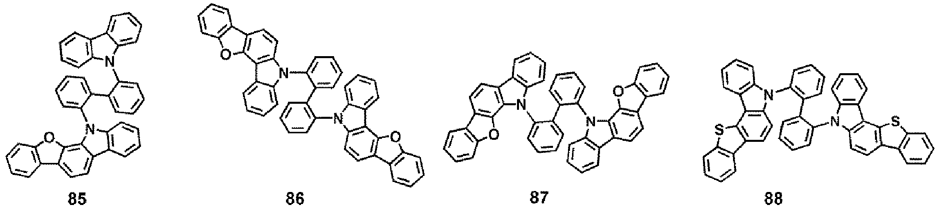

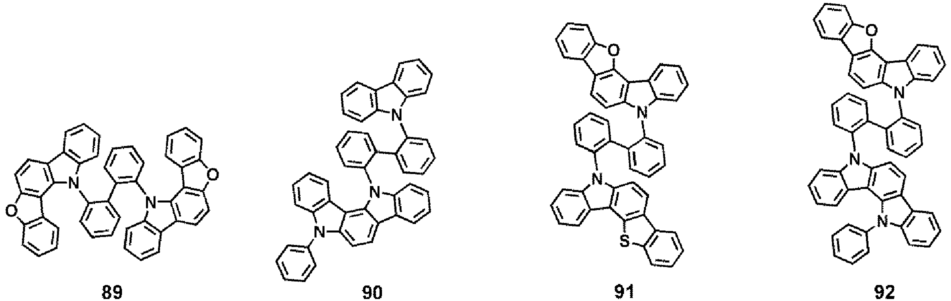

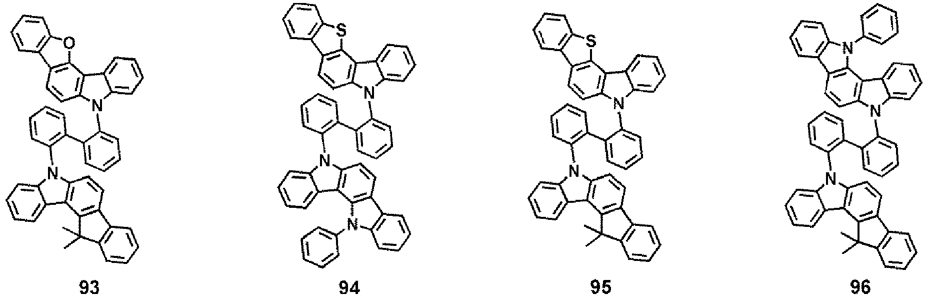

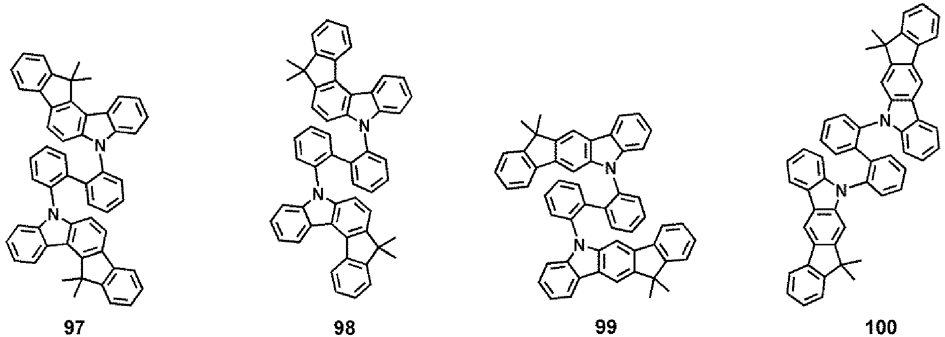

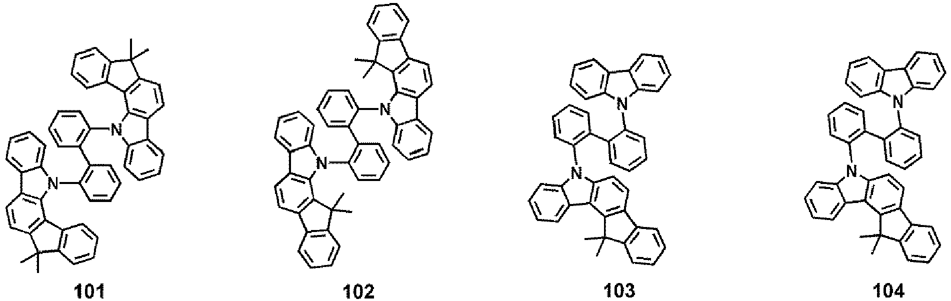

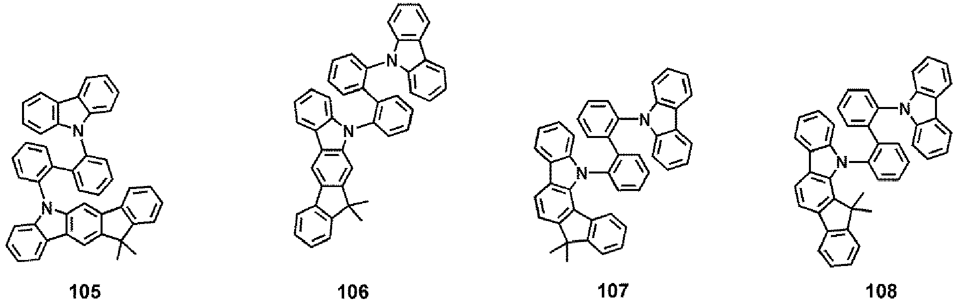

8. Dispositif organique électroluminescent comprenant : une première électrode ; une seconde électrode ; et
une couche organique disposée entre la première électrode et la seconde électrode et comprenant une couche d'émission,
dans lequel la couche organique comprend au moins un composé cyclique condensé représenté par les Formules 1-1 à 1-3 selon l'une quelconque des revendications 1 à 7.

9. Dispositif organique électroluminescent selon la revendication 8, dans lequel la première électrode est une anode,
la seconde électrode est une cathode ; et
la couche organique comprend une région de transport de trous disposée entre la première électrode et la couche d'émission et une région de transport d'électrons disposée entre la couche d'émission et la seconde électrode ;
dans lequel la région de transport de trous comprend une couche d'injection de trous, une couche de transport de trous, une couche de blocage d'électrons, ou toute combinaison de celles-ci ; et
dans lequel la région de transport d'électrons comprend une couche de blocage de trous, une couche de transport d'électrons, une couche d'injection d'électrons, ou toute combinaison de celles-ci.

10. Dispositif organique électroluminescent selon la revendication 8 ou la revendication 9, dans lequel
la couche d'émission comprend le composé cyclique condensé représenté par les Formules 1-1 à 1-3.

11. Dispositif organique électroluminescent selon l'une quelconque des revendications 8 à 10, dans lequel la couche d'émission comprend un hôte et un dopant,
l'hôte comprend le composé cyclique condensé représenté par les Formules 1-1 à 1-3, et
une quantité de l'hôte est supérieure à une quantité du dopant.

12. Dispositif organique électroluminescent selon l'une quelconque des revendications 9 à 11, dans lequel la région de transport de trous comprend une couche de transport de trous, et
la couche de transport de trous comprend le composé cyclique condensé représenté par les Formules 1-1 à 1-3.

13. Dispositif organique électroluminescent selon l'une quelconque des revendications 9 à 11, dans lequel la région de transport de trous comprend la couche de blocage d'électrons, et
la couche de blocage d'électrons comprend le composé cyclique condensé représenté par les Formules 1-1 à 1-3.
